(19) Europäisches Patentamt / European Patent Office / Office européen des brevets

(11) **EP 4 097 257 B1**

(12) **FASCICULE DE BREVET EUROPEEN**

(45) Date de publication et mention de la délivrance du brevet:
**20.11.2024 Bulletin 2024/47**

(21) Numéro de dépôt: **21702485.0**

(22) Date de dépôt: **28.01.2021**

(51) Classification Internationale des Brevets (IPC):
**C12Q 1/6883** (2018.01)

(52) Classification Coopérative des Brevets (CPC):
**C12Q 1/6883;** C12Q 2600/124; C12Q 2600/158

(86) Numéro de dépôt international:
**PCT/EP2021/052041**

(87) Numéro de publication internationale:
**WO 2021/152051 (05.08.2021 Gazette 2021/31)**

(54) **PROCEDE DE SELECTION DES SPERMATOZOÏDES NOTAMMENT EN VUE D'UNE PROCREATION MEDICALEMENT ASSISTEE (PMA)**

AUSWAHLVERFAHREN VON SPERMATOZOIDEN, INSBESONDERE IM HINBLICK AUF EINE MEDIZINISCH UNTERSTÜTZTE FORTPFLANZUNG

METHOD FOR SELECTING SPERMATOZOA, IN PARTICULAR WITH A VIEW TO MEDICALLY ASSISTED REPRODUCTION (MAR)

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **30.01.2020 EP 20305084**

(43) Date de publication de la demande:
**07.12.2022 Bulletin 2022/49**

(73) Titulaire: **Cassuto, Nino Guy**
**92100 Boulogne (FR)**

(72) Inventeur: **Cassuto, Nino Guy**
**92100 Boulogne (FR)**

(74) Mandataire: **Santarelli**
**Tour Trinity**
**1 bis Esplanade de la Défense**
**92035 Paris La Défense Cedex (FR)**

(56) Documents cités:
**EP-A1- 2 532 757**

- S. BONACHE ET AL: "Sperm gene expression profile is related to pregnancy rate after insemination and is predictive of low fecundity in normozoospermic men", HUMAN REPRODUCTION, vol. 27, no. 6, 1 June 2012 (2012-06-01), pages 1556 - 1567, XP055182184, ISSN: 0268-1161, DOI: 10.1093/humrep/des074
- JOHNSON MARQUITA L ET AL: "Novel localization of Aurora A kinase in mouse testis suggests multiple roles in spermatogenesis", BIOCHEMICAL AND BIOPHYSICAL RESEARCH COMMUNICATIONS, ELSEVIER, AMSTERDAM, NL, vol. 503, no. 1, 6 June 2018 (2018-06-06), pages 51 - 55, XP085965405, ISSN: 0006-291X, [retrieved on 20180606], DOI: 10.1016/J.BBRC.2018.05.170
- NGUYEN M T ET AL: "Gene expression alterations in cryptorchid males using spermatozoal microarray analysis", FERTILITY AND STERILITY, ELSEVIER, AMSTERDAM, NL, vol. 92, no. 1, 1 July 2009 (2009-07-01), pages 182 - 187, XP026542734, ISSN: 0015-0282, [retrieved on 20080808], DOI: 10.1016/J.FERTNSTERT.2008.05.043

- PHOEBE H LEONARD ET AL: "The Aurora A-HP1? pathway regulates gene expression and mitosis in cells from the sperm lineage", BMC DEVELOPMENTAL BIOLOGY, BIOMED CENTRAL LTD., LONDON, GB, vol. 15, no. 1, 29 May 2015 (2015-05-29), pages 23, XP021222418, ISSN: 1471-213X, DOI: 10.1186/S12861-015-0073-X
- CASSUTO N G ET AL: "A new real-time morphology classification for human spermatozoa: a link for fertilization and improved embryo quality", FERTILITY AND STERILITY, ELSEVIER, AMSTERDAM, NL, vol. 92, no. 5, 1 November 2009 (2009-11-01), pages 1616 - 1625, XP026761379, ISSN: 0015-0282, [retrieved on 20081105], DOI: 10.1016/J.FERTNSTERT.2008.08.088

Remarques:

Le document complet y compris le(s) table(s) de référence et le(s) listage(s) de séquence sont téléchargeables sur le site internet de l'OEB

**Description**

La Procréation Médicalement Assistée (PMA) ou l'Assistance Médicale à la Procréation (AMP) est l'ensemble de techniques et traitements médicaux destinés à obtenir une grossesse et la naissance d'un enfant en bonne santé.

Il existe des infertilités masculines, féminines ou mixtes (lorsqu'elles concernent les deux partenaires).

Un couple est considéré comme hypofertile s'il est dans l'incapacité de concevoir d'enfant après 12 mois de relations sexuelles non protégées.

Dans certains cas, cette incapacité de concevoir est liée à une altération de la qualité du sperme, à un trouble de l'ovulation ou encore à un problème mécanique chez la femme. Parfois, l'infertilité reste inexpliquée.

Concrètement, la PMA regroupe de nombreuses techniques, telles que la congélation de sperme ou d'ovules, l'insémination artificielle (ou IAC, technique simple et peu coûteuse), la fécondation *in vitro* (ou FIV).

La FIV classique est la première technique *in vitro* permettant d'obtenir une grossesse. Elle consiste, le jour de la ponction ovocytaire J0, à mettre en présence les spermatozoïdes et les ovocytes pour obtenir une fécondation à J1 puis un embryon à partir de J2. Les embryons obtenus sont alors transférés dans l'utérus au moyen d'un cathéter sous contrôle échographique, deux, trois ou cinq jours après la fécondation.

Il existe une technique plus particulièrement préconisée dans l'infertilité d'origine masculine ou après un échec en FIV classique. Elle consiste à injecter sous microscope, un spermatozoïde directement dans l'ovocyte.

La fécondation *in vitro* par ICSI ("*IntraCytoplasmic Sperm Injection*") représente désormais plus des deux tiers des FIV et présente de très bons taux de succès. Cette technique repose notamment sur le fait de sélectionner le « bon » spermatozoïde à injecter. Cette sélection passe par l'identification d'indicateurs morphologiques capable de signaler la défectuosité d'un spermatozoïde. L'IMSI (pour « *Intracytoplasmic Morphologically Selected sperm Injection* ») par exemple, est une technique de fécondation *in vitro* dans laquelle la sélection du spermatozoïde destiné à être micro-injecté est effectuée en examinant leur morphologie à un fort grossissement (plus de x6000 contre un grossissement de x400 lors d'une ICSI). Elle permet en effet de ne pas retenir pour l'injection des spermatozoïdes dont la morphologie altérée n'est pas décelable au grossissement x400 (« faible grossissement »). Cette technique permet d'être plus efficace qu'une ICSI pour des spermes altérés.

Le principal inconvénient de la sélection d'un spermatozoïde à fort grossissement est le facteur temps, car il faut compter entre une heure et trois heures selon l'échantillon de sperme afin de sélection les « bons » spermatozoïdes. De plus, l'appréciation étant effectuée par un biologiste, il y a également un facteur humain, du fait d'une certaine subjectivité dans l'appréciation. De ce fait, cette technique de sélection reste chronophage avec un coût relativement élevé et dont les indications sont mal définies. Pourtant, dans certaines indications d'infertilité masculine, cette technique permet non seulement un meilleur taux de grossesse mais également une diminution des malformations majeures chez le foetus.

Le travail des inventeurs a visé à pallier les inconvénients ci-avant en proposant une méthode de sélection des spermatozoïdes, qui permette de définir les indications dans lesquels l'identification du spermatozoïde à injecter doit être effectué au fort grossissement.

Décrit est donc un procédé d'analyse d'un spermatozoïde, comprenant les étapes suivantes :

a) extraction des acides nucléiques des spermatozoïdes contenus dans un premier échantillon de spermatozoïdes préalablement obtenu auprès d'un sujet humain ;
b) mesure du niveau d'expression d'au moins un gène marqueur choisi parmi le groupe constitué par AURKA, CFAP46, CCDC60, CCDC88B, HDAC4, CACNA1C, CACNA1H, CARHSP1, DNAH2 et SPATA18 ou un gène homologue de ceux-ci, à partir des acides nucléiques extraits; et
c) détermination de l'existence d'un différentiel d'expression du au moins un gène marqueur par rapport à un contrôle.

Un spermatozoïde est une cellule reproductrice mâle, comprenant une tête (comportant notamment le noyau) et un moyen de locomotion appelé flagelle.

Par « acide nucléique », on désigne de l'ADN, de l'ARN, des séquences nucléiques codantes ou non codantes.

La détermination du niveau d'expression du gène peut s'effectuer par toute méthode connue de l'homme du métier.

De préférence, les échantillons de sperme et de spermatozoïdes mis en oeuvre dans les procédés selon l'invention sont des échantillons préparés par centrifugation en double gradient de densité. Une telle méthode est plus amplement détaillée à l'exemple 2.

Par « homologue », on vise une séquence de polynucléotides présentant un degré d'identité d'au moins 90%, préférentiellement au moins 95%, et encore plus préférentiellement de 99% avec la séquence du gène (de longueur totale) sauvage. Le degré d'identité fait référence à une identité de séquence entre deux séquences. L'identité peut être déterminée en comparant une position dans chaque séquence qui peut être alignée à des fins de comparaison. Quand une position équivalente dans les séquences comparées est occupée par la même base, alors les molécules sont identiques à cette position. Divers algorithmes et / ou programmes d'alignement peuvent être utilisés pour la détermination de l'homologie de deux séquences, dont FASTA et BLAST.

De préférence, le au moins un gène marqueur est choisi parmi le groupe constitué par AURKA, CFAP46, CCDC60, CCDC88B, HDAC4, CACNA1C, CACNA1H, CARHSP1, DNAH2 et SPATA18.

Plus préférentiellement, le au moins un gène marqueur est AURKA ou un gène homologue de celui-ci, encore plus préférentiellement AURKA.

L'invention vise un procédé de sélection d'un spermatozoïde, comprenant le procédé d'analyse décrit ci-dessus suivie de l'étape suivante :

d) sélection du spermatozoïde au sein d'un second échantillon de spermatozoïdes préalablement obtenu auprès d'un sujet humain : si il existe un différentiel d'expression, la sélection du spermatozoïde est réalisée par observation à un grossissement supérieur à x5000; si il n'existe pas de différentiel d'expression, la sélection du spermatozoïde est réalisée par observation à un grossissement inférieur à x500.

Le procédé de sélection est un procédé in vitro. Il permet de choisir un spermatozoïde apte à féconder un ovule, c'est-à-dire un spermatozoïde mobile présentant peu ou pas d'altération de sa morphologie.

Un tel spermatozoïde présente un score de 5 ou 6 selon la classification HVB (également appelée classification Cassuto Barak, voir notamment l'article « A new real-time morphology classification for human spermatozoa: a link for fertilization and improved embryo quality » par NG Cassuto et al., Fertility and Sterility, Vol. 92, No. 5, pages 1616-1625, Novembre 2009).

Le score HVB correspond à l'évaluation et la classification de la morphologie de la tête spermatique au fort grossissement optique (tel que x6100) et prend en compte trois éléments de la tête spermatique : la forme de la tête (H= « Head » ou Tête), la présence d'une vacuole dans le noyau (V=Vacuole) et la base de la tête (B= « Basis » ou Base).

La formule pour déterminer le score est comme suit :

- + 2 points pour une tête normale dans sa taille et dans sa forme, selon les critères de l'OMS (World Health Organization, « Laboratory manual for the examination of human semen and sperm-cervical mucus interaction », 4th ed. Cambridge: Cambridge University Press, 1999).

Autrement dit, par « tête normale dans sa taille et dans sa forme », on entend une tête de forme ovale avec un contour régulier; de longueur de 3 à 5 mm, de largeur de 2 à 3 mm ;

- + 3 points pour une tête sans vacuole, et
- + 1 point pour une base normale dans sa taille et dans sa forme, c'est-à-dire qu'elle est dans le prolongement de l'ovale de la tête.

Les spermatozoïdes de haut score, score 5 et 6, ont une morphologie normale tandis que les spermatozoïdes de bas score (score de 0) sont caractérisés par une morphologie de leur tête et de leur base anormale dans leurs tailles et dans leurs formes et la présence d'au moins une vacuole.

L' (Les) échantillon(s) de spermatozoïdes peu(ven)t être issu(s) d'un éjaculat préalablement obtenu auprès d'un sujet humain. Le sujet humain est de préférence un homme souffrant d'infertilité ou d'une impossibilité à concevoir un enfant tel que par exemple un homme souffrant d'azoospermie obstructive partielle, d'oligozoospermie, d'asthénozoospermie et/ou de tératozoospermie ou de toutes autres indications ayant pour conséquence une infertilité ou une impossibilité à concevoir un enfant.

Plus particulièrement, à l'étape d), la sélection est effectuée « par observation », c'est-à-dire qu'elle est effectuée par microscopie optique ou photonique.

Par « grossissement », on vise le grossissement de l'appareil optique (microscope) qui permet d'effectuer l'observation. Il est obtenu en multipliant les grossissements individuels des divers éléments de l'appareil optique (objectif, oculaire, zoom, etc.). Il est noté sous la forme « xY», avec Y le grossissement utilisé.

De préférence, lorsque la sélection est effectuée à un grossissement supérieur à x5000, ce grossissement est préférentiellement supérieur à x5500, plus préférentiellement supérieur à x6000, tel que par exemple, x6100 ou x6300.

Le grossissement supérieur à x5000 est généralement obtenu avec un objectif (x100), un oculaire (x10), une lentille effectuant un premier grossissement (x1.5) puis un zoom dont le grossissement permet d'obtenir le grossissement final désiré. De préférence, l'objectif est un objectif sous huile à immersion.

De préférence, lorsque la sélection est effectuée à un grossissement supérieur à x5000, elle est effectuée avec un microscope inversé équipé d'optique à contraste interférentiel Nomarski.

Un microscope inversé est un microscope optique dont l'échantillon est illuminé par le dessus et observé par en dessous.

Par « optique à contraste interférentiel Nomarski », on entend une optique avec un contraste interférentiel différentiel (CID). C'est une technique utilisée en microscopie, tant par réflexion que par transmission, pour faire apparaître, à l'instar du contraste de phase, des contrastes dans des objets microscopiques sans coloration ou préparation spécifique.

De préférence, lorsque la sélection est effectuée à un grossissement inférieur à x500, ce grossissement est préférentiellement inférieur à x450, tel que par exemple, x200 à x400. De préférence, lorsque la sélection est effectuée à un grossissement inférieur à x500, elle est effectuée avec un microscope inversé simple, tel que par exemple muni d'un objectif (x40) et d'un oculaire (x10).

Décrit est également un procédé permettant d'évaluer la qualité d'un sperme, comprenant le procédé d'analyse selon l'invention, suivie de l'étape suivante :

e) si un différentiel d'expression est établi à l'étape c), alors le sperme comporte au moins 90% de spermatozoïdes ayant une morphologie altérée ; s'il n'est pas établi de différentiel d'expression à l'étape c), alors le sperme comporte au moins 5% de spermatozoïdes ayant une morphologie non altérée.

Par « spermatozoïde comportant une morphologie altérée », on vise un spermatozoïde présentant un score 0 selon la classification HVB.

Par « spermatozoïde comportant une morphologie non altérée », on vise un spermatozoïde présentant un score de 5 ou 6 selon la classification HVB.

De préférence, s'il n'est pas établi de différentiel d'expression à l'étape c), alors le sperme comporte au moins 5% de spermatozoïdes ayant une morphologie non altérée et moins de 50% de spermatozoïdes comportant une morphologie altérée.

Avantageusement, dans les procédés selon l'invention, à l'étape b), le niveau d'expression du ou des gène(s) marqueur est mesuré par le niveau de la transcription de l'ARN ou de l'ADNc dudit gène.

De préférence, le niveau d'expression est mesuré par amplification des acides nucléiques par PCR, plus préférentiellement encore par le nombre de cycle(s) (Cp) par PCR en temps réel.

Avantageusement, dans les procédés selon l'invention, à l'étape b), outre le niveau d'expression du au moins gène, le niveau d'expression d'au moins un gène de référence est effectué.

Par « gène de référence », on vise un gène dont le niveau d'expression ne varie pas entre un spermatozoïde de score 0 et un spermatozoïde de score 6.

De préférence, le gène de référence est choisi parmi B2M, PRM1.

De préférence, la mesure du niveau d'expression d'au moins deux gènes de références est réalisée, plus préférentiellement, B2M et/ou PRM1.

Avantageusement, le niveau d'expression (Cp) moyen des deux gènes de référence est calculé comme suit :

$$\text{Cp moyen GRs} = \frac{1}{2} \times (\text{Cp moyen B2M} + \text{Cp moyen PRM1})$$

La mesure du ou des gènes de références permet l'obtention d'un niveau d'expression normalisé.

De préférence, la normalisation du niveau d'expression (Cp) du gène marqueur mesuré à l'étape b) par rapport à un niveau d'expression du au moins un gène de référence est effectuée selon :

$$\Delta\text{Cp}_{GM} = [\text{Cp}_{GM}] - [\text{Cp}_{GR}]$$

Où GM représente le gène marqueur et GR représente le au moins un gène de référence. De préférence, le contrôle de l'étape c) est le niveau d'expression dudit au moins un gène marqueur, mesuré dans un échantillon de spermatozoïdes comportant une morphologie non altérée.

Le contrôle peut être déterminé par la même méthode que celle du niveau d'expression du gène marqueur, préférentiellement par PCR, plus préférentiellement encore par le nombre de cycle(s) (Cp) par PCR en temps réel.

Avantageusement, pour chaque gène marqueur, le contrôle est donné par la valeur de ΔCpGM contrôle suivante :

| | $[\Delta Cp_{GM}]$ contrôle |
|---|---|
| *AURKA* | **8,83** |
| *CACNA1C* | **11,05** |
| *CACNA1H* | **6,84** |
| *CARHSP1* | **1,04** |
| *CCDC60* | **7,42** |
| *CCDC88B* | **10,61** |
| *CFAP46* | **9,55** |
| *DNAH2* | **8,78** |
| *HDAC4* | **9,73** |
| *SPATA18* | **3,33** |

### Tableau 1 : Valeurs ΔCp de contrôle pour chaque gène

De préférence, le différentiel entre le niveau d'expression du gène marqueur issu des acides nucléiques extraits de l'échantillon de spermatozoïdes préalablement obtenu auprès d'un sujet humain et le contrôle, est une sous-expression du gène marqueur.

Avantageusement, à l'étape b), la mesure du niveau d'expression (Cp) de chaque gène est effectuée par PCR quantitative en temps réel (qPCR) ; et à l'étape c), pour un gène marqueur, le différentiel d'expression est calculé selon les étapes suivantes :

i) normalisation du niveau d'expression (Cp) du gène marqueur mesuré à l'étape b) par rapport à un niveau d'expression d'un gène de référence selon

$$\Delta Cp_{GM} = [Cp_{GM}] - [Cp_{GR}]$$

où GM représente le gène marqueur et GR représente le gène de référence ; puis

ii) détermination du différentiel d'expression $\Delta\Delta Cp_{GM}$ selon

$$\Delta\Delta Cp_{GM} = [\Delta Cp_{GM}] - [\Delta Cp_{GM}]_{contrôle}$$

Par « chaque gène », on vise notamment :

- un gène marqueur issu des acides nucléiques extraits de l'échantillon de spermatozoïdes préalablement obtenu auprès d'un sujet humain,
- au moins un gène de référence.

Avantageusement, à l'étape b), pour chaque gène, le niveau d'expression est un niveau d'expression moyen obtenu à partir d'une moyenne d'au moins deux mesures de niveau d'expression (Cp).

A titre d'exemple, le Cp moyen pour chaque gène peut être calculé selon

$$Cp\ moyen = \frac{1}{n}\sum_{i=1}^{n}(Cp)$$

Avec n le nombre de mesure(s) de Cp pour un gène donné, n étant supérieur ou égal à 2, de préférence compris entre 2 et 4, de préférence, 3.

Dans la présente demande, les gammes de valeurs s'entendent bornes incluses. Avantageusement, à l'étape b), le niveau d'expression d'un gène de référence est :

- le niveau d'expression moyen dudit gène de référence, ou
- un niveau d'expression moyen des gènes de référence calculé selon

$$\textbf{Cp moyen GRs} = \frac{1}{k} \sum_{i=1}^{k} (\textbf{Cp moyen GRi})$$

Lorsque le niveau d'expression k gènes de référence est mesuré, k étant supérieur ou égal à 2.

De préférence, k est égale à 2.

De préférence, et comme indiqué précédemment, la mesure du niveau d'expression d'au moins deux gènes de références est réalisé, plus préférentiellement, la mesure du niveau d'expression des gènes de référence B2M et PRM1.

Dans ce cas, le niveau d'expression moyen des gènes de référence B2M et PRM1 est calculé comme suit :

$$\textbf{Cp moyen GRs} = \frac{1}{2} \, \text{x} \, (\, \textbf{Cp moyen B2M} + \, \textbf{Cp moyen PRM1})$$

Avantageusement, l'existence d'un différentiel d'expression est établie lorsqu'un différentiel d'expression d'au moins 10% est déterminé entre le niveau d'expression du au moins un gène extrait de l'échantillon de spermatozoïdes et le contrôle.

De préférence, le différentiel est d'au moins 12%, plus préférentiellement d'au moins 15%. Avantageusement, dans les procédés selon l'invention, on mesure à l'étape c) le niveau d'expression d'au moins deux gènes, avantageusement de 5, et préférentiellement de 10 gènes.

Par au moins deux gènes, on vise AURKA et CFAP46, AURKA et CCDC60, AURKA et CCDC88B, AURKA et HDAC4, AURKA et CACNA1C, AURKA et CACNA1H, AURKA et CARHSP1, AURKA et DNAH2, AURKA et SPATA18, CFAP46 et CCDC60, CFAP46 et CCDC88B, CFAP46 et HDAC4, CFAP46 et CACNA1C, CFAP46 et CACNA1H, CFAP46 et CARHSP1, CFAP46 et DNAH2, CFAP46 et SPATA18, CCDC60 et CCDC88B, CCDC60 et HDAC4, CCDC60 et CACNA1C, CCDC60 et CACNA1H, CCDC60 et CARHSP1, CCDC60 et DNAH2, CCDC60 et SPATA18, CCDC88B et HDAC4, CCDC88B et CACNA1C, CCDC88B et CACNA1H, CCDC88B et CARHSP1, CCDC88B et DNAH2, CCDC88B et SPATA18, HDAC4 et CACNA1C, HDAC4 et CACNA1H, HDAC4 et CARHSP1, HDAC4 et DNAH2, HDAC4 et SPATA18, CACNA1C et CACNA1H, CACNA1C et CARHSP1, CACNA1C et DNAH2, CACNA1C et SPATA18, CACNA1H et CARHSP1, CACNA1H et DNAH2, CACNA1H et SPATA18, CARHSP1 et DNAH2, CARHSP1 et SPATA18, DNAH2 et SPATA18, de préférence AURKA et CFAP46.

Par au moins cinq gènes, on vise de préférence AURKA, CFAP46, CCDC60, HDAC4 et CCDC88B.

Par au moins dix gènes, on vise de préférence AURKA, CFAP46, CCDC60, HDAC4, CCDC88B, CACNA1C, CACNA1H, CARHSP1, DNAH2 et SPATA18.

Si la méthode met en évidence à la fois l'existence de différentiels pour un ou plusieurs gènes et l'absence de différentiels pour un ou plusieurs gènes, alors l'analyse sera considérée comme non significative.

L'invention vise enfin l'utilisation *in vitro* d'au moins un gène choisi parmi le groupe constitué par les gènes *AURKA, CFAP46,* CCDC60, CCDC88B, *HDAC4, CACNA1C, CACNA1H, CARHSP1, DNAH2* et *SPATA18* ou des gènes homologues, ou de la combinaison des 10 gènes ou des gènes homologues, pour la détermination de la méthode de sélection visuelle d'un spermatozoïde , effectuée par microscopie optique ou photonique.

Décrit est également l'utilisation *in vitro* d'au moins un gène choisi parmi le groupe constitué par les gènes *AURKA, CFAP46,* CCDC60, CCDC88B, *HDAC4, CACNA1C, CACNA1H, CARHSP1, DNAH2* et *SPATA18* ou des gènes homologues, ou de la combinaison des 10 gènes ou des gènes homologues, pour la détermination de la qualité d'un sperme.

La présente invention est illustrée, de manière non limitative, par les exemples ci-après ainsi que la figure suivante :

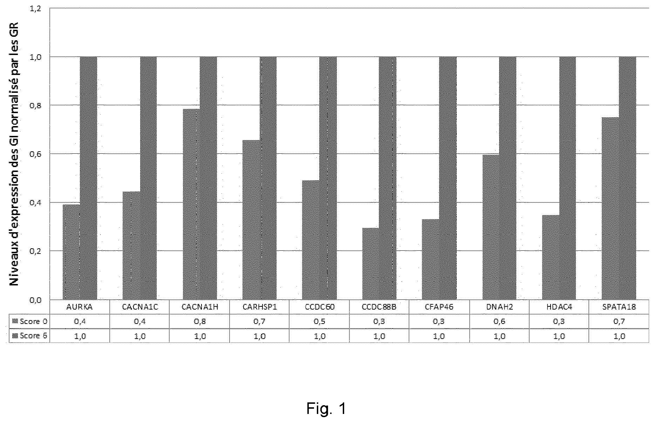

- La figure 1 représente un histogramme du niveau d'expression des gènes d'intérêts *AURKA, CFAP46,* CCDC60, CCDC88B, *HDAC4, CACNA1C, CACNALH, CARHSP1, DNAH2* et *SPATA18.* Leurs niveaux d'expression sont normalisés par deux gènes de références (invariants), PRM1 et B2M. Le graphique représente le ratio du niveau d'expression de ces gènes d'intérêts pour les spermatozoïdes de score d'échantillon 0 rapporté au niveau d'expression de ces mêmes gènes pour des spermatozoïdes de score d'échantillon 6.

**Exemple 1 : Identification d'une corrélation entre la morphologie des spermatozoïdes et l'expression d'un ensemble de gènes**

**1.1 Description de l'étude**

On a souhaité mesurer l'expression relative de gènes par qPCR, en utilisant 10 échantillons de sperme humain divisés en 2 groupes, dont le score HVB pour la qualité du sperme est respectivement 0 et 6.

Cette étude a permis de sélectionner 12 gènes (10 gènes d'intérêt et 2 gènes de référence). Les profils d'expression des gènes ont été mesurés par PCR en temps réel pour les 10 (2 groupes de 5) hommes.

Des échantillons de sperme des 10 hommes recueillis par masturbation, préparé par centrifugation en double gradient de densité et conditionnés dans du réactif Qiazol ont été reçus et conservés à -80°C. Les tubes ont été identifiés par leur « n° tube » ; ces identifiants uniques ont été utilisés pendant toute la durée du processus afin de garantir la confidentialité des données personnelles du donneur.

**Tableau 2** : **Score des spermes**

| Donneur | N° tube échantillon de sperme | Scores HVB* | Score de l'échantillon** |
|---------|-------------------------------|-------------|--------------------------|
| 1 | 01 | 0/10/90 | 0 |
| 2 | 02 | 10/65/25 | 6 |
| 3 | 03 | 0/10/90 | 0 |
| 4 | 04 | 0/10/90 | 0 |
| 5 | 05 | 0/10/90 | 0 |
| 6 | 06 | 15/65/20 | 6 |
| 7 | 07 | 5/65/30 | 6 |
| 8 | 08 | 10/60/30 | 6 |
| 9 | 09 | 10/60/30 | 6 |
| 10 | 10 | 0/10/90 | 0 |

\* Les scores HVB pour un échantillon de sperme sont présentés comme suit : % de spermatozoïdes de score 6-5 / % de spermatozoïdes de score 4-3-2-1 / % de spermatozoïdes de score 0.

\** Le score de l'échantillon a été attribué comme suit :

Les spermes des hommes ayant un score d'échantillon de 0 ont au moins 90% de spermatozoïdes de score 0.

Les spermes des hommes ayant un score d'échantillon de 6 ont au moins 5% de spermatozoïdes de score 5 et 6 et moins de 50% de spermatozoïdes de score 0, préférentiellement moins de 30%.

Les gènes étudiés sont les suivants :

**Tableau 3 : Gènes étudiés**

| Gène d'intérêt (GI) | Nom du gène |
|---------------------|-------------|
| SPATA18 | spermatogenesis associated 18 |
| CCDC60 | coiled-coil domain containing 60 |
| CACNA1H | calcium voltage-gated channel subunit alpha1 H |
| CCDC88B | coiled-coil domain containing 88B |
| DNAH2 | dynein axonemal heavy chain 2 |
| CACNA1C | calcium voltage-gated channel subunit alpha1 C |
| CARHSP1 | calcium regulated heat stable protein 1 |

(suite)

| Gène d'intérêt (GI) | Nom du gène |
|---|---|
| HDAC4 | histone deacetylase 4 |
| CFAP46 | cilia and flagella associated protein 46 |
| AURKA | aurora kinase A |

**1.2 Traitement des échantillons : préparation de l'ARN**

L'extraction de l'ARN des 10 échantillons a été réalisée à l'aide du miRNeasy Kit (QIAGEN) en suivant le protocole du constructeur. En bref, chaque échantillon de sperme a été lysé dans 700 μL de réactif de lyse QIAzol® dans un tube de micro centrifugation SafeLock de 2 mL contenant une perle inoxydable de 5 mm Chaque échantillon a été ensuite broyé en utilisant l'appareil Qiagen TissueLyzer pendant 2 x 2 minutes à 20 Hz. L'homogénat a été mis à incuber sur le plan de travail à la température ambiante (15-25 °C) pendant 5 min. 140 μL de chloroforme ont été ajoutés à l'homogénat afin d'obturer le tube. Le tube a été agité vigoureusement pendant 15 secondes et replacé sur le plan de travail pendant encore 2-3 minutes. Le lysat a été centrifugé à 12000 x g pendant 15 min à 4 °C dans une micro centrifugeuse. La phase aqueuse supérieure (environ 350 μL) a été soigneusement transférée dans une micro centrifugeuse propre de 2 ml.

La suite de la procédure a été effectuée en suivant le protocole du fabricant sur un poste de travail automatisé (QIAcube - QIAGEN), conformément au protocole préinstallé, afin d'optimiser la reproductibilité et la normalisation de l'extraction de miARN. À la fin de la procédure, chaque ARN a été élué dans 30 μL d'$H_2O$ ultra-pur. Une partie aliquote de 3 μL a été introduite à la pipette dans un nouveau tube à centrifuger pour une validation de la qualité et tous les tubes ont été immédiatement conservés au congélateur à température ultra-basse (-80 °C). On obtient ainsi 10 échantillons d'ARN.

**1.3 Analyse de l'expression relative des gènes**

**1.3.1 Préparation des modèles d'ADNc**

Les modèles d'ADNc ont été préparées à l'aide du kit de synthèse d'ADNc ReadyScript (Sigma-Aldrich) à partir de 100 ng de matrice d'ARN totale selon le protocole du fabricant. Le mélange maître d'ADNc ReadyScript contient un mélange d'amorces aléatoires et oligo(dT) dans un rapport spécifique pour assurer une représentation optimale de toutes les séquences de transcription dans le produit d'ADNc. La synthèse de l'ADNc a été réalisée en utilisant des plaques à 96 puits dans un volume réactionnel final de 20μL en utilisant le thermocycleur C1000 (Bio-Rad) selon le programme thermique suivant : 5 minutes à 25°C, 30 minutes à 42°C, 5 minutes à 85°C.

Les matrices d'ADNc ont été diluées à 1/11e dans 0,1X TE avant les expériences de qPCR.

**1.3.2 Expériences de qPCR TaqMan en temps réel**

Les expériences de qPCR ont été réalisées à l'aide de tests d'expression génique TaqMan (TaqMan Gene Expression Assays, Applied Biosystems) sur LightCycler480 (Roche Diagnostics). Les gènes PRM1 et B2M ont été utilisés comme gènes de référence (GR).

**Tableau 4 : Liste des identifiants TaqMan**

| Gène d'intérêt/ de référence | Identifiant "TaqMan Gène Expression Assay" |
|---|---|
| SPATA18 | Hs01102818_m1 |
| CCDC60 | Hs00905317_m1 |
| CACNA1H | Hs01103527_m1 |
| CCDC88B | Hs00989955_g1 |
| DNAH2 | Hs01044842_m1 |
| CACNA1C | Hs00167681_m1 |
| CARHSP1 | Hs00183933_m1 |

(suite)

| Gène d'intérêt/ de référence | Identifiant "TaqMan Gène Expression Assay" |
|---|---|
| HDAC4 | Hs01041648_m1 |
| CFAP46 | Hs00929098_m1 |
| AURKA | Hs01582072_m1 |
| B2M | Hs00187842_m1 |
| PRM1 | Hs00358158_g1 |

Toutes les qPCR ont été réalisées en utilisant des plaques à 384 puits réparties en 3 réplicats techniques, dans un volume réactionnel final de 10 μL, en utilisant le TaqMan FastAdvanced Master Mix (2X).

### 1.3.3 Programme de qPCR

Les cycles de qPCR consistent en 20 secondes à 95 ° C pour l'activation de l'enzyme, suivies de 45 cycles de 3 secondes pour la dénaturation et de 30 secondes pour l'annelage et l'extension avec mesure de la fluorescence. Les qPCR ont été réalisées sur la plate-forme LightCycler 480 (Roche). La procédure standard de Roche Diagnostics a été mise en place pour contrôler l'appareillage qPCR en temps réel. L'intensité de la lampe au xénon a été contrôlée avant chaque analyse.

### 1.4 Analyses statistiques

### 1.4.1 Validation des gènes de référence

Afin d'accroître la stabilité de l'expérience de qPCR, il est recommandé d'utiliser plusieurs gènes de référence afin d'obtenir une stabilité d'expression moyenne des gènes de référence en utilisant la méthode de Vandesompele des M-Values (Hellemans J et al. Genome Bio 2007). Dans cette méthode, un gène de référence est testé par rapport à l'autre gène de référence selon une variation par paires excluant le gène le moins stable de l'analyse. Le gène de référence le plus stable présente les M-values les plus basses. Les gènes de référence sont acceptés pour une stabilité telle que la M-value franchit le seuil de 0,25. Pour cette expérience, seuls deux gènes de référence ont été utilisés : la β2-microglobuline (B2M) et la protamine 1 (PRM1). L'expression des M-values pour ces gènes a été évaluée :

B2M : 0,14
PRM1 : 0,16
Ces valeurs se situent dans une plage raisonnable de M-values (<0,2) ; ces deux gènes sont donc des candidats solides pour les gènes de référence.

### 1.4.2 Test de spécificité par contrôles négatifs

Ce test de spécificité consiste à contrôler la possible réactivité croisée des amorces au cours du processus de PCR. Pour chaque gène, deux contrôles négatifs ont été utilisés pour mettre en évidence toute réactivité croisée des amorces, tels que la formation d'homo- ou d'hétérodimères, la contamination de la PCR, la contamination par ADN génomique, etc. :

- Un contrôle « NTC » (« No Template Control ») qui correspond à un mélange réactionnel de PCR, traité exactement de la même manière que les autres réactions de PCR en temps réel, mais dans lequel aucun ADNc n'a été ajouté. Ce contrôle permet de détecter toute contamination externe ou tout autre facteur pouvant entraîner une augmentation non spécifique du signal de fluorescence.
- Un contrôle « No RT » (No Reverse Transcriptase Control) qui correspond à un échantillon traité exactement de la même manière que les autres réactions de PCR en temps réel, mais dans lequel la matrice d'ADNc a été remplacée volontairement par une matrice d'ARN.

Chacun des 2 tests a été réalisé simultanément pour chacun des 12 gènes sur une plaque PCR à 384 puits. Pour chaque gène, les valeurs Cp (« Crossing Point ») des 2 contrôles négatifs « NTC » et « No RT » doivent être à 45 Cp, ou supérieures de 10 Cp à la valeur la plus élevée de Cp pour l'ARN correspondant.

Les contrôles NTC et No RT sont validés.

**1.4.3 Calculs de l'analyse d'expression relative des 10 gènes d'intérêt (GI) entre des spermes de score 0 et des spermes de score 6 et résultats**

Les calculs statistiques ont été effectués à partir des valeurs Delta.Cp (DCp) selon la formule décrite ci-dessous.
Les valeurs de Cp sont données pour chaque GI et GR et pour chaque donneur.
Les n=3 valeurs sont évaluées pour chaque donneur, pour chaque GI, GR et selon la formule décrite ci-dessous (Livak et al. 2001).
Pour chaque donneur, le Cp moyen de chaque gène de référence a été calculé comme suit : Le Cp moyen pour chacun des gènes, gènes de référence (GR) et gènes d'intérêt (GI), est calculé à l'aide de la formule suivante :

$$\text{Cp moyen} = \frac{1}{n} \sum_{i=1}^{n} (\text{Cp})$$

Le Cp moyen des 2 gènes de références (GR), pour chaque donneur, a été calculé comme suit :

$$\text{Cp moyen GRs} = \frac{1}{2} \times (\text{ Cp moyen B2M} + \text{ Cp moyen PRM1})$$

Le Delta.Cp est calculé pour chaque gène d'intérêt (GI) selon la formule suivante :

$$\Delta\text{Cp}_{\text{GI}} = [\text{Cp moyen}_{\text{GI}}] - [\text{Cp moyen GR}]$$

Les résultats statistiques sont les suivants :

| | Médiane* ΔCp score 0 | Médiane** ΔCp score 6 | Limite*** ΔCp |
|---|---|---|---|
| *AURKA* | 9,50 | 8,16 | **8,83** |
| *CACNA1C* | 12,03 | 10,07 | **11,05** |
| *CACNA1H* | 6,95 | 6,74 | **6,84** |
| *CARHSP1* | 1,10 | 0,99 | **1,04** |
| *CCDC60* | 7,62 | 7,23 | **7,42** |
| *CCDC88B* | 11,76 | 9,47 | **10,61** |
| *CFAP46* | 10,04 | 9,06 | **9,55** |
| *DNAH2* | 9,07 | 8,49 | **8,78** |
| *HDAC4* | 10,00 | 9,45 | **9,73** |
| *SPATA18* | 3,29 | 3,38 | **3,33** |

* Médiane statistique établie sur 5 patients ayant un échantillon de score 0.

** Médiane statistique établie sur 5 patients ayant un échantillon de score 6.

*** Limites : valeurs seuils.

## Tableau 5 : résultats statistiques des niveaux d'expression normalisé pour chaque gène

Afin de présenter les résultats sous la forme d'un diagramme comparatif, les calculs suivants ont été effectués : Le Delta.Delta.Cp ($\Delta\Delta$Cp) est calculé pour chaque GI selon la formule suivante :

$$\Delta\Delta Cp_{GI} = [\Delta Cp_{GI}]_{score\ 0} - [\Delta Cp_{GI}]_{score\ 6}$$

Le ratio est calculé pour chaque GI selon la formule suivante :

$$Ratio_{GI} = 2^{-(\Delta\Delta Cp)}$$

Pour faciliter la visualisation des résultats, la valeur arbitraire de 1 est attribuée aux valeurs de score 6.

Les résultats montrent une tendance générale à la sous-expression pour les 10 gènes (barre bleue) dans un échantillon marqué comme ayant un faible score HVB. Les résultats sont représentés à la figure 1.

Afin d'évaluer ces résultats préliminaires, pour chaque gène d'intérêt un test de Mann-Whitney a été utilisé. Le tableau ci-dessous montre les Pvalues données par ce test :

**Tableau 6 : Pvalues pour chaque gène**

| Nom du gène | Pvalue |
| --- | --- |
| AURKA | 0.004316 |
| CACNA1C | 0.3057 |
| CACNA1H | 0.6764 |
| CARHSP1 | 0.4697 |
| CCDC60 | 0.09758 |
| CCDC88B | 0.1028 |
| CFAP46 | 0.01651 |
| DNAH2 | 0.1815 |
| HDAC4 | 0.07368 |
| SPATA18 | 0.6764 |

En appliquant une erreur alpha de 5%, 2 gènes, AURKA et CFAP46 ont montré une Pvalue significative et 3 gènes ont présenté une Pvalue non significative mais toujours intéressante. Les diagrammes en boîtes de la figure 2 montrent les 5 gènes préférés pour discriminer les deux groupes de scores HVB.

## 2. Conclusion

Dans cette étude, l'analyse du niveau d'expression de 12 gènes a été réalisée par PCR en temps réel, en utilisant des échantillons de sperme prélevés chez 10 hommes.

Cette étude a permis d'identifier 12 gènes comme étant déterminants pour la qualité des spermatozoïdes des hommes.

Le niveau d'expression de ces gènes est corrélé avec une certaine morphologie précise et bien définie de la tête du spermatozoïde vivant, mobile au fort grossissement.

**Exemple 2 : Mise en oeuvre de la méthode de sélection d'un spermatozoïde en vue d'une PMA**

Un premier échantillon de sperme préalablement obtenu auprès d'un patient est traité comme suit :

1. Préparation par centrifugation en double gradient de densité

La migration des spermatozoïdes a été réalisée avec un gradient bicouche.

Dans un tube, 1 ml de spermatozoïdes totaux a été préparé sur un gradient de concentration à deux couches de 45% et 90% de milieu de séparation d'isolats de sperme, puis centrifugé à 300g pendant 15 min. Le surnageant a été jeté et le culot de sperme lavé avec un milieu de culture HAM'S supplémenté avec 10% Albumine sérique humaine (ASH) et centrifugé à 300g pendant 5 min. Le culot final du total des spermatozoïdes migrés a été remis en suspension dans 1 ml de culture HAM'S supplémenté avec 10% ASH.

2. Expression génique par qPCR

L'expression génique par qPCR est réalisée comme suit sur le culot final obtenu à l'étape précédente :

- Préparation de l'ARN comme cela est décrit au point 1.2 ci-avant,
- Réalisation d'une qPCR sur le ou les gènes marqueurs et les gènes de références comme cela est décrit aux points 1.3.2 et 1.3.3 ci-avant,
- Calcul des Cp moyens pour chaque gène,
- Calcul du Cp moyen GRs pour les gènes de références B2M et PRM1,
- Calcul des $\Delta$Cp pour chaque gène marqueur et comparaison avec les valeurs de $\Delta$CpGM de contrôle (Tableau 1) afin d'évaluer s'il existe un différentiel d'expression.

Si un différentiel d'expression (« positif ») est mis en évidence sur la base de l'analyse de l'expression génique effectuée sur le premier échantillon, la PMA est réalisée avec une sélection au fort grossissement des spermatozoïdes dans un second échantillon de sperme du même patient. Une telle manière de procéder permettra notamment de choisir un spermatozoïde avec une chromatine plus condensée, d'augmenter le taux de grossesse, de diminuer les malformations majeures et éventuellement de mettre en évidence un facteur génétique dans une infertilité masculine.

Si un différentiel d'expression (« positif ») n'est pas mis en évidence sur la base de l'analyse de l'expression génique effectuée sur le premier échantillon, la PMA est réalisée selon la méthode classique (faible grossissement).

**Séquences des gènes :**

>NM_145263.3 Homo sapiens spermatogenesis associated 18 **(SPATA18)**, transcript variant 1, mRNA

```
ACCCAGGGCGGGGCGGCGCGGGCGTTGCCACGACGCGGGCCGCGCGCGTCCCTGGCAGCCAACCCGTCCA
CGTCAAGGTTTGTTTAATAATCGCCAGGGTATCTATGGCCGGGCTCAGGCGGCTGCTGGGGAGCCAGGAG
ACCGCGCGGGACGGCGGATGAGGCGCGGCGGCTGCGGCCCAGGGCACCTCCCCTCTGGCTTCCCGAACCC
GGCCAGGTCCGACCCGAGGGGGAGGATGGAAACACCTGCCGCGCTCTGAGCCCCCCAGAAGAGAACACCC
TTCCCGCCATATCACCCCACGGTCCTGCGGAGGCCACCGCCTGGTCCCCCCAAGTCTCCATCGCGCAGCG
TGGGGCCGAGAGGAATAGTGAGCGATGGCGGAAAACCTGAAAAGACTGGTCTCAAACGAAACTTTACGAA
CGTTGCAGGAAAAGCTAGACTTCTGGCTGAAGGAGTACAACACAAACACGTGTGATCAAAATCTAAACCA
TTGCCTTGAACTCATTGAGCAAGTTGCCAAGGTGCAGGGACAACTCTTTGGGATCCTCACAGCAGCAGCC
CAAGAAGGAGGACGTAATGATGGTGTGGAAACAATCAAGTCACGCCTTTTGCCTTGGCTGGAGGCTTCCT
TTACTGCTGCTTCCCTGGGAAAATCTGTTGACAGCAAGGTCCCCTCTCTGCAGGACACGTTTGATAGGGA
GAGACATAAAGATCCCAGTCCTCGGGATCGGGATATGCAACAGTTAGACTCTAATTTGAACTCAACCCGG
AGTCAATGCAACCAGGTTCAAGACGATCTGGTTGAAACTGAAAAGAATCTTGAAGAAAGCAAGAACAGAT
CGGCCATATCCCTTTTGGCTGCAGAGGAGGAAATAAATCAGCTGAAAAAGCAGCTTAAATCTCTTCAAGC
TCAGGAGGATGCCCGCCACAGAAACACAGATCAGAGGAGCTCAGAGAATAGGCGGTCAGAGCCTTGGAGC
TTGGAGGAGCGGAAGCGTGAGCAGTGGAACTCACTCAAGCAGAATGCAGACCAGCAGGACACAGAAGCCA
TGTCCGATTATAAGAAACAGCTCCGAAACCTGAAGGAGGAGATAGCTGTTCTGTCTGCTGAGAAAAGTGC
ACTCCAAGGAAGGTCCTCCAGGAGCCGGTCTCCCAGCCCTGCCCCTCGCAGCCGTAGCTGCAGCCGCAGC
AGATCTGCCAGCCCCTCCACCGCTGTCAAGGTCAGGAGACCGTCCCCAAACCGCTCCAAGCTGTCCAATG
TGGCGCGCAAGGCTGCCCTCTTGTCCCGGTTCAGCGATTCCTATTCCCAGGCCCGCCTGGACGCGCAGTG
CCTGCTGCGGCGCTGCATCGACAAGGCTGAGACCGTTCAGCGGATCATCTACATCGCCACAGTGGAGGCA
TTCCATGTAGCAAAAATGGCATTCAGACACTTCAAGATCCATGTGAGAAAATCGTTGACACCATCTTATG
TGGGGTCGAATGACTTTGAGAATGCTGTCTTGGATTATGTCATTTGTCATCTTGATCTATATGATTCTCA
AAGCAGTGTCAATGATGTGATCCGAGCCATGAATGTCAATCCCAAGATTTCATTCCCTCCTGTCGTTGAC
TTTTGCCTTCTCAGTGACTTCATCCAGGAGATATGTTGCATTGCCTTTGCAATGCAGGCCTTAGAACCAC
CCCTAGATATTGCATATGGAGCAGATGGAGAAGTTTTTAATGATTGCAAATACCGCCGCAGCTACGACTC
GGATTTCACTGCTCCCTTAGTCCTCTATCACGTGTGGCCTGCTCTCATGGAGAATGACTGTGTCATTATG
AAGGGAGAAGCTGTCACCAGGAGAGGGGCTTTTTGGAATTCGGTGCGATCTGTAAGTCGTTGTCGAAGCA
GGAGTTTAAGTCCCATTTGCCCCCGTAGCCAAATTGGTTTAAACACGATGTCTCGAAGTCGGAGTCCTTC
TCCAATAAGATGTGGATTGCCAAGATTTTAAAAGCACCAGACCTGCTCCTTTGACCCAGTGCGTGGAAAC
AGCTGCTTTCTCCAGTGCCGCCATCTGTCTTCTGTGTCTGCCTCAGACCTCACTTAAGATAATGTCAAAA
GGCAATTCTGTGTATCACCCCACACAGAGAGTTAAATGTTTTGGCTTGGCGCATTTGTAACTTTAGATAT
ATTGCATTCTATTTTATTTTATAGATACTAATTCCATTAATTTCATAAAAATGATTGTATAGGCATTTAG
GATCATATTCATTCGAAGCAAAGTCCGTTACAAAGGTTCAAGATTTCCATCTCAAAACACTACGCTCTTT
TATGGGAACTGTGTGAACTGAAGTGGAAAGCATCTACCATGCTGAGGCTAAAAGAAAAGATGAATCATTT
TAGTTTGCAGATGGATCGTAAATATAATTGTTGGTATCAGCTTTAGCTCAAAACCAATATTAGGTGTTTT
AATTTCCTTTTAAGGTTTGGAAGACAGCCCTAATCTCAGGTTGGGGAGCTCATGTTAGTAGCAGTGACTT
AAGGCTAAGTGTAGAAGATAATTTAAGATACATTTTCTTTATATATTAGCCAACAAATTATATTTATTGG
TTGGCTTGCTTTTCCGTTCTGATTTTGAGAGTGCCCAGTTTGGTTTAGTTGACCAATGAATGTCAAAGCT
ACTTAGTTGAGAGAATTTCCTTGTTCATAAATGTAGAGCAGTGATTTGATTAGAAGCCAGCTTTGAGATA
AATGTTAATTACCTCATGCATATCTCCTGGGAATATTTCAAACTGTTTTAATGCATGTGTTATATATAAA
AGTTTCTTGGGACATGCTCTTCACCTGTTCTACCTAGTTATTTGCAAATTCAGACCTCCTATTGAACTCT
```

```
GTCTGACCAAAACTACTTAAACTCAAGGCCCAAAACTAGGGGCACCATTTACTGATTTTAAATTGAGTAT
ATATCCCTTGACTTCTTCACTGTCAAATACTTTTGAAACTTCACGTTCAAGATAAGAATGGAATGTTGCT
TTCTTGCAATAAGTAATGTTCTTTCTGCCTTTTTTTCACTTTTAAGTCAGCCTTAAACACATGCCTCACA
AACATCTACTTTCTCCACATACCTTTGAGAGAGACACTGAATTGGCCTCAGCTCAGTTTTGCATAAGCTT
AGTGCCAGAACCAGCACCTGATGCTTTTCAGGTGAAAATAAAACAAACAGCTTCTCTAAAGCATCTTACC
CCTGTGCTGGAGGTTTGAGGGACCTCTTCAGTGCCTGCCCCTTGAGTCTAATGGTCACCACCTCATTCTG
AAGTATGAGTTGAATTTTTTGCCCTCTTTGCATATTTACATTAGTCATCACTTTGAAGCAATGCAGTGTG
CTGGAAGGAGCACTATCTGCCTAGGTAACTGCTAGTCATGACTTGGTCATCAGCTTGCTTTGTGGCACTG
AGCAAGTTACTTCACTTCTCTGAGCCTTGGTTTTATCATAGGGTGAGGAGGTTGGATACAATTAGTGCCC
CTCTTAACCCTGCAGACTCAATGTTCCCTTTTATAACAAGTATTTTATTCTGAATATGAAATGAAAATTA
AAGTTAATATAATCATCTATGTGCATGTATAATTTTAAGCAGTGAACATAGTACCCTAACTATAATATAA
AGCAGAAAAAAGGCAACTTTTAATAAAATAAATGTATTTCAATAAAAAGCTTGGGTATAACCACCCT
AGAAGATAAATTAAGTCATTAGATGGCTGAACCTGCATGTAGAGCCACCAGCTACAAATGAAATCAAT
GTGTGTATTGGCAACAGAAATCACGGTGTTGTCATTGGATGTGACTTTCTGAAGTGGTGGGCAATTCTT
GTTAATGTTTTAAACAAAAAAAAAAAAACCTTACAGTCTTGCCCTGATTACACAGCAGTCACATTCCTG
GAAAATTCAAGTGTTTATTAAAACTATGCAACAGTTACTGTGTGTTATACGTTGAAAGGTCTTCACTAAT
ATCTCACTAAGTAATGAGAATGCCTACATATCAGAATTTTTTTTTTCAGGAGCCAAGCACATATACTGAT
TTGGAAAAAGGCACAGGTAGCTCAGTTTATTTGCTTTCTACCCTGCCTGGCCACTTGCTGTTTCTTCAGT
TTCTAATTTGAGCTGTAACTACACAAGGAAAGCTAAATAGTCTGGAAATTTTTGGAAAGAATCCACAAA
GCCAAAGGAGACTGGCCTATACTCATTTTATCTGGGGATGTACCTTACCCTTAGAGACTTTGAAAAATGT
GAAGCTCTTATTTTGTAACCTGGGTAAATGTTAGTTTCTAGATTTTCGGCTTAACATCTAATAATAACAT
TTAAAAAGTGCTTTTGTAACTATTAGTTATTTGCAATAAATGCTTTCCTTCTACAGTCCCAAGTTCAAA
AAAAAAAAAAAAAA
```

>NM_001297608.1 Homo sapiens spermatogenesis associated 18 **(SPATA18)**, transcript variant 2, mRNA

```
ACCCAGGGCGGGGCGGCGCGGGCGTTGCCACGACGCGGGCCGCGCGCGTCCCTGGCAGCCAACCCGTCCA
CGTCAAGGTTTGTTTAATAATCGCCAGGGTATCTATGGCCGGGCTCAGGCGGCTGCTGGGGAGCCAGGAG
ACCGCGCGGGACGGCGGATGAGGCGCGGCGGCTGCGGCCCAGGGCACCTCCCCTCTGGCTTCCCGAACCC
GGCCAGGTCCGACCCGAGGGGGAGGATGGAAACACCTGCCGCGCTCTGAGCCCCCCAGAAGAGAACACCC
TTCCCGCCATATCACCCCACGGTCCTGCGGAGGCCACCGCCTGGTCCCCCCAAGTCTCCATCGCGCAGCG
TGGGGCCGAGAGGAATAGTGAGCGATGGCGGAAAACCTGAAAAGACTGGTCTCAAACGAAACTTTACGAA
CGTTGCAGGAAAAGCTAGACTTCTGGCTGAAGGAGTACAACACAAACACGTGTGATCAAAATCTAAACCA
TTGCCTTGAACTCATTGAGCAAGTTGCCAAGGTGCAGGGACAACTCTTTGGGATCCTCACAGCAGCAGCC
CAAGAAGGAGGACGTAATGATGGTGTGGAAACAATCAAGTCACGCCTTTTGCCTTGGCTGGAGGCTTCCT
TTACTGCTGCTTCCCTGGGAAAATCTGTTGACAGCAAGGTCCCCTCTCTGCAGGACACGTTTGATAGGGA
GAGACATAAAGATCCCAGTCCTCGGGATCGGGATATGCAACAGTTAGACTCTAATTTGAACTCAACCCGG
AGTCAATGCAACCAGGTTCAAGACGAGCTTAAATCTCTTCAAGCTCAGGAGGATGCCCGCCACAGAAACA
CAGATCAGAGGAGCTCAGAGAATAGGCGGTCAGAGCCTTGGAGCTTGGAGGAGCGGAAGCGTGAGCAGTG
GAACTCACTCAAGCAGAATGCAGACCAGCAGGACACAGAAGCCATGTCCGATTATAAGAAACAGCTCCGA
AACCTGAAGGAGGAGATAGCTGTTCTGTCTGCTGAGAAAAGTGCACTCCAAGGAAGGTCCTCCAGGAGCC
GGTCTCCCAGCCCTGCCCCTCGCAGCCGTAGCTGCAGCCGCAGCAGATCTGCCAGCCCCTCCACCGCTGT
CAAGGTCAGGAGACCGTCCCCAAACCGCTCCAAGCTGTCCAATGTGGCGCGCAAGGCTGCCCTCTTGTCC
CGGTTCAGCGATTCCTATTCCCAGGCCCGCCTGGACGCGCAGTGCCTGCTGCGGCGCTGCATCGACAAGG
CTGAGACCGTTCAGCGGATCATCTACATCGCCACAGTGGAGGCATTCCATGTAGCAAAAATGGCATTCAG
ACACTTCAAGATCCATGTGAGAAAATCGTTGACACCATCTTATGTGGGGTCGAATGACTTTGAGAATGCT
GTCTTGGATTATGTCATTTGTCATCTTGATCTATATGATTCTCAAAGCAGTGTCAATGATGTGATCCGAG
CCATGAATGTCAATCCCAAGATTTCATTCCCTCCTGTCGTTGACTTTTGCCTTCTCAGTGACTTCATCCA
GGAGATATGTTGCATTGCCTTTGCAATGCAGGCCTTAGAACCACCCCTAGATATTGCATATGGAGCAGAT
GGAGAAGTTTTTAATGATTGCAAATACCGCCGCAGCTACGACTCGGATTTCACTGCTCCCTTAGTCCTCT
ATCACGTGTGGCCTGCTCTCATGGAGAATGACTGTGTCATTATGAAGGGAGAAGCTGTCACCAGGAGAGG
GGCTTTTTGGAATTCGGTGCGATCTGTAAGTCGTTGTCGAAGCAGGAGTTTAAGTCCCATTTGCCCCCGT
AGCCAAATTGGTTTAAACACGATGTCTCGAAGTCGGAGTCCTTCTCCAATAAGATGTGGATTGCCAAGAT
TTTAAAAGCACCAGACCTGCTCCTTTGACCCAGTGCGTGGAAACAGCTGCTTTCTCCAGTGCCGCCATCT
GTCTTCTGTGTCTGCCTCAGACCTCACTTAAGATAATGTCAAAAGGCAATTCTGTGTATCACCCCACACA
GAGAGTTAAATGTTTTGGCTTGGCGCATTTGTAACTTTAGATATATTGCATTCTATTTTATTTTATAGAT
ACTAATTCCATTAATTTCATAAAAATGATTGTATAGGCATTTAGGATCATATTCATTCGAAGCAAAGTCC
GTTACAAAGGTTCAAGATTTCCATCTCAAAACACTACGCTCTTTTATGGGAACTGTGTGAACTGAAGTGG
AAAGCATCTACCATGCTGAGGCTAAAAGAAAAGATGAATCATTTTAGTTTGCAGATGGATCGTAAATATA
ATTGTTGGTATCAGCTTTAGCTCAAAACCAATATTAGGTGTTTTAATTTCCTTTTAAGGTTTGGAAGACA
```

```
GCCCTAATCTCAGGTTGGGGAGCTCATGTTAGTAGCAGTGACTTAAGGCTAAGTGTAGAAGATAATTTAA
GATACATTTTCTTTATATATTAGCCAACAAATTATATTTATTGGTTGGCTTGCTTTTCCGTTCTGATTTT
GAGAGTGCCCAGTTTGGTTTAGTTGACCAATGAATGTCAAAGCTACTTAGTTGAGAGAATTTCCTTGTTC
ATAAATGTAGAGCAGTGATTTGATTAGAAGCCAGCTTTGAGATAAATGTTAATTACCTCATGCATATCTC
CTGGGAATATTTCAAACTGTTTTAATGCATGTGTTATATATAAAAGTTTCTTGGGACATGCTCTTCACCT
GTTCTACCTAGTTATTTGCAAATTCAGCCTCCTATTGAACTCTGTCTGACCAAAACTACTTAAACTCAA
GGCCCAAAACTAGGGGCACCATTTACTGATTTTAAATTGAGTATATATCCCTTGACTTCTTCACTGTCAA
ATACTTTTGAAACTTCACGTTCAAGATAAGAATGGAATGTTGCTTTCTTGCAATAAGTAATGTTCTTTCT
GCCTTTTTTTCACTTTTAAGTCAGCCTTAAACACATGCCTCACAAACATCTACTTTCTCCACATACCTTT
GAGAGAGACACTGAATTGGCCTCAGCTCAGTTTTGCATAAGCTTAGTGCCAGAACCAGCACCTGATGCTT
TTCAGGTGAAAATAAAACAAACAGCTTCTCTAAAGCATCTTACCCCTGTGCTGGAGGTTTGAGGGACCTC
TTCAGTGCCTGCCCCTTGAGTCTAATGGTCACCACCTCATTCTGAAGTATGAGTTGAATTTTTTGCCCTC
TTTGCATATTTACATTAGTCATCACTTTGAAGCAATGCAGTGTGCTGGAAGGAGCACTATCTGCCTAGGT
AACTGCTAGTCATGACTTGGTCATCAGCTTGCTTTGTGGCACTGAGCAAGTTACTTCACTTCTCTGAGCC
TTGGTTTTATCATAGGGTGAGGAGGTTGGATACAATTAGTGCCCCTCTTAACCCTGCAGACTCAATGTTC
CCTTTTATAACAAGTATTTTATTCTGAATATGAAATGAAAATTAAAGTTAATATAATCATCTATGTGCAT
GTATAATTTTAAGCAGTGAACATAGTACCCTAACTATAATATAAAGCAGAAAAAAAGGCAACTTTTAATA
AAATAAAATGTATTTCAATAAAAAGCTTGGGTATAACCACCCTAGAAGATAAAATTAAGTCATTAGATG
GCTGAACCTGCATGTAGAGCCACCAGCTACAAATGAAAATCAATGTGTGTATTGGCAACAGAAATCACG
GTGTTGTCATTGGATGTGACTTTCTGAAGTGGTGGGCAATTCTTGTTAATGTTTTAAACAAAAAAAAAAA
AACCTTACAGTCTTGCCCTGATTTACACAGCAGTCACATTCCTGGAAAATTCAAGTGTTTATTAAAACTA
TGCAACAGTTACTGTGTGTTATACGTTGAAAGGTCTTCACTAATATCTCACTAAGTAATGAGAATGCCTA
CATATCAGAATTTTTTTTTTCAGGAGCCAAGCACATATACTGATTTGGAAAAAGGCACAGGTAGCTCAGT
TTATTTGCTTTCTACCCTGCCTGGCCACTTGCTGTTTCTTCAGTTTCTAATTTGAGCTGTAACTACACAA
GGAAAGCTAAATAGTCTGGAAAATTTTTGGAAAGAATCCACAAAGCCAAAGGAGACTGGCCTATACTCAT
TTTATCTGGGGATGTACCTTACCCTTAGAGACTTTGAAAAATGTGAAGCTCTTATTTTGTAACCTGGGTA
AATGTTAGTTTCTAGATTTTCGGCTTAACATCTAATAATAACATTTAAAAAGTGCTTTTGTAACTATTAG
TTATTTGCAATAAATGCTTTCCTTCTACAGTCCCAAGTTCAAAAAAAAAAAAAAAAAA
```

>NM 178499.4 Homo sapiens coiled-coil domain containing 60 **(CCDC60)**, mRNA

AGTTGCCTACTTTCCCCGCAGTGCGATAAACCCCTCGTTGGGGCCGCCTTAGTTCTCGGCCGCTCTCGGA
GGATGGCGATCTGGGAGCCCCTCCATGGGACCCCTCTCACACTTTGTCACTGGAATTTTATTTATTTTTT
AGTTCATATTTTATTTTGCTTATAGAAGAGAAAGATATCCCTTCCGAAGAGGAGGAAGCTTACAGAAGTT
TATAACCTTTCAAAAAGTAAATAAGTCCAGGCTTGCCTGATTCTGCCCTACCCGGACTTCCTTATCCCGT
CTGTGGGAGACCCAGGTGCTTTCTCATTACTCTTCAGAAGGAAACCGCTTTGGAGTTCGTGTAATTGGGA
CTTGGGGATCAGGGAGAAGTTGCCGAAACTTCTCATACCAGTAACTACTGAAGTAGAAGATTCTGGAAAA
ATCCTTGTCTTGGGGGCACAGGCTAAAACCTGAAGGATTTTTAAGATGACCAAGGTTCCAGCCACCAAGA
AGCTTCAGAGTTCCCCCAACTCGGGGGCTGTCCGGCCCTTTTATGCCTCGGAGAACCTAAGGCAGGTCCC
AGACAAGCCAATGAAGAGCATCAAGTATATGGACAAGGAATAATAAACCTCAAAAAGGACCTTATACGA
AGCCGCTTTTTGATCCAGTCTGTGAAGATAGGCCGTGGATATTTTGCTATTCTGAGGGAAGAGACTGCAA
AGAAAAAGAAGCAACAACAACTTCAGAAACTGAAAGAGGAGGAAAGAAATAAATTCCAGCCAGCCGAAAA
GATCTCAGAAATCCACTATGGGGACACCTTATTGAGCACATATGATGATGAGAAGTTGAAGACACTGGGA
GCTAGAGTCACACGTCGCCCATTCACTCCCATCCACAGCTGCATCATTTCTCCCTCGCTAACCGAGGCTC
ACGTCGAGCCCTCTTCCGCCAGCTCTGTGCTCTCCACTGGCTTCTGGAGGCCCTGACTATTGACCACAC
CCACCACACCATGAAGCCTGTGATCACCTGCTGGAACCCAAAGGACCCGGGTGGAAGCAAGAGCACCATT
AAAAAAATCAATAAGGACAAGTCCATGGGACAGAAATGGGAGCATTTCATCACAGCGCCAAAGACCAAGA
AATTCAAAATTCCCACAATGCGAGTCACCAACCGCAAACCAAGCCGGCGAGGCTCCACACTCAGTCTGAG
TCGGGCCAGTGGGGGGTCCTCTCCCCAGAGCAGCATGATCTCTGTGAACCCTGGCTCGGATGAGCCCCCA
AGTGTGAACACCCAGGTGACCAGCAGCAAGGACATTGAGGACAATGAGTCATCTTCAACCAAGCCAGATG
AAGAACCTCTGTATATGAATCTGCAGAAGCTCCTGGAGATGGTTCGGGAAGATGCCCGGAGGACAGTCAC
AATAGAAATGGGATGCAAAGAAAGCACCCAGCATCTTGTCAGTGCTGAAACAAAACAAGAGTAATTCT
GCTTATAAGGAAATGCAGACCACTCTCAAATCAAGTGAGAGATCCAGCAGTACAAGTGCAGAAAGCCACA
TCCAACCAGTCCAGAAGAAGTCTAAAAACCGCACTAATTGTGACATCAACATCCACTACAAGAGTGGGGT
GTGTAACACCATGAGGGCCAAGTTTTACAGCGTAGCCCAGGAGGCTGGCTTCTGCCTGCAGGACAAGATG
GAAATCCTCATGAAGCGCCAAGAAGAGAGAGGTATCCAGAAGTTCCGTGCTTTTGTCCTTGTCTCAAATT
TTCAAAAGGACATAGCAAAATGAGACATCACATATCTGTAGTAAAAGGAGATGCAGAAGAAATTGCAGA
CCACTGGTACTTTGATCTGTTGTCCAAACTGCCAGAGGATCTAAAGAACTTCCGCCCCGCCAAAAAGATC
CTGGTGAAACTGCAGAAGTTTGGAGAAAACCTGGACTTGCGGATTCGACCCCATGTCCTCCTGAAGGTGC
TGCAGGATCTGAGGATTTGGGAACTGTGCTCCCCTGACATCGCTGTGGCTATTGAGTTTGTGCGAGAACA
CATCATCCATATGCCTCAAGAGGATTACATCAGCTGGCTGCAGAGCCGGATCAACATACCCATTGGGCCC

TACAGCGCCCTGAGGTAGGCTGGGCCTGGGTTGACCAGCTGTCTCAGTGGAGGAGTGTTTGCCTATATCA
TGTTCCTGTATCCTGCCTGTGTTCCTGCCTCCTGACTACCCTCATGGATGCTCTTTATGGATGACCCTTT
ACAGTAGGGTCATCTGGAGACTGACTTCCAGCAACATTTTTAGAGGGGGATGGCCCCGGTGGCCCTCCCC
TCAATTCCACACCCCAGACCCAACCTACCAGTCTCTGTTCTTCAATGATCCAGCCTGACTCTACCTACTT
CCTCTTCAGATTCCTTCACCCTATTTACCTTCCTCAAGTACTGGAGAATAAAATTGAACTGAATGTTTGA
AAAAA

>NM_001005407.1 Homo sapiens calcium voltage-gated channel subunit alphal H **(CACNA1H)**, transcript variant 2, mRNA

```
CGAGGCCGCCGCCGTCGCCTCCGCCGGGCGAGCCGGAGCCGGAGTCGAGCCGCGGCCGGGAGCCGGGCGG
GCTGGGGACGCGGGCCGGGGGCGGAGGCGCTGGGGGCCGGGGCCGGGGCCGGGGGCGGAGGCGCTGGGGG
CCGGGGCCGGGGCCGGGCGCCGAGCGGGGTCCGCGGTGACCGCGCCGCCCGGGCGATGCCCGCGGGGACG
CCGCCGGCCAGCAGAGCGAGGTGCTGCCGGCCGCCACCATGACCGAGGGCGCACGGGCCGCCGACGAGGT
CCGGGTGCCCCTGGGCGCGCCGCCCCCTGGCCCTGCGGCGTTGGTGGGGGCGTCCCCGGAGAGCCCCGGG
GCGCCGGGACGCGAGGCGGAGCGGGGGTCCGAGCTCGGCGTGTCACCCTCCGAGAGCCCGGCGGCCGAGC
GCGGCGCGGAGCTGGGTGCCGACGAGGAGCAGCGCGTCCCGTACCCGGCCTTGGCGGCCACGGTCTTCTT
CTGCCTCGGTCAGACCACGCGGCCGCGCAGCTGGTGCCTCCGGCTGGTCTGCAACCCATGGTTCGAGCAC
GTGAGCATGCTGGTAATCATGCTCAACTGCGTGACCCTGGGCATGTTCCGGCCCTGTGAGGACGTTGAGT
GCGGCTCCGAGCGCTGCAACATCCTGGAGGCCTTTGACGCCTTCATTTTCGCCTTTTTTGCGGTGGAGAT
GGTCATCAAGATGGTGGCCTTGGGGCTGTTCGGGCAGAAGTGTTACCTGGGTGACACGTGGAACAGGCTG
GATTTCTTCATCGTCGTGGCGGGCATGATGGAGTACTCGTTGGACGGACACAACGTGAGCCTCTCGGCTA
TCAGGACCGTGCGGGTGCTGCGGCCCCTCCGCGCCATCAACCGCGTGCCTAGCATGCGGATCCTGGTCAC
TCTGCTGCTGGATACGCTGCCCATGCTCGGGAACGTCCTTCTGCTGTGCTTCTTCGTCTTCTTCATTTTC
GGCATCGTTGGCGTCCAGCTCTGGGCTGGCCTCCTGCGGAACCGCTGCTTCCTGGACAGTGCCTTTGTCA
GGAACAACAACCTGACCTTCCTGCGGCCGTACTACCAGACGGAGGAGGGCGAGGAGAACCCGTTCATCTG
CTCCTCACGCCGAGACAACGGCATGCAGAAGTGCTCGCACATCCCCGGCCGCCGCGAGCTGCGCATGCCC
TGCACCCTGGGCTGGGAGGCCTACACGCAGCCGCAGGCCGAGGGGGTGGGCGCTGCACGCAACGCCTGCA
TCAACTGGAACCAGTACTACAACGTGTGCCGCTCGGGTGACTCCAACCCCCACAACGGTGCCATCAACTT
CGACAACATCGGCTACGCCTGGATTGCCATCTTCCAGGTGATCACGCTGGAAGGCTGGGTGGACATCATG
TACTACGTCATGGACGCCCACTCATTCTACAACTTCATCTATTTCATCCTGCTCATCATCGTGGGCTCCT
TCTTCATGATCAACCTGTGCCTGGTGGTGATTGCCACGCAGTTCTCGGAGACGAAGCAGCGGGAGAGTCA
GCTGATGCGGGAGCAGCGGGCACGCCACCTGTCCAACGACAGCACGCTGGCCAGCTTCTCCGAGCCTGGC
AGCTGCTACGAAGAGCTGCTGAAGTACGTGGGCCACATATTCCGCAAGGTCAAGCGGCGCAGCTTGCGCC
TCTACGCCCGCTGGCAGAGCCGCTGGCGCAAGAAGGTGGACCCCAGTGCTGTGCAAGGCCAGGGTCCCGG
GCACCGCCAGCGCCGGGCAGGCAGGCACACAGCCTCGGTGCACCACCTGGTCTACCACCACCATCACCAC
CACCACCACCACTACCATTTCAGCCATGGCAGCCCCGCAGGCCCGGCCCCGAGCCAGGCGCCTGCGACA
CCAGGCTGGTCCGAGCTGGCGCGCCCCCCTCGCCACCTTCCCCAGGCCGCGGACCCCCCGACGCAGAGTC
TGTGCACAGCATCTACCATGCCGACTGCCACATAGAGGGGCCGCAGGAGAGGGCCCGGGTGGCACATGCC
GCAGCCACTGCCGCTGCCAGCCTCAGACTGGCCACAGGGCTGGGCACCATGAACTACCCCACGATCCTGC
CCTCAGGGGTGGGCAGCGGCAAAGGCAGCACCAGCCCCGGACCCAAGGGGAAGTGGGCCGGTGGACCGCC
AGGCACCGGGGGGCACGGCCCGTTGAGCTTGAACAGCCCTGATCCCTACGAGAAGATCCCGCATGTGGTC
GGGGAGCATGGACTGGGCCAGGCCCTGGCCATCTGTCGGGCCTCAGTGTGCCCTGCCCCCTGCCCAGCC
CCCCAGCGGGCACACTGACCTGTGAGCTGAAGAGCTGCCCGTACTGCACCCGTGCCCTGGAGGACCCGGA
GGGTGAGCTCAGCGGCTCGGAAAGTGGAGACTCAGATGGCCGTGGCGTCTATGAATTCACGCAGGACGTC
CGGCACGGTGACCGCTGGGACCCCACGCGACCACCCCGTGCGACGGACACACCAGGCCCAGGCCCAGGCA
GCCCCCAGCGGCGGGCACAGCAGAGGGCAGCCCCGGGCGAGCCAGGCTGGATGGGCCGCCTCTGGGTTAC
CTTCAGCGGCAAGCTGCGCCGCATCGTGGACAGCAAGTACTTCAGCCGTGGCATCATGATGGCCATCCTT
GTCAACACGCTGAGCATGGGCGTGGAGTACCATGAGCAGCCCGAGGAGCTGACTAATGCTCTGGAGATCA
GCAACATCGTGTTCACCAGCATGTTTGCCCTGGAGATGCTGCTGAAGCTGCTGGCCTGCGGCCCTCTGGG
CTACATCCGGAACCCGTACAACATCTTCGACGGCATCATCGTGGTCATCAGCGTCTGGGAGATCGTGGGG
CAGGCGGACGGTGGCTTGTCTGTGCTGCGCACCTTCCGGCTGCTGCGTGTGCTGAAGCTGGTGCGCTTTC
TGCCAGCCCTGCGGCGCCAGCTCGTGGTGCTGGTGAAGACCATGGACAACGTGGCTACCTTCTGCACGCT
GCTCATGCTCTTCATTTTCATCTTCAGCATCCTGGGCATGCACCTTTTCGGCTGCAAGTTCAGCCTGAAG
ACAGACACCGGAGACACCGTGCCTGACAGGAAGAACTTCGACTCCCTGCTGTGGGCCATCGTCACCGTGT
TCCAGATCCTGACCCAGGAGGACTGGAACGTGGTCCTGTACAACGGCATGGCCTCCACCTCCTCCTGGGC
CGCCCTCTACTTCGTGGCCCTCATGACCTTCGGCAACTATGTGCTCTTCAACCTGCTGGTGGCCATCCTC
GTGGAGGGCTTCCAGGCGGAGGGCGATGCCAACAGATCCGACACGGACGAGGACAAGACGTCGGTCCACT
TCGAGGAGGACTTCCACAAGCTCAGAGAACTCCAGACCACAGAGCTGAAGATGTGTTCCCTGGCCGTGAC
CCCCAACGGGCACCTGGAGGGACGAGGCAGCCTGTCCCCTCCCCTCATCATGTGCACAGCTGCCACGCCC
ATGCCTACCCCCAAGAGCTCACCATTCCTGGATGCAGCCCCCAGCCTCCCAGACTCTCGGCGTGGCAGCA
```

```
GCAGCTCCGGGGACCCGCCACTGGGAGACCAGAAGCCTCCGGCCAGCCTCCGAAGTTCTCCCTGTGCCCC
CTGGGGCCCCAGTGGCGCCTGGAGCAGCCGGCGCTCCAGCTGGAGCAGCCTGGGCCGTGCCCCCAGCCTC
AAGCGCCGCGGCCAGTGTGGGGAACGTGAGTCCCTGCTGTCTGGCGAGGGCAAGGGCAGCACCGACGACG
AAGCTGAGGACGGCAGGGCCGCGCCCGGGCCCCGTGCCACCCCACTGCGGCGGGCCGAGTCCCTGGACCC
ACGGCCCCTGCGGCCGGCCGCCCTCCCGCCTACCAAGTGCCGCGATCGCGACGGGCAGGTGGTGGCCCTG
CCCAGCGACTTCTTCCTGCGCATCGACAGCCACCGTGAGGATGCAGCCGAGCTTGACGACGACTCGGAGG
ACAGCTGCTGCCTCCGCCTGCATAAAGTGCTGGAGCCCTACAAGCCCCAGTGGTGCCGGAGCCGCGAGGC
CTGGGCCCTCTACCTCTTCTCCCCACAGAACCGGTTCCGCGTCTCCTGCCAGAAGGTCATCACACACAAG
ATGTTTGATCACGTGGTCCTCGTCTTCATCTTCCTCAACTGCGTCACCATCGCCCTGGAGAGGCCTGACA
TTGATCCCGGCAGCACCGAGCGGGTCTTCCTCAGCGTCTCCAATTACATCTTCACGGCCATCTTCGTGGC
GGAGATGATGGTGAAGGTGGTGGCCCTGGGGCTGCTGTCCGGCGAGCACGCCTACCTGCAGAGCAGCTGG
AACCTGCTGGATGGGCTGCTGGTGCTGGTGTCCCTGGTGGACATTGTCGTGGCCATGGCCTCGGCTGGTG
GCGCCAAGATCCTGGGTGTTCTGCGCGTGCTGCGTCTGCTGCGGACCCTGCGGCCTCTGAGGGTCATCAG
CCGGGCCCCGGGCCTCAAGCTGGTGGTGGAGACGCTGATATCATCACTCAGGCCCATTGGGAACATCGTC
CTCATCTGCTGCGCCTTCTTCATCATTTTTGGCATTTTGGGTGTGCAGCTCTTCAAAGGGAAGTTCTACT
ACTGCGAGGGCCCCGACACCAGGAACATCTCCACCAAGGCACAGTGCCGGGCCGCCCACTACCGCTGGGT
GCGACGCAAGTACAACTTCGACAACCTGGGCCAGGCCCTGATGTCGCTGTTCGTGCTGTCATCCAAGGAT
GGATGGGTGAACATCATGTACGACGGGCTGGATGCCGTGGGTGTCGACCAGCAGCCTGTGCAGAACCACA
ACCCCTGGATGCTGCTGTACTTCATCTCCTTCCTGCTCATCGTCAGCTTCTTCGTGCTCAACATGTTCGT
GGGCGTCGTGGTCGAGAACTTCCACAAGTGCCGGCAGCACCAGGAGGCGGAGGAGGCGCGGCGGCGAGAG
GAGAAGCGGCTGCGGCGCCTAGAGAGGAGGCGCAGGAAGGCCCAGCGCCGGCCCTACTATGCCGACTACT
CGCCCACGCGCCGCTCCATTCACTCGCTGTGCACCAGCCACTATCTCGACCTCTTCATCACCTTCATCAT
CTGTGTCAACGTCATCACCATGTCCATGGAGCACTATAACCAACCCAAGTCGCTGGACGAGGCCCTCAAG
TACTGCAACTACGTCTTCACCATCGTGTTTGTCTTCGAGGCTGCACTGAAGCTGGTAGCATTTGGGTTCC
GTCGGTTCTTCAAGGACAGGTGGAACCAGCTGGACCTGGCCATCGTGCTGCTGTCACTCATGGGCATCAC
GCTGGAGGAGATAGAGATGAGCGCCGCGCTGCCCATCAACCCCACCATCATCCGCATCATGCGCGTGCTT
CGCATTGCCCGTGTGCTGAAGCTGCTGAAGATGGCTACGGGCATGCGCGCCCTGCTGGACACTGTGGTGC
AAGCTCTCCCCCAGGTGGGGAACCTGGGCCTTCTTTTCATGCTCCTGTTTTTTATCTATGCTGCGCTGGG
AGTGGAGCTGTTCGGGAGGCTGGAGTGCAGTGAAGACAACCCCTGCGAGGGCCTGAGCAGGCACGCCACC
TTCAGCAACTTCGGCATGGCCTTCCTCACGCTGTTCCGCGTGTCCACGGGGGACAACTGGAACGGGATCA
TGAAGGACACGCTGCGCGAGTGCTCCCGTGAGGACAAGCACTGCCTGAGCTACCTGCCGGCCCTGTCGCC
CGTCTACTTCGTGACCTTCGTGCTGGTGGCCCAGTTCGTGCTGGTGAACGTGGTGGTGGCCGTGCTCATG
AAGCACCTGGAGGAGAGCAACAAGGAGGCACGGGAGGATGCGGAGCTGGACGCCGAGATCGAGCTGGAGA
TGGCGCAGGGCCCCGGGAGTGCACGCCGGGTGGACGCGGACAGGCCTCCCTTGCCCCAGGAGAGTCCGGG
CGCCAGGGACGCCCCAAACCTGGTTGCACGCAAGGTGTCCGTGTCCAGGATGCTCTCGCTGCCCAACGAC
AGCTACATGTTCAGGCCCGTGGTGCCTGCCTCGGCGCCCCACCCCCGCCCGCTGCAGGAGGTGGAGATGG
AGACCTATGGGGCCGGCACCCCCTTGGGCTCCGTTGCCTCTGTGCACTCTCCGCCCGCAGAGTCCTGTGC
CTCCCTCCAGATCCCATTGGCTGTGTCGTCCCCAGCCAGGAGCGGCGAGCCCCTCCACGCCCTGTCCCCT
CGGGGCACAGCCCGCTCCCCCAGTCTCAGCCGGCTGCTCTGCAGACAGGAGGCTGTGCACACCGATTCCT
TGGAAGGGAAGATTGACAGCCCTAGGGACACCCTGGATCCTGCAGAGCCTGGTGAGAAAACCCCGGTGAG
GCCGGTGACCCAGGGGGGCTCCCTGCAGTCCCCACCACGCTCCCCACGGCCCGCCAGCGTCCGCACTCGT
AAGCATACCTTCGGACAGCGCTGCGTCTCCAGCCGGCCGGCGGCCCCAGGCGGAGAGGAGGCCGAGGCCT
CGGACCCAGCCGACGAGGAGGTCAGCCACATCACCAGCTCCGCCTGCCCCTGGCAGCCCACAGCCGAGCC
CCATGGCCCCGAAGCCTCTCCGGTGGCCGGCGGCGAGCGGGACCTGCGCAGGCTCTACAGCGTGGATGCT
CAGGGCTTCCTGGACAAGCCGGGCCGGGCAGACGAGCAGTGGCGGCCCTCGGCGGAGCTGGGCAGCGGGG
AGCCTGGGGAGGCGAAGGCCTGGGGCCCTGAGGCCGAGCCCGCTCTGGGTGCGCGCAGAAAGAAGAAGAT
GAGCCCCCCTGCATCTCGGTGGAACCCCCTGCGGAGGACGAGGGCTCTGCGCGGCCCTCCGCGGCAGAG
GGCGGCAGCACCACACTGAGGCGCAGGACCCCGTCCTGTGAGGCCACGCCTCACAGGGACTCCCTGGAGC
CCACAGAGGGCTCAGGCGCCGGGGGGGACCCTGCAGCCAAGGGGGAGCGCTGGGGCCAGGCCTCCTGCCG
GGCTGAGCACCTGACCGTCCCCAGCTTTGCCTTTGAGCCGCTGGACCTCGGGGTCCCCAGTGGAGACCCT
TTCTTGGACGGTAGCCACAGTGTGACCCCAGAATCCAGAGCTTCCTCTTCAGGGGCCATAGTGCCCCTGG
AACCCCCAGAATCAGAGCCTCCATGCCCGTCGGTGACCCCCAGAGAAGAGGCGGGGGCTGTACCTCAC
AGTCCCCCAGTGTCCTCTGGAGAAACCAGGGTCCCCCTCAGCCACCCCTGCCCCAGGGGGTGGTGCAGAT
GACCCCGTGTAGCTCGGGGCTTGGTGCCGCCCACGGCTTTGGCCCTGGGGTCTGGGGGCCCCGCTGGGGT
GGAGGCCCAGGCAGAACCCTGCATGGACCCTGACTTGGGTCCCGTCGTGAGCAGAAAGGCCCGGGGAGGA
TGACGGCCCAGGCCCTGGTTCTCTGCCCAGCGAAGCAGGAGTAGCTGCCGGGCCCCACGAGCCTCCGTCC
GTTCTGGTTCGGGTTTCTCCGAGTTTTGCTACCAGCCGAGGCTGTGCGGGCAACTGGGTCAGCCTCCCGT
CAGGAGAGAAGCCGCGTCTGTGGGACGAAGACCGGGCACCCGCCAGAGAGGGGAAGGTACCAGGTTGCGT
CCTTTCAGGCCCCGCGTTGTTACAGGACACTCGCTGGGGGCCCTGTGCCCTTGCCGGCGGCAGGTTGCAG
CCACCGCGGCCCAATGTCACCTTCACTCACAGTCTGAGTTCTTGTCCGCCTGTCACGCCCTCACCACCCT
```

```
CCCCTTCCAGCCACCACCCTTTCCGTTCCGCTCGGGCCTTCCCAGAAGCGTCCTGTGACTCTGGGAGAGG
TGACACCTCACTAAGGGGCCGACCCCATGGAGTAACGCGCCCGGCCCCGATGCGAATCAGGCCTCCCCTA
CATCTGGGGGCGTTGGCCGCGAGATTCCCATTGACACCTTTGTTTCGTGTGCTTTTAAATTCAGGTTAAA
TGTTGCAATAATCTGATGCAGAAGACTCAGCTTCTCAAGGGAGAGGGAGGGGGCGGAGCGGAATAAATAG
TAACTTATTTAAGAAATGCAAAAAAAAA
```

>NM_021098.2 Homo sapiens calcium voltage-gated channel subunit alphal H **(CACNA1H)**, transcript variant 1, mRNA

```
CGAGGCCGCCGCCGTCGCCTCCGCCGGGCGAGCCGGAGCCGGAGTCGAGCCGCGGCCGGGAGCCGGGCGG
GCTGGGGACGCGGGCCGGGGGCGGAGGCGCTGGGGGCCGGGGCCGGGGCCGGGGGCGGAGGCGCTGGGGG
CCGGGGCCGGGGCCGGGCGCCGAGCGGGGTCCGCGGTGACCGCGCCGCCCGGGCGATGCCCGCGGGGACG
CCGCCGGCCAGCAGAGCGAGGTGCTGCCGGCCGCCACCATGACCGAGGGCGCACGGGCCGCCGACGAGGT
CCGGGTGCCCCTGGGCGCGCCGCCCCCTGGCCCTGCGGCGTTGGTGGGGGCGTCCCCGGAGAGCCCCGGG
GCGCCGGGACGCGAGGCGGAGCGGGGGTCCGAGCTCGGCGTGTCACCCTCCGAGAGCCCGGCGGCCGAGC
GCGGCGCGGAGCTGGGTGCCGACGAGGAGCAGCGCGTCCCGTACCCGGCCTTGGCGGCCACGGTCTTCTT
CTGCCTCGGTCAGACCACGCGGCCGCGCAGCTGGTGCCTCCGGCTGGTCTGCAACCCATGGTTCGAGCAC
GTGAGCATGCTGGTAATCATGCTCAACTGCGTGACCCTGGGCATGTTCCGGCCCTGTGAGGACGTTGAGT
GCGGCTCCGAGCGCTGCAACATCCTGGAGGCCTTTGACGCCTTCATTTTCGCCTTTTTTGCGGTGGAGAT
GGTCATCAAGATGGTGGCCTTGGGGCTGTTCGGGCAGAAGTGTTACCTGGGTGACACGTGGAACAGGCTG
GATTTCTTCATCGTCGTGGCGGGCATGATGGAGTACTCGTTGGACGGACACAACGTGAGCCTCTCGGCTA
TCAGGACCGTGCGGGTGCTGCGGCCCCTCCGCGCCATCAACCGCGTGCCTAGCATGCGGATCCTGGTCAC
TCTGCTGCTGGATACGCTGCCCATGCTCGGGAACGTCCTTCTGCTGTGCTTCTTCGTCTTCTTCATTTTC
GGCATCGTTGGCGTCCAGCTCTGGGCTGGCCTCCTGCGGAACCGCTGCTTCCTGGACAGTGCCTTTGTCA
GGAACAACAACCTGACCTTCCTGCGGCCGTACTACCAGACGGAGGAGGGCGAGGAGAACCCGTTCATCTG
CTCCTCACGCCGAGACAACGGCATGCAGAAGTGCTCGCACATCCCCGGCCGCCGCGAGCTGCGCATGCCC
TGCACCCTGGGCTGGGAGGCCTACACGCAGCCGCAGGCCGAGGGGGTGGGCGCTGCACGCAACGCCTGCA
TCAACTGGAACCAGTACTACAACGTGTGCCGCTCGGGTGACTCCAACCCCCACAACGGTGCCATCAACTT
CGACAACATCGGCTACGCCTGGATTGCCATCTTCCAGGTGATCACGCTGGAAGGCTGGGTGGACATCATG
TACTACGTCATGGACGCCCACTCATTCTACAACTTCATCTATTTCATCCTGCTCATCATCGTGGGCTCCT
TCTTCATGATCAACCTGTGCCTGGTGGTGATTGCCACGCAGTTCTCGGAGACGAAGCAGCGGGAGAGTCA
GCTGATGCGGGAGCAGCGGGCACGCCACCTGTCCAACGACAGCACGCTGGCCAGCTTCTCCGAGCCTGGC
AGCTGCTACGAAGAGCTGCTGAAGTACGTGGGCCACATATTCCGCAAGGTCAAGCGGCGCAGCTTGCGCC
TCTACGCCCGCTGGCAGAGCCGCTGGCGCAAGAAGGTGGACCCCAGTGCTGTGCAAGGCCAGGGTCCCGG
GCACCGCCAGCGCCGGGCAGGCAGGCACACAGCCTCGGTGCACCACCTGGTCTACCACCACCATCACCAC
CACCACCACCACTACCATTTCAGCCATGGCAGCCCCGCAGGCCCGGCCCCGAGCCAGGCGCCTGCGACA
CCAGGCTGGTCCGAGCTGGCGCGCCCCCCTCGCCACCTTCCCCAGGCCGCGGACCCCCCGACGCAGAGTC
TGTGCACAGCATCTACCATGCCGACTGCCACATAGAGGGGCCGCAGGAGAGGGCCCGGGTGGCACATGCC
GCAGCCACTGCCGCTGCCAGCCTCAGACTGGCCACAGGGCTGGGCACCATGAACTACCCCACGATCCTGC
CCTCAGGGGTGGGCAGCGGCAAAGGCAGCACCAGCCCCGGACCCAAGGGGAAGTGGGCCGGTGGACCGCC
AGGCACCGGGGGGGCACGGCCCGTTGAGCTTGAACAGCCCTGATCCCTACGAGAAGATCCCGCATGTGGTC
GGGGAGCATGGACTGGGCCAGGCCCCTGGCCATCTGTCGGGCCTCAGTGTGCCCTGCCCCCTGCCCAGCC
CCCCAGCGGGCACACTGACCTGTGAGCTGAAGAGCTGCCCGTACTGCACCCGTGCCCTGGAGGACCCGGA
GGGTGAGCTCAGCGGCTCGGAAAGTGGAGACTCAGATGGCCGTGGCGTCTATGAATTCACGCAGGACGTC
CGGCACGGTGACCGCTGGGACCCCACGCGACCACCCCGTGCGACGGACACACCAGGCCCAGGCCCAGGCA
GCCCCCAGCGGCGGGCACAGCAGAGGGCAGCCCCGGGCGAGCCAGGCTGGATGGGCCGCCTCTGGGTTAC
CTTCAGCGGCAAGCTGCGCCGCATCGTGGACAGCAAGTACTTCAGCCGTGGCATCATGATGGCCATCCTT
GTCAACACGCTGAGCATGGGCGTGGAGTACCATGAGCAGCCCGAGGAGCTGACTAATGCTCTGGAGATCA
GCAACATCGTGTTCACCAGCATGTTTGCCCTGGAGATGCTGCTGAAGCTGCTGGCCTGCGGCCCTCTGGG
CTACATCCGGAACCCGTACAACATCTTCGACGGCATCATCGTGGTCATCAGCGTCTGGGAGATCGTGGGG
CAGGCGGACGGTGGCTTGTCTGTGCTGCGCACCTTCCGGCTGCTGCGTGTGCTGAAGCTGGTGCGCTTTC
TGCCAGCCCTGCGGCGCCAGCTCGTGGTGCTGGTGAAGACCATGGACAACGTGGCTACCTTCTGCACGCT
GCTCATGCTCTTCATTTTCATCTTCAGCATCCTGGGCATGCACCTTTTCGGCTGCAAGTTCAGCCTGAAG
ACAGACACCGGAGACACCGTGCCTGACAGGAAGAACTTCGACTCCCTGCTGTGGGCCATCGTCACCGTGT
TCCAGATCCTGACCCAGGAGGACTGGAACGTGGTCCTGTACAACGGCATGGCCTCCACCTCCTCCTGGGC
CGCCCTCTACTTCGTGGCCCTCATGACCTTCGGCAACTATGTGCTCTTCAACCTGCTGGTGGCCATCCTC
GTGGAGGGCTTCCAGGCGGAGGGCGATGCCAACAGATCCGACACGGACGAGGACAAGACGTCGGTCCACT
TCGAGGAGGACTTCCACAAGCTCAGAGAACTCCAGACCACAGAGCTGAAGATGTGTTCCCTGGCCGTGAC
CCCCAACGGGCACCTGGAGGGACGAGGCAGCCTGTCCCCTCCCCTCATCATGTGCACAGCTGCCACGCCC
ATGCCTACCCCCAAGAGCTCACCATTCCTGGATGCAGCCCCCAGCCTCCCAGACTCTCGGCGTGGCAGCA
GCAGCTCCGGGGACCCGCCACTGGAGACCAGAAGCCTCCGGCCAGCCTCCGAAGTTCTCCCTGTGCCCC
```

CTGGGGCCCCAGTGGCGCCTGGAGCAGCCGGCGCTCCAGCTGGAGCAGCCTGGGCCGTGCCCCCAGCCTC
AAGCGCCGCGGCCAGTGTGGGGAACGTGAGTCCCTGCTGTCTGGCGAGGGCAAGGGCAGCACCGACGACG
AAGCTGAGGACGGCAGGGCCGCGCCCGGGCCCCGTGCCACCCCACTGCGGCGGGCCGAGTCCCTGGACCC
ACGGCCCCTGCGGCCGGCCGCCCTCCCGCCTACCAAGTGCCGCGATCGCGACGGGCAGGTGGTGGCCCTG
CCCAGCGACTTCTTCCTGCGCATCGACAGCCACCGTGAGGATGCAGCCGAGCTTGACGACGACTCGGAGG
ACAGCTGCTGCCTCCGCCTGCATAAAGTGCTGGAGCCCTACAAGCCCCAGTGGTGCCGGAGCCGCGAGGC
CTGGGCCCTCTACCTCTTCTCCCCACAGAACCGGTTCCGCGTCTCCTGCCAGAAGGTCATCACACACAAG
ATGTTTGATCACGTGGTCCTCGTCTTCATCTTCCTCAACTGCGTCACCATCGCCCTGGAGAGGCCTGACA
TTGATCCCGGCAGCACCGAGCGGGTCTTCCTCAGCGTCTCCAATTACATCTTCACGGCCATCTTCGTGGC
GGAGATGATGGTGAAGGTGGTGGCCCTGGGGCTGCTGTCCGGCGAGCACGCCTACCTGCAGAGCAGCTGG
AACCTGCTGGATGGGCTGCTGGTGCTGGTGTCCCTGGTGGACATTGTCGTGGCCATGGCCTCGGCTGGTG
GCGCCAAGATCCTGGGTGTTCTGCGCGTGCTGCGTCTGCTGCGGACCCTGCGGCCTCTGAGGGTCATCAG
CCGGGCCCCGGGCCTCAAGCTGGTGGTGGAGACGCTGATATCATCACTCAGGCCCATTGGGAACATCGTC
CTCATCTGCTGCGCCTTCTTCATCATTTTTGGCATTTTGGGTGTGCAGCTCTTCAAAGGGAAGTTCTACT
ACTGCGAGGGCCCCGACACCAGGAACATCTCCACCAAGGCACAGTGCCGGGCCGCCCACTACCGCTGGGT
GCGACGCAAGTACAACTTCGACAACCTGGGCCAGGCCCTGATGTCGCTGTTCGTGCTGTCATCCAAGGAT
GGATGGGTGAACATCATGTACGACGGGCTGGATGCCGTGGGTGTCGACCAGCAGCCTGTGCAGAACCACA
ACCCCTGGATGCTGCTGTACTTCATCTCCTTCCTGCTCATCGTCAGCTTCTTCGTGCTCAACATGTTCGT
GGGCGTCGTGGTCGAGAACTTCCACAAGTGCCGGCAGCACCAGGAGGCGGAGGAGGCGCGGCGGCGAGAG
GAGAAGCGGCTGCGGCGCCTAGAGAGGAGGCGCAGGAGCACTTTCCCCAGCCCAGAGGCCCAGCGCCGGC
CCTACTATGCCGACTACTCGCCCACGCGCCGCTCCATTCACTCGCTGTGCACCAGCCACTATCTCGACCT
CTTCATCACCTTCATCATCTGTGTCAACGTCATCACCATGTCCATGGAGCACTATAACCAACCCAAGTCG
CTGGACGAGGCCCTCAAGTACTGCAACTACGTCTTCACCATCGTGTTTGTCTTCGAGGCTGCACTGAAGC
TGGTAGCATTTGGGTTCCGTCGGTTCTTCAAGGACAGGTGGAACCAGCTGGACCTGGCCATCGTGCTGCT
GTCACTCATGGGCATCACGCTGGAGGAGATAGAGATGAGCGCCGCGCTGCCCATCAACCCCACCATCATC
CGCATCATGCGCGTGCTTCGCATTGCCCGTGTGCTGAAGCTGCTGAAGATGGCTACGGGCATGCGCGCCC
TGCTGGACACTGTGGTGCAAGCTCTCCCCCAGGTGGGGAACCTGGGCCTTCTTTTCATGCTCCTGTTTTT
TATCTATGCTGCGCTGGGAGTGGAGCTGTTCGGGAGGCTGGAGTGCAGTGAAGACAACCCCTGCGAGGGC
CTGAGCAGGCACGCCACCTTCAGCAACTTCGGCATGGCCTTCCTCACGCTGTTCCGCGTGTCCACGGGGG
ACAACTGGAACGGGATCATGAAGGACACGCTGCGCGAGTGCTCCCGTGAGGACAAGCACTGCCTGAGCTA
CCTGCCGGCCCTGTCGCCCGTCTACTTCGTGACCTTCGTGCTGGTGGCCCAGTTCGTGCTGGTGAACGTG
GTGGTGGCCGTGCTCATGAAGCACCTGGAGGAGAGCAACAAGGAGGCACGGGAGGATGCGGAGCTGGACG
CCGAGATCGAGCTGGAGATGGCGCAGGGCCCCGGGAGTGCACGCCGGGTGGACGCGGACAGGCCTCCCTT
GCCCCAGGAGAGTCCGGGCGCCAGGGACGCCCCAAACCTGGTTGCACGCAAGGTGTCCGTGTCCAGGATG
CTCTCGCTGCCCAACGACAGCTACATGTTCAGGCCCGTGGTGCCTGCCTCGGCGCCCCACCCCCGCCCGC
TGCAGGAGGTGGAGATGGAGACCTATGGGGCCGGCACCCCCTTGGGCTCCGTTGCCTCTGTGCACTCTCC
GCCCGCAGAGTCCTGTGCCTCCCTCCAGATCCCATTGGCTGTGTCGTCCCCAGCCAGGAGCGGCGAGCCC
CTCCACGCCCTGTCCCCTCGGGGCACAGCCCGCTCCCCCAGTCTCAGCCGGCTGCTCTGCAGACAGGAGG
CTGTGCACACCGATTCCTTGGAAGGGAAGATTGACAGCCCTAGGGACACCCTGGATCCTGCAGAGCCTGG
TGAGAAAACCCCGGTGAGGCCGGTGACCCAGGGGGGCTCCCTGCAGTCCCCACCACGCTCCCCACGGCCC
GCCAGCGTCCGCACTCGTAAGCATACCTTCGGACAGCGCTGCGTCTCCAGCCGGCCGGCGGCCCCAGGCG
GAGAGGAGGCCGAGGCCTCGGACCCAGCCGACGAGGAGGTCAGCCACATCACCAGCTCCGCCTGCCCCTG
GCAGCCCACAGCCGAGCCCCATGGCCCCGAAGCCTCTCCGGTGGCCGGCGGCGAGCGGGACCTGCGCAGG
CTCTACAGCGTGGATGCTCAGGGCTTCCTGGACAAGCCGGGCCGGGCAGACGAGCAGTGGCGGCCCTCGG
CGGAGCTGGGCAGCGGGGAGCCTGGGGAGGCGAAGGCCTGGGGCCCTGAGGCCGAGCCCGCTCTGGGTGC
GCGCAGAAAGAAGAAGATGAGCCCCCCTGCATCTCGGTGGAACCCCCTGCGGAGGACGAGGGCTCTGCG
CGGCCCTCCGCGGCAGAGGGCGGCAGCACCACACTGAGGCGCAGGACCCCGTCCTGTGAGGCCACGCCTC
ACAGGGACTCCCTGGAGCCCACAGAGGGCTCAGGCGCCGGGGGGGACCCTGCAGCCAAGGGGGAGCGCTG
GGGCCAGGCCTCCTGCCGGGCTGAGCACCTGACCGTCCCCAGCTTTGCCTTTGAGCCGCTGGACCTCGGG
GTCCCCAGTGGAGACCCTTTCTTGGACGGTAGCCACAGTGTGACCCCAGAATCCAGAGCTTCCTCTTCAG
GGGCCATAGTGCCCCTGGAACCCCCAGAATCAGAGCCTCCATGCCCGTCGGTGACCCCCAGAGAAGAG
GCGGGGGCTGTACCTCACAGTCCCCCAGTGTCCTCTGGAGAAACCAGGGTCCCCCTCAGCCACCCCTGCC
CCAGGGGGTGGTGCAGATGACCCCGTGTAGCTCGGGGCTTGGTGCCGCCCACGGCTTTGGCCCTGGGGTC
TGGGGGCCCCGCTGGGGTGGAGGCCCAGGCAGAACCCTGCATGGACCCTGACTTGGGTCCCGTCGTGAGC
AGAAAGGCCCGGGGAGGATGACGGCCCAGGCCCTGGTTCTCTGCCCAGCGAAGCAGGAGTAGCTGCCGGG
CCCCACGAGCCTCCGTCCGTTCTGGTTCGGGTTTCTCCGAGTTTTGCTACCAGCCGAGGCTGTGCGGGCA
ACTGGGTCAGCCTCCCGTCAGGAGAGAAGCCGCGTCTGTGGGACGAAGACCGGGCACCGCCAGAGAGGG
GAAGGTACCAGGTTGCGTCCTTTCAGGCCCCGCGTTGTTACAGGACACTCGCTGGGGGCCCTGTGCCCTT
GCCGGCGGCAGGTTGCAGCCACCGCGGCCCAATGTCACCTTCACTCACAGTCTGAGTTCTTGTCCGCCTG
TCACGCCCTCACCACCCTCCCCTTCCAGCCACCACCCTTTCCGTTCGCTCGGGCCTTCCCAGAAGCGTC

CTGTGACTCTGGGAGAGGTGACACCTCACTAAGGGGCCGACCCCATGGAGTAACGCGCCCGGCCCCGATG
CGAATCAGGCCTCCCCTACATCTGGGGGCGTTGGCCGCGAGATTCCCATTGACACCTTTGTTTCGTGTGC
TTTTAAATTCAGGTTAAATGTTGCAATAATCTGATGCAGAAGACTCAGCTTCTCAAGGGAGAGGGAGGGG
GCGGAGCGGAATAAATAGTAACTTATTTAAGAAATGCAAAAAAAAA

>NM_032251.5 Homo sapiens coiled-coil domain containing 88B (**CCDC88B**), mRNA

CTCAGGGCAGGTGCAGCTGCCACAGTGAGACGGGCACCCCGACCCGGGCATGGAGGGGGGCAAGGGGCCC
AGGCTCAGAGACTTCCTGAGTGGGAGTCTGGCTACCTGGGCGCTGGGACTGGCCGGGCTGGTCGGGGAGG
CGGAGGACTCGGAGGGGGAAGAAGAGGAAGAGGAGGAAGAGCCGCCCCTTTGGTTGGAGAAGAGATTCCT
GCGCCTCAGCGATGGGGCCCTGCTCCTCCGGGTGCTGGGCATCATTGCCCCCAGCTCCCGAGGGGGACCT
CGGATGCTCAGAGGCCTTGACGGACCTGCTGCCTGGCGAGTGTGGAACCTGAACCACCTGTGGGGCCGAC
TGAGGGACTTCTACCAGGAGGAGCTGCAGCTGCTGATCCTGTCGCCACCCCCAGACCTCCAGACATTGGG
ATTTGACCCTCTCTCAGAAGAAGCGGTGGAGCAGCTGGAAGGCGTTCTTCGGCTACTGTTGGGAGCGTCA
GTACAGTGTGAGCACCGGGAACTCTTCATCCGCCACATCCAGGGCCTCAGTCTCGAGGTCCAGAGCGAGC
TGGCCGCTGCCATCCAGGAGGTGACCCAGCCGGGGGCCGGCGTGGTGCTGGCACTGTCTGGGCCAGATCC
TGGGGAGCTGGCACCTGCCGAGCTGGAGATGCTGTCCCGGAGCCTGATGGGGACACTGTCGAAGCTGGCA
CGGGAGCGTGACCTGGGGGCCCAGCGGCTGGCTGAACTGCTGCTGGAGCGAGAACCCCTCTGCTTGAGGC
CTGAGGCTCCCTCTAGGGCTCCCGCCGAGGGCCCCTCGCACCATCTGGCCCTGCAGCTGGCCAACGCCAA
GGCTCAGCTGCGGCGTCTGCGGCAGGAGCTGGAGGAGAAGGCCGAGCTGCTGCTAGACTCCCAGGCCGAG
GTGCAGGGTTTGGAGGCCGAAATAAGAAGGCTCCGCCAGGAGGCCCAGGCGCTGTCGGGACAGGCCAAGC
GGGCCGAGCTGTACCGCGAGGAGGCAGAGGCGCTGCGGGAGCGGGCCGGCCGCCTGCCCCGCCTGCAGGA
GGAGCTGAGGCGCTGCCGCGAGCGGCTGCAGGCGGCTGAGGCCTACAAGAGTCAGCTGGAGGAGGAGCGG
GTGCTCTCGGGGGTGCTGGAGGCGTCCAAGGCGCTGCTGGAAGAGCAGCTGGAGGCTGCCCGAGAGCGCT
GCGCCCGGCTGCACGAGACCCAGCGCGAGAACCTGCTGCTGCGAACCCGGCTGGGCGAGGCCCATGCGGA
GCTGGACTCTCTGCGGCATCAGGTGGACCAGCTGGCTGAGGAGAATGTGGAGCTGGAGCTGGAGCTTCAG
CGGAGCTTGGAGCCACCTCCAGGATCCCCTGGGGAGGCACCCCTAGCAGGAGCGGCCCCCTCGCTGCAAG
ATGAGGTGAGGGAGGCAGAGGCTGGGCGGCTTCGGACCCTTGAGAGGGAGAACCGGGAGCTTCGGGGGCT
GCTTCAGGTGCTTCAGGGGCAGCCAGGGGGCCAGCACCCCCTGCTGGAGGCACCGAGAGAGGACCCTGTT
CTTCCAGTGCTGGAGGAGGCTCCCCAGACTCCTGTGGCCTTCGACCACAGCCCTCAGGGCTTGGTTCAGA
AGGCAAGGGATGGAGGCCCCCAGGCCTTGGACTTGGCTCCCCGGCATTAGACTCAGTGCTCGAGGCATC
AGCTGAGTGTCCCCAGGCACCTGATTCAGACCCACAGGAGGCAGAGAGTCCCCTTCAGGCAGCTGCCATG
GACCCCCAGGCCTCAGACTGGTCCCCGCAAGAGTCAGGCTCTCCTGTGGAGACACAGGAGTCCCCGGAGA
AGGCTGGCCGTAGATCCTCTCTCCAGAGCCCTGCCTCTGTGGCCCCACCTCAGGGTCCAGGGACCAAAAT
TCAGGCCCCGCAGTTGCTGGGAGGAGAGACAGAGGGAAGAGAGGCTCCCCAAGGCGAGTTGGTGCCTGAG
GCCTGGGGGTTGAGACAGGAGGGCCCTGAGCACAAGCCAGGGCCTTCGGAGCCCAGCTCTGTGCAGCTGG
AGGAGCAGGAGGGCCCAAACCAGGGCCTGGACCTGGCCACGGGACAAGCAGAGGCCAGAGAGCATGACCA
GAGGCTGGAAGGGACGGTCAGGGACCCAGCCTGGCAAAAACCACAGCAGAAGTCAGAAGGGGCTCTTGAG
GTCCAGGTCTGGGAAGGCCCAATCCCAGGGGAGAGCCTGGCCAGTGGTGTCGCAGAGCAGGAGGCCCTCA
GGGAGGAGGTGGCACAGTTGAGGAGAAAGGCTGAGGCCCTTGGAGATGAGCTGGAAGCCCAGGCCCGCAA
GCTGGAGGCCCAAAACACGGAGGCTGCCCGCCTCTCCAAGGAGCTGGCCCAAGCGCGAAGGGCAGAGGCC
GAGGCCCACCGGGAGGCAGAGGCCCAGGCCTGGGAGCAAGCCCGGCTGCGGGAGGCAGTGGAGGCTGCTG
GCCAGGAGCTGGAGTCTGCGTCCCAGGAACGGGAGGCGCTGGTGGAGGCGCTGGCAGCAGCGGGCCGGGA
GCGGAGGCAGTGGGAGCGTGAGGGGTCCAGGCTGCGGGCCCAGTCGGAGGCCGCCGAGGAACGGATGCAG
GTGCTGGAGAGCGAGGGCCGCCAGCACTTGGAGGAGGCTGAGAGGGAGCGCCGGGAGAAGGAGGCCCTCC
AGGCGGAGCTGGAGAAAGCTGTGGTGCGGGGCAAGGAGTTGGGGGACCGGCTGGAGCATTTGCAGCGTGA
GCTGGAGCAGGCGGCTCTCGAGCGCCAGGAATTTCTGCGAGAAAAGGAAAGCCAGCACCAGAGGTACCAG
GGCTTGGAGCAGCGGCTGGAAGCTGAGCTGCAGGCGGCGGCGACCAGCAAGGAGGAGGCGCTGATGGAGC
TCAAGACCAGGGCCCTGCAGCTGGAAGAGGAGCTGTTCCAGCTGCGCCAGGGCCCCGCGGGGCTGGGGCC
CAAAAAGCGTGCGGAGCCTCAGCTGGTGGAGACCCAGAATGTGCGGCTTATTGAGGTGGAGCGCAGTAAT
GCGATGCTGGTGGCAGAGAAGGCAGCTTTGCAGGGGCAGCTGCAGCACCTGGAGGGGCAGCTGGGGAGCC
TGCAGGGCCGTGCCCAGGAGCTGCTGCTGCAGAGCCAGCGGGCGCAGGAGCACAGCAGCCGCCTGCAGGC
CGAGAAGTCTGTGCTGGAGATTCAGGGCCAGGAGCTGCACCGGAAGCTGGAGGTGCTGGAGGAGGAGGTG
CGGGCGGCACGGCAGTCCCAGGAGGAGACCCGCGGGCAGCAGCAGGCCCTGCTTCGGGACCACAAGGCCC
TGGCACAGCTGCAGCGGCGGCAGGAGGCCGAGCTAGGGGACTGCTGGTGCGGCACCGAGACCTCAAGGC
CAACATGCGGGCACTGGAGCTGGCCCACCGGGAGCTGCAGGGCCGGCACGAGCAGCTGCAGGCCCAGCGG
GCCAGCGTGGAGGCACAGGAGGTGGCCCTGCTGGCAGAGCGTGAACGCCTGATGCAAGATGGGCATCGGC
AGCGGGGCCTGGAGGAGGAGCTGCGGAGGCTTCAGAGCGAGCACGACAGGGCTCAGATGCTGCTGGCAGA
GTTGTCTCGGGAGCGGGGTGAGCTGCAGGGTGAACGCGGGGAGCTACGGGGCCGGCTGGCGCGGCTGGAG
CTGGAGCGGGCACAGCTGGAGATGCAGAGCCAGCAGCTGCGCGAGTCCAACCAGCAGCTGGACCTGAGCG

```
CCTGCCGGCTGACCACGCAGTGTGAGCTATTGACACAGCTGCGAAGTGCCCAGGAAGAGGAGAACCGGCA
GCTGCTGGCTGAAGTTCAGGCCCTGAGCCGGGAGAACAGGGAGCTCCTGGAGCGCAGCCTGGAGAGTCGG
GACCACCTGCACCGCGAACAGCGGGAGTACCTGGACCAGCTTAATGCCCTGCGCCGCGAGAAGCAGAAGC
TCGTGGAGAAGATCATGGACCAATACCGCGTGCTGGAGCCTGTGCCCCTGCCCCGGACCAAGAAGGGCAG
CTGGCTGGCAGACAAGGTGAAGAGGCTGATGCGGCCCCGGCGGGAGGGGGGCCCCCCTGGGGGGCTGCGC
CTGGGGGCCGATGGGGCTGGCAGCACCGAGAGCCTGGGGGGCCCCCCGGAGACGGAGCTTCCTGAGGGCA
GGGAGGCAGATGGGACAGGGTCCCCTTCCCCGGCACCCATGCGCCGGGCCCAGAGCTCCCTCTGCCTGCG
GGATGAGACCTTGGCAGGCGGGCAGCGGCGGAAACTCAGCTCAAGGTTCCCGGTGGGGCGAAGCTCTGAG
TCATTCAGCCCTGGGGACACCCCTAGGCAACGATTCCGACAGCGCCATCCAGGCCCCCTGGGGGCGCCCG
TCTCCACAGCAAAGGACCTGGTGTGGGATGGGAGAACTCCGCTGAGACCCTGCAGGAACACGAAACAGA
TGCCAACCGAGAGGGCCCTGAGGTACAGGAACCGGAGAAACGTCCCCTCACCCCATCCCTCAGCCAGTGA
CACCGTGGGAACAGCAGGCTTGGGAGTGCAGCCTTCTCGGCACTGGAGTGTCAGCGGAGGCCCCAGGCAG
CCCAAGAGCTCAGGGAGCCAGGGACCCCAAGGGGAGTCCTTGGACAAGGAGGCCTGGGCCCTGAGATCCT
CCACGGTCAGCGCCGGGGCCCGGAGATGGAGCTGGGACGAGTGTGTGGACAGGGGGGATGGCTGGCCCCC
ACGAGCAGCTCCAGGCTGGAGTTCTGGTTCTTCCAGGTGGCTCCCGCTGAGGCAGCGGTCTCTGGGGGAT
CCCCCAGCTGAAGGAGGCTGGCAGGAGTTGGCAAGAGAACCCCCTGCCCTGTCCAGGTGGGAAGCTGAGT
CCCAGTGCTGGGGGACTGTGGCCTGGGCTGATCTTGAGCCTTAACTGGACATGAGGGGCATGAGAATAAA
GCTGAACTGCAGCCTCCTGAAAAAAAAAAAAA
```

>NM_020877.3 Homo sapiens dynein axonemal heavy chain 2 **(DNAH2)**, transcript variant 1, mRNA

```
CTTCTACATCGCGAATATCCCTCGCCGGCCTGGCGCCCGGCGCCATCTGCTGGACCCGTTCCGGCTGGGG
CGCACAGACCTGGGCGCGGGGTCGGGCGATTGGGTTCTCCCTGCCTTTTCAATGTCCTGCGGGGCGGTGG
CTCACTGTCTCTCGCCCCAGCCCTTCCAGCTGGGTCCTGACACCAGGCCCCCCGCACCCTTCGGCTCGCC
AGATGTGGCTACTTTCCTTTAGTCCCGGAGCCTTCAAGCCCTGGGTCCCTGAGGGGTAGACCTGGGCGGG
GTGGGGCCGCGAAGCAGGGATCCAGGGCGCCTGCGCGGAGGAGGCGGGCTCCGAGGGGCCGCACACCGGC
CACTCGCCCCTCCCTGCCCGGGGTTGCCATGGGACGCGTGCAGACGCCGGCCCGAGAGGGCTGCGAGGG
GCAACTTCTTAGAGTGGCCCATCGGTCGGTCTAGGGAGGGGAGGGTCAGCGTGGCAAGGTGTGTGTCGGG
GGCAGAGGGAAGGAGGGAAGGAGTGCTGGGGAGAAGGGGCAGTAGTGTCGGGGTAGGAAGGGACAGTTGT
TGGGGAGAAGAGGCAGTTGTGGAGGGAGAGGGAGCAAGGAATATTTGTGTGGGGGAGGGGGAGGAGAAGG
GGCAGTTGTGACAGCTGGGGGGGAAGAGCCATTCTGAGGGGAAATTTGCCTGCTGGTAACCAGTTTAAGG
ATACCAGCTGCTGGTATAAATACTGCTGGATAAATACTGCTGGATTATACTGCAGGATAAATACTGCTG
GCCCCTGCGGTATTTCCTAGTACTAGAGTGAGCCCCGACTTAGCAGAGCAGTTCCTCCTGGGGCCTGCGG
TGTGGGATCGCGTGGTGAACCCCACGGTGCATGCGCCTCAGGCTCTAGTTTGAGGCAGGAAAGCGCAGCT
TGATGCTTCTCTGGAGACTGATGGAGGAAGCCTCCTTGCTGTAGTTGGTTTTATTGATTTGCTGGCCTAA
CAGAACGTTTTTCCTTGGAGCAAAGTACAAATCCTTCAAGTTTGAAATTCATAACCTGAGATCAATGCCT
GTGGCAGCCTGTGGGGATGAGGAAGGAGAGCCACAGGTGCCGTTAGGCTGCAGCCTAATGAAAAGAGAGT
GCCCAGCGCCTCAGACTTTGCGCCTGGGATTCTGAGCACCTGTCCGAGATCCCCGCTTCCTGCCATCCTA
CCTTTCTGAGAGAGGCACCACTGTGACCTTCCTCGTGCCCCAATTGCTTTTTCCTCATGACACAATTTGA
AATTTATGATTGGATGACTTTTGTCCTTCTTTCTTCCAATCTGTTCTCAGGGCATTTTGAGTCAAATAAA
TGATCCTGACTGATCTTAACCATTAGCACAGAGTTCCTCAGCCAACTCTGCTAAGAGACCTCAGTACACA
CAAAACAGTGTTCCTGCCCCTCAGGACTTCAAAGCGATAGACCCAGGTTTTGCCTGCACGATGTCCAGCA
AAGCTGAGAAGAAGCAGCGATTGAGTGGCCGAGGAAGCTCCCAGGCAAGCTGGTCAGGGCGGGCCACTCG
GGCTGCTGTGGCCACACAGGAGCAGGGGAATGCCCCGGCTGTCAGTGAGCCAGAGCTGCAGGCTGAGCTC
CCCAAGGAGGAGCCTGAGCCACGGTTGGAGGGACCTCAAGCACAGAGTGAAGAATCAGTGGAGCCCGAGG
CAGATGTGAAGCCCCTCTTCCTTTCCCGAGCTGCGCTGACAGGACTGGCGGATGCAGTGTGGACACAGGA
GCATGATGCCATTCTGGAACACTTTGCCCAGGACCCTACAGAATCCATCCTCACCATCTTCATTGACCCT
TGTTTTGGGCTGAAGCTAGAGCTGGGCATGCCTGTACAGACCCAGAACCAGCTTGTCTACTTCATTCGCC
AAGCACCAGTTCCCATCACCTGGGAGAACTTCGAGGCAACTGTGCAGTTTGGGACGGTGCGGGGCCCCTA
TATCCCGGCCCTGCTTCGGCTGCTCGGTGGAGTCTTTGCCCCTCAGATCTTTGCAAACACAGGCTGGCCT
GAGAGCATTAGAAATCATTTTGCTTCTCATCTGCACAAGTTCTTGGCCTGCCTGACAGACACTCGGTACA
AACTGGAGGGGCACACGGTCCTCTACATCCCTGCAGAGGCCATGAACATGAAGCCTGAGATGGTGATAAA
GGACAAAGAGCTGGTGCAACGGCTAGAGACCTCCATGATCCACTGGACCCGGCAGATAAAGGAGATGCTC
AGTGCCCAGGAGACTGTGGAGACAGGAGAAAATTTAGGTCCTCTGGAGGAGATTGAGTTCTGGCGCAACC
GATGCATGGACCTGTCTGGCATCAGTAAGCAGCTGGTGAAGAAGGGAGTGAAGCACGTTGAATCCATCCT
GCACCTTGCCAAGTCGTCCTACTTGGCGCCCTTTATGAAACTGGCACAGCAGATCCAGGATGGCTCTCGT
CAAGCACAGTCAAACCTGACCTTTTTGTCAATCCTGAAGGAACCTTACCAGGAGTTGGCTTTCATGAAGC
CCAAGGACATCTCTAGCAAGCTCCCTAAGCTGATCAGTCTCATCCGCATCATCTGGGTCAACTCTCCCCA
CTACAACACTCGGGAGAGACTGACCTCGCTCTTCCGAAAGGTATGTGACTGTCAGTATCACTTCGCCCGC
TGGGAAGATGGCAAGCAGGGTCCCCTTCCTTGCTTCTTTGGTGCCCAGGGGCCACAGATAACACGGAACT
```

```
TGCTGGAGATTGAGGACATCTTTTCATAAAAATCTGCACACGCTGCGAGCCGTTCGCGGGGGTATCCTGGA
TGTCAAGAACACCTGTTGGCATGAAGACTACAATAAGTTCCGTGCCGGAATCAAGGACCTGGAGGTGATG
ACCCAGAACCTGATCACCTCAGCCTTCGAGTTGGTGCGGGACGTGCCGCACGGCGTGCTTCTGCTGGACA
CCTTCCACAGGCTTGCCTCCCGCGAGGCTATCAAGCGGACTTATGACAAGAAGGCGGTGGATCTCTACAT
GCTGTTCAATAGCGAGCTGGCCCTGGTGAACCGTGAACGGAACAAGAAATGGCCAGACCTGGAGCCCTAC
GTGGCCCAGTATTCCGGAAAGGCGCGCTGGGTGCACATCCTCCGGCGTCGCATCGACAGAGTCATGACCT
GCCTTGCTGGTGCTCATTTCCTGCCCCGTATTGGGACTGGAAAGGAGAGTGTGCACACCTATCAGCAGAT
GGTCCAGGCCATTGATGAGCTGGTTCGAAAAACCTTCCAAGAGTGGACATCAAGTCTGGACAAGGATTGC
ATTCGGCGGTTGGATACCCCATTGCTGCGAATCAGCCAGGAGAAGGCGGGCATGCTGGATGTCAACTTTG
ACAAGTCCCTTCTGATTCTCTTTGCGGAAATTGACTACTGGGAGCGGCTGCTGTTTGAGACGCCCCATTA
CGTGGTGAACGTAGCTGAGCGAGCCGAGGACCTGCGCATTCTGCGTGAAAATCTGCTACTCGTTGCTAGA
GACTACAATAGGATTATTGCCATGCTGTCCCCAGATGAGCAGGCCCTATTCAAAGAGCGTATTCGGCTCC
TGGATAAGAAGATCCACCCGGGACTCAAGAAACTGCACTGGGCCTTGAAGGGGGCCAGTGCCTTCTTCAT
CACGGAGTGCCGTATACATGCCAGCAAGGTGCAGATGATTGTGAATGAGTTCAAGGCATCCACTCTGACC
ATTGGCTGGCGAGCCCAAGAGATGTCAGAGAAGCTGCTGGTACGCATTAGTGGCAAACGGGTATACAGGG
ACCTGGAATTTGAAGAGGACCAAAGAGAGCATCGGGCAGCTGTACAGCAGAAATTGATGAACCTGCACCA
GGATGTGGTGACCATCATGACCAACTCCTATGAGGTCTTCAAGAATGATGGTCCTGAGATTCAGCAGCAG
TGGATGCTGTACATGATTCGGCTGGACCGCATGATGGAGGATGCCCTGCGCCTGAATGTGAAGTGGTCAC
TGCTAGAACTATCCAAGGCTATCAACGGGGATGGAAAGACCAGCCCAAACCCACTCTTCCAAGTCCTTGT
CATTTTGAAGAATGATCTGCAAGGAAGTGTGGCACAGGTGGAATTCTCACCCACTCTGCAGACTTTGGCA
GGTGTGGTCAATGACATTGGCAACCACCTCTTTTCCACCATCTCTGTCTTCTGCCACCTCCCTGACATTC
TCACCAAGCGCAAGTTACATCGTGAACCCATCCAAACAGTTGTGGAGCAAGATGAGGACATCAAGAAGAT
CCAGACCCAAATCAGCAGCGGCATGACTAACAACGCAAGCCTGCTGCAGAACTACCTCAAGACCTGGGAC
ATGTACCGGGAGATCTGGGAGATCAACAAGGACTCCTTCATTCATCGCTACCAGCGCCTCAACCCTCCTG
TCTCTTCTTTTGTTGCCGACATTGCCCGCTACACGGAAGTTGCTAATAACGTGCAGAAGGAGGAGACAGT
CACCAACATCCAGTTTGTGCTGCTGGACTGTTCGCACCTCAAGTTCTCCCTGGTGCAGCACTGCAATGAA
TGGCAGAACAAGTTCGCGACTCTGCTCAGGGAGATGGCTGCTGGGCGCCTCCTGGAGCTGCACACCTACC
TGAAGGAGAACGCAGAGAAAATCAGCCGCCCTCCGCAGACACTGGAGGAACTGGGGGGTCAGCTTGCAGCT
CGTGGATGCCCTGAAGCACGACTTGGCCAACGTGGAGACTCAGATCCCTCCCATACACGAGCAATTTGCC
ATTCTTGAAAAGTACGAGGTGCCAGTCGAGGACAGTGTCCTGGAGATGCTGGACAGTCTCAACGGGGAGT
GGGTTGTCTTCCAACAAACTCTGCTGGACAGTAAGCAAATGCTGAAGAAACACAAGGAGAAATTCAAGAC
AGGCCTGATCCACTCGGCAGATGACTTCAAGAAGAAAGCACATACACTTCTGGAAGATTTCGAATTCAAA
GGCCATTTCACCAGCAACGTGGGATACATGTCTGCCTTAGACCAGATTACACAAGTGCGGGCCATGCTGA
TGGCCATGCGGGAAGAGGAAAATAGTCTCCGAGCCAACCTGGGCATCTTCAAGATCGAGCAGCCACCCTC
CAAGGACCTTCAGAACCTGGAGAAGGAGCTCGATGCCCTCCAGCAAATCTGGGAGATCGCACGAGACTGG
GAGGAGAACTGGAATGAGTGGAAGACTGGCCGGTTCCTGATCCTGCAGACGGAAACCATGGAGACCACGG
CCCACGGGCTGTTTCGTCGCCTCACAAAATTAGCCAAAGAGTATAAGGACCGAAACTGGGAAATTATTGA
AACCACTCGCTCAAAAATAGAGCAGTTCAAGAGGACCATGCCTCTCATCTCAGACCTGCGGAACCCTGCC
CTTAGAGAGAGGCACTGGGACCAGGTCCGGGATGAGATCCAGCGGGAGTTTGATCAGGAATCTGAAAGCT
TCACCTTGGAGCAGATTGTGGAGCTTGGGATGGATCAGCATGTGGAGAAAATTGGGGAGATCTCTGCTTC
AGCAACTAAAGAGCTGGCTATAGAAGTGGCTTTACAAAACATTGCCAAGACCTGGGATGTGACTCAGCTC
GACATAGTACCCTACAAGGATAAGGGCCATCATCGGCTCAGAGGTACAGAAGAAGTATTCCAGGCACTGG
AAGATAACCAGGTAGCTCTGTCTACCATGAAGGCATCACGCTTTGTCAAGGCCTTTGAGAAGGATGTGGA
CCACTGGGAACGCTGCCTCTCCCTCATTTTGGAGGTTATTGAGATGATTCTCACAGTGCAGCGTCAGTGG
ATGTACTTAGAGAATATCTTCCTAGGAGAAGACATCCGCAAGCAGCTGCCCAATGAATCGACCTTATTTG
ACCAGGTCAACAGCAACTGGAAAGCCATCATGGACAGGATGAACAAGGACAACAATGCTCTCCGGAGCAC
CCATCACCCAGGCCTCCTGGACACATTGATAGAAATGAATACAATCCTGGAAGATATTCAGAAATCTCTG
GATATGTATTTAGAGACCAAGCGACATATTTTCCCCCGCTTCTACTTCTTGTCCAATGATGACCTGCTGG
AGATTCTGGGCCAGTCCCGAAACCCAGAGGCTGTGCAGCCACACCTCAAAAAATGCTTTGACAACATCAA
GTTGCTGAGAATCCAGAAGGTTGGAGGGCCCAGCAGCAAATGGGAAGCTGTGGGGATGTTCTCGGGCGAC
GGCGAGTACATTGACTTCCTCCACTCAGTATTTTTAGAAGGCCCTGTGGAGTCCTGGCTTGGCGATGTGG
AACAGACCATGAGGGTGACCCTGCGGGACCTTCTCCGGAACTGCCACCTGGCCCTCAGGAAGTTCCTCAA
CAAGAGGGACAAATGGGTGAAGGAGTGGGCTGGCCAGGTGGTGATCACTGCCAGTCAGATCCAGTGGACG
GCTGATGTCACCAAGTGCCTGCTGACAGCGAAGGAGCGGGCAGACAAGAAATCCTCAAGGTCATGAAGA
AGAACCAGGTGTCAATCCTGAATAAGTATTCAGAAGCCATCAGGGGGAACTTGACCAAGATCATGCGGCT
TAAAATTGTGGCTCTGGTGACGATAGAAATTCATGCCCGGGATGTGTTGGAGAAGCTTTACAAGAGTGGC
CTCATGGATGTCAATTCCTTTGACTGGCTCAGCCAACTTCGGTTCTACTGGGAGAAGGATCTTGATGACT
GTGTCATCCGCCAGACCAACACGCAATTTCAGTATAATTATGAGTACTTGGGTAACTCGGGCCGGCTCGT
CATCACCCCCCTGACGGACAGGTGTTACATGACACTGACCACGGCATTGCACCTGCACCGAGGGGGCTCC
CCCAAAGGCCCTGCAGGCACAGGCAAGACCGAGACCGTCAAGGACCTGGGCAAGGCCCTGGGCATATATG
```

TCATTGTGGTCAACTGCTCTGAGGGCCTGGACTACAAGTCCATGGGCCGAATGTACTCAGGTCTGGCCCA
GACTGGAGCTTGGGGCTGCTTTGATGAGTTTAACCGCATCAACATCGAGGTGCTGTCAGTGGTGGCCCAC
CAGATCCTGTGCATCCTGTCTGCCCTGGCTGCCGGCCTCACCCATTTCCATTTTGATGGCTTTGAAATAA
ATCTGGTGTGGTCCTGTGGGATCTTCATTACCATGAATCCTGGCTATGCTGGCCGCACAGAGCTTCCCGA
AAATCTTAAATCCATGTTCCGCCCAATTGCCATGGTGGTGCCTGACTCCACCCTCATTGCAGAAATCATT
CTCTTTGGAGAGGGCTTTGGCAACTGCAAGATTCTGGCCAAGAAGGTGTACACACTCTACTCACTGGCTG
TGCAGCAGCTGTCCAGACAGGACCACTATGACTTTGGCCTGCGTGCCCTCACCTCCCTTCTGCGCTATGC
TGGCAAGAAGCGCCGCCTACAGCCGGATCTGACTGATGAAGAGGTTCTGCTGCTCTCAATGAGAGATATG
AACATCGCCAAGCTCACTTCAGTTGATGCACCCCTGTTCAATGCCATCGTGCAAGATCTGTTTCCCAACA
TTGAGCTGCCTGTCATTGACTATGGCAAGCTGCGGGAGACCGTTGAGCAGGAGATTCGAGACATGGGCCT
GCAAAGCACGCCGTTCACCCTCACCAAGGTTTTCCAGTTGTATGAAACCAAGAACTCCCGCCACTCCACC
ATGATCGTGGGCTGCACGGGCAGCGGCAAGACTGCCTCATGGCGCATTCTACAGGCCTCCCTGTCCTCTC
TGTGCCGCGCCGGAGACCCTAACTTCAACATTGTTAGAGAGTTCCCTTTGAACCCCAAGGCATTGTCCCT
AGGGGAACTGTATGGGGAATATGACCTCAGCACCAATGAATGGACAGATGGCATCTTGTCCAGTGTCATG
CGGACGGCATGTGCAGATGAGAAACCCGACGAGAAGTGGATCCTGTTCGATGGCCCCGTGGACACACTGT
GGATCGAGAACATGAACTCCGTCATGGACGATAACAAGGTGTTGACCCTCATCAACGGCGAGCGCATCGC
GATGCCCGAGCAGGTGTCTCTCCTGTTTGAAGTGGAGGACCTGGCAATGGCCTCTCCGGCCACTGTATCC
CGCTGCGGGATGGTCTACACTGACTACGCTGACCTGGGCTGGAAGCCCTATGTTCAGTCATGGCTGGAGA
AGAGGCCAAAGGCTGAGGTGGAGCCCCTTCAACGCATGTTCGAAAAGCTCATCAACAAGATGCTGGCCTT
TAAGAAGGACAACTGCAAGGAGCTGGTGCCCCTGCCCGAGTACAGCGGTATCACCTCCCTCTGCAAGCTG
TACTCTGCCCTGGCCACGCCAGAGAATGGGGTGAACCCAGCTGACGGCGAGAACTATGTCACCATGGTAG
AGATGACATTTGTGTTCAGCATGATCTGGTCTGTGTGTGCCTCTGTGGATGAGGAGGGCCGGAAGAGGAT
CGACAGCTACCTCCGAGAGATCGAGGGCTCCTTTCCCAATAAGGACACGGTATATGAGTATTTTGTGGAC
CCCAAAATACGGAGTTGGACATCATTTGAGGACAAGCTCCCTAAGAGTTGGCGCTACCCTCCAAACGCCC
CCTTCTATAAGATCATGGTGCCCACCGTCGACACTGTTCGCTACAACTACCTGGTGAGCAGCTTGGTGGC
CAACCAGAATCCCATTCTGCTGGTGGGTCCCGTGGGGACTGGGAAGACCTCCATCGCCCAGAGCGTTCTG
CAGTCCCTGCCCTCCAGCCAGTGGTCGGTGCTCGTTGTCAACATGTCCGCACAGACCACATCCAATAACG
TGCAGAGCATCATTGAGAGCAGGGTTGAGAAGCGAACCAAGGGTGTCTACGTGCCATTCGGGGGCAAAAG
CATGATCACCTTTATGGATGACCTAAATATGCCCGCTAAGGACATGTTTGGGTCCCAGCCACCCCTGGAG
CTGATCCGCCTCTGGATTGACTATGGCTTCTGGTATGACCGTACGAAGCAGACCATCAAGTACATTCGAG
AAATGTTCCTGATGGCTGCCATGGGCCCCCCTGGGGGTGGACGGACTGTCATCTCCCCAAGGCTACGGAG
TCGCTTCAACATTATCAACATGACCTTCCCCACAAAGTCCCAGATCATCCGCATATTCGGCACCATGATC
AATCAGAAGCTTCAGGACTTTGAGGAAGAGGTGAAGCCCATTGGGAACGTGGTGACAGAGGCCACCCTGG
ACATGTACAACACCGTGGTACAGCGCTTCCTGCCCACGCCCACCAAGATGCATTACCTCTTCAACCTTCG
AGACATCTCCAAGGTGTTCCAGGGCATGCTTAGAGCCAACAAGGACTTCCATGATACCAAGTCCAGCATC
ACACGGCTCTGGATCCATGAATGTTTCAGAGTCTTCTCTGACCGGCTGGTTGATGCGGCAGACACAGAAG
CCTTCATGGGCATCATAAGCGACAAGCTCGGCTCCTTCTTTGACCTCACATTTCATCATCTCTGTCCCAG
CAAGCGTCCTCCTATCTTTGGGGATTTCCTGAAGGAGCCCAAGGTGTATGAAGACCTCACGGATCTGACA
GTGCTGAAGACAGTCATGGAGACAGCTCTAAATGAGTATAACCTGTCACCCTCTGTCGTGCCCATGCAGC
TAGTGCTCTTCCGAGAGGCTATTGAACACATCACACGGATCGTGCGGGTCATTGGACAGCCTCGGGGCAA
CATGCTCCTGGTGGGTATCGGGGGCAGCGGACGCCAGAGTCTGGCCCGCCTGGCTTCATCCATCTGCGAC
TACACCACCTTCCAGATCGAGGTCACCAAACATTATCGGAAGCAGGAGTTCCGAGATGATATCAAGCGTC
TGTATCGCCAGGCTGGGGTGGAGCTCAAGACCACGTCCTTCATTTTTGTGGACACCCAAATAGCTGATGA
GTCCTTCCTAGAGGACATCAACAACATCCTCAGCTCAGGCGAGGTGCCCAATCTCTACAAGCCTGATGAA
TTTGAAGAGATCCAGTCGCATATCATAGACCAGGCCCGGGTGGAGCAGGTGCCTGAGTCATCGGACAGCC
TCTTCGCCTACCTCATTGAACGCGTGCAGAACAACCTGCACATCGTGCTCTGCCTCAGCCCCATGGGGGA
TCCCTTCAGGAACTGGATCCGCCAGTACCCAGCCTTGGTGAACTGCACAACCATCAACTGGTTCTCAGAG
TGGCCCCAAGAGGCCCTGCTCGAGGTGGCTGAGAAGTGCCTCATAGGAGTAGACCTGGGAACTCAGGAGA
ATATCCACAGGAAGGTGGCCCAGATCTTTGTCACTATGCACTGGTCAGTAGCTCAGTATTCCCAGAAGAT
GCTGTTGGAACTGCGGAGACACAACTATGTCACACCCACCAAATACCTGGAACTCCTGTCTGGATATAAG
AAGTTGCTGGGAGAAAAACGGCAGGAGCTGCTGGCCCAAGCCAATAAACTGCGGACAGGCTTGTTCAAGA
TCGACGAAACTAGGGAAAAGGTGCAAGTGATGTCGTTGGAGCTGGAGGATGCCAAGAAGAAGGTGGCTGA
GTTCCAGAAGCAGTGTGAGGAGTACCTGGTCATCATTGTGCAGCAGAAGCGGGAGGCAGATGAGCAGCAG
AAGGCCGTAACAGCCAACAGTGAAAAGATTGCAGTTGAGGAAATCAAGTGTCAGGCACTGGCTGACAATG
CCCAGAAAGATCTAGAAGAGGCACTGCCCGCCCTGGAAGAGGCCATGCGGGCCCTGGAGTCTCTGAACAA
GAAGGATATAGGAGAGATCAAGTCTTATGGACGGCCCCCAGCCCAAGTGGAGATAGTGATGCAGGCAGTT
ATGATTCTTCGAGGCAACGAGCCCACATGGGCAGAGGCCAAGAGGCAGCTAGGGGAACAGAACTTCATCA
AGTCACTGATCAACTTTGATAAAGACAATATCTCAGATAAGGTTCTGAAGAAGATTGGGGCCTACTGCGC
CCAGCCTGACTTCCAGCCTGATATCATCGGCCGCGTCTCCCTGGCTGCCAAGTCCCTCTGCATGTGGGTG
CGGGCCATGGAGCTGTATGGGCGGCTATATCGGGTGGTGGAGCCCAAGCGAATCCGAATGAACGCTGCCT

```
TGGCTCAGCTTCGGGAGAAGCAAGCCGCGCTCGCTGAGGCCCAGGAGAAGCTGCGGGAGGTAGCTGAGAA
ACTGGAGATGCTAAAGAAACAGTATGATGAGAAGCTGGCACAGAAGGAGGAGCTTCGCAAGAAGTCTGAA
GAGATGGAGCTGAAGCTGGAGCGAGCTGGGATGCTCGTGTCGGGGTTGGCTGGCGAGAAGGCCAGATGGG
AGGAGACAGTCCAGGGCCTGGAGGAGGACCTGGGCTACCTGGTGGGGGACTGTCTCCTGGCAGCTGCCTT
CCTGTCCTACATGGGACCCTTCCTGACCAACTACCGGGATGAGATTGTCAACCAAATCTGGATCGGGGAAG
ATCTGGGAGCTTCAGGTTCCTTGCTCCCCTTCTTTCGCCATCGATAACTTCTGTGCAATCCTACCAAAG
TCCGGGACTGGAACATCCAAGGGTTGCCCTCAGACGCCTTCTCCACTGAGAATGGCATCATCGTCACCCG
AGGCAACAGGTGGGCACTGATGATCGACCCTCAGGCCCAGGCCCTGAAATGGATTAAGAACATGGAAGGA
GGCCAGGGCCTGAAGATCATCGACCTGCAGATGAGCGATTACCTGCGAATCCTAGAACACGCCATTCACT
TTGGATACCCGGTGCTACTTCAGAACGTGCAGGAATATCTGGACCCCACACTGAACCCCATGCTCAACAA
ATCTGTAGCCCGAATCGGTGGTCGGCTGTTGATGCGCATTGGCGATAAGGAGGTGGAATATAATACCAAT
TTCCGTTTCTACATCACCACCAAGCTCTCCAACCCCCACTACAGCCCAGAGACCTCAGCCAAGACCACCA
TCGTCAACTTTGCTGTTAAAGAACAGGGCCTGGAGCCCAGCTGCTGGGCATTGTGGTGCGGAAGGAGCG
GCCTGAGCTGGAGGAGCAGAAGGACTCACTGGTCATCAACATCGCGGCTGGTAAAAGGAAGCTCAAGGAG
CTGGAGGATGAGATCCTGCGGCTGCTGAATGAGGCCACCGGCTCCCTGCTGGATGATGTGCAGCTGGTGA
ACACGCTGCATACCTCCAAGATCACAGCCACAGAGGTGACTGAGCAGCTGGAGACCAGTGAGACCACAGA
GATCAACACTGACTTGGCGCGGGAGGCTTACCGCCCATGCGCCCAGCGGGCATCAATCCTGTTCTTCGTG
CTCAATGATATGGGCTGCATCGACCCCATGTACCAGTTCTCACTGGATGCCTACATCAGCCTCTTTATTC
TCAGCATTGACAAAAGCCACCGCAGCAATAAGCTGGAGGACCGCATTGACTACCTGAATGACTACCACAC
CTACGCTGTCTACAGGTACACCTGCCGTACCCTTTTCGAACGCCACAAACTACTATTCAGTTTTCATATG
TGTGCCAAAATCTTGGAGACTTCTGGCAAGCTCAACATGGATGAATACAACTTCTTTCTACGTGGGGGTG
TGGTCTTGGATCGGGGAGGCCCAAATGGACAATCCATGTAGTAGCTGGCTTGCAGATGCCTACTGGGATAA
CATCACAGAGCTAGACAAACTGACCAACTTCCACGGACTCATGAACTCCTTTGAGCAGTACCTCGTGAC
TGGCACCTGTGGTATACCAATGCTGCCCCGGAGAAGGCGATGCTGCCAGGTGAGTGGGAAAATGCCTGCA
ATGAAATGCAACGGATGCTGATCGTTCGCTCCCTGCGCCAGGACCGCGTGGCCTTCTGCGTGACCTCCTT
CATCATCACCAACCTTGGCTCCCGCTTCATCGAGCCGCCTGTGCTGAATATGAAGTCGGTGCTGGAGGAT
TCAACCCCACGATCCCCACTCGTGTTCATCCTGTCCCCTGGTGTGGACCCCACCAGTGCCCTGCTGCAGC
TGGCAGAGCACATGGGCATGGCCCAGCGCTTCCACGCCCTGTCCCTGGGCCAGGGCCAGGCCCCCATCGC
TGCTCGGCTCCTCCGAGAGGGTGTGACTCAGGGACACTGGGTGTTCCTGGCAAACTGCCACCTGTCACTG
TCTTGGATGCCTAATCTGGACAAGCTGGTGGAGCAGCTGCAGGTGGAGGATCCTCATCCATCCTTCCGCC
TCTGGCTCAGCTCCATCCCCCACCCAGACTTCCCTATCTCAATCTTGCAGGTCAGCATCAAGATGACCAC
AGAGCCACCAAAGGGCCTAAAGGCCAACATGACACGTCTTTACCAACTGATGTCAGAACCACAGTTTTCC
CGCTGCTCCAAACCTGCCAAATATAAGAAGCTGCTGTTTTCACTCTGTTTCTTCCACTCTGTGTTACTTG
AACGCAAAAAGTTCCTGCAGCTTGGCTGGAACATCATCTATGGCTTCAATGACTCCGACTTTGAGGTGTC
AGAAAACTTGCTGAGCCTCTATCTCGATGAGTACGAGGAGACACCTTGGGACGCACTTAAGTACCTCATT
GCCGGCATCAACTATGGTGGACATGTCACAGATGACTGGGACCGGCGCCTGCTGACCACCACATCAATG
ATTATTTCTGTGACCAGTCTCTATCAACTCCCTTCCACCGGTTGTCAGCACTGGAGACTTATTTCATCCC
CAAGGATGGCAGCCTCGCTTCTTACAAGGAATACATCAGCTTATTGCCTGGCATGGACCCCCCTGAGGCC
TTTGGCCAGCACCCCAATGCTGATGTGGCCTCTCAGATCACTGAGGCACAAACCCTCTTTGATACTTTGC
TTTCCTTGCAACCTCAGATTACACCCACCAGGGCTGGAGGCCAGACCCGGGAAGAGAAGGTCCTTGAGTT
GGCCGCTGATGTGAAGCAGAAGATCCCTGAAATGATCGACTATGAGGGGACTCAAAAACTGCTAGCTCTC
GACCCCTCCCCCCTCAATGTGGTCCTTCTGCAGGAGATCCAGAGATACAACACACTGATGCAGACCATCC
TGTTCTCACTGACAGACCTAGAGAAAGGCATCCAGGGTCTCATCGTCATGTCTACAAGCCTGGAAGAGAT
TTTCAATTGCATCTTTGATGCCCATGTTCCTCCGCTCTGGGGAAAGGCATACCCCTCACAAAAGCCATTG
GCTGCCTGGACCCGGGACTTGGCCATGCGTGTGGAGCAGTTTGAGCTGTGGGCCAGCCGGGCCCGGCCTC
CTGTGATCTTCTGGTTGTCTGGTTTCACCTTTCCCACTGGCTTCCTCACTGCTGTGCTGCAGTCTTCAGC
TCGCCAAAACAACGTTTCAGTGGACAGCCTCTCCTGGGAGTTTATCGTTTCCACTGTGGATGACAGCAAC
CTAGTGTATCCCCCCAAGGATGGTGTCTGGGTCCGGGGCCTGTACCTGGAAGGTGCTGGCTGGACCGGA
AGAACTCCTGCTTGGTGGAGGCAGAGCCCATGCAGCTTGTCTGCCTCATGCCCACGATCCACTTCCGGCC
TGCAGAGAGCCGCAAGAAGAGCGCCAAGGGCATGTACTCCTGCCCCTGCTATTACTATCCCAACCGGGCA
GGCAGCTCAGACCGAGCCTCCTTTGTCATCGGCATTGACCTGCGGTCTGGGGCCATGACACCTGATCATT
GGATCAAGAGGGGCACTGCTCTACTCATGAGCCTGGACAGCTGAGACCTCCTCCTCTTCTCCGCTTGAGA
GAGAGGGTCAGGGACTCCAGGAGCTAAGACAGATGTTGCACCTAGGACTGAGGCCGGACCTCACTCAGAC
TTTGACCTTGGCCGAATTTGTGTGATGTGGCCCTGGAGATACCTAGTTGTGTTAGCCATAAAAGTGAAAG
AGTTGTATTGGAGCTCAGTGCTGTAAACACCCGCGACAACAAGC
```

>NM_000719.6 Homo sapiens calcium voltage-gated channel subunit alpha1 C **(CACNA1C)**, transcript variant 18, mRNA

TTATTTTTTTCAAATGGTGTAGCCGCCGGAGGTGCGGTGCTCAGTTCTTGGAAGGGGCCCGGATGTACTGA
GGATGCGTTACAGTTTCACTCGAGGAGGCAGTAGTGGAAAGGAGCAGTTTTTGGGGTTTGATGCCATAAT

GGGAATCAGGTAATCGTCGGCGGGGAAGAAGAAACGCTGCAGACCACGGCTTCCTCGAATCTTGCGCGAA
AGCCGCCGGCCTCGGAGGAGGGATTAATCCAGACCCGCCGGGGGGTGTTTTCACATTTCTTCCTCTTCGT
GGCTGCTCCTCCTATTAAAACCATTTTTGGTCCATGGTCAATGAGAATACGAGGATGTACATTCCAGAGG
AAAACCACCAAGGTTCCAACTATGGGAGCCCACGCCCCGCCCATGCCAACATGAATGCCAATGCGGCAGC
GGGGCTGGCCCCTGAGCACATCCCCACCCCGGGGGCTGCCCTGTCGTGGCAGGCGGCCATCGACGCAGCC
CGGCAGGCTAAGCTGATGGGCAGCGCTGGCAATGCGACCATCTCCACAGTCAGCTCCACGCAGCGGAAGC
GGCAGCAATATGGGAAACCCAAGAAGCAGGGCAGCACCACGGCCACACGCCCGCCCCCGAGCCCTGCTCTG
CCTGACCCTGAAGAACCCCATCCGGAGGGCCTGCATCAGCATTGTCGAATGGAAACCATTTGAAATAATT
ATTTTACTGACTATTTTTGCCAATTGTGTGGCCTTAGCGATCTATATTCCCTTTCCAGAAGATGATTCCA
ACGCCACCAATTCCAACCTGGAACGAGTGGAATATCTCTTTCTCATAATTTTTACGGTGGAAGCGTTTTT
AAAAGTAATCGCCTATGGACTCCTCTTTCACCCCAATGCCTACCTCCGCAACGGCTGGAACCTACTAGAT
TTTATAATTGTGGTTGTGGGGCTTTTTAGTGCAATTTTAGAACAAGCAACCAAAGCAGATGGGGCAAACG
CTCTCGGAGGGAAAGGGGCCGGATTTGATGTGAAGGCGCTGAGGGCCTTCCGCGTGCTGCGCCCCCTGCG
GCTGGTGTCCGGAGTCCCAAGTCTCCAGGTGGTCCTGAATTCCATCATCAAGGCCATGGTCCCCCTGCTG
CACATCGCCCTGCTTGTGCTGTTTGTCATCATCATCTACGCCATCATCGGCTTGGAGCTCTTCATGGGGA
AGATGCACAAGACCTGCTACAACCAGGAGGGCATAGCAGATGTTCCAGCAGAAGATGACCCTTCCCCTTG
TGCGCTGGAAACGGGCCACGGGCGGCAGTGCCAGAACGGCACGGTGTGCAAGCCCGGCTGGGATGGTCCC
AAGCACGGCATCACCAACTTTGACAACTTTGCCTTCGCCATGCTCACGGTGTTCCAGTGCATCACCATGG
AGGGCTGGACGGACGTGCTGTACTGGGTCAATGATGCCGTAGGAAGGGACTGGCCCTGGATCTATTTTGT
TACACTAATCATCATAGGGTCATTTTTTGTACTTAACTTGGTTCTCGGTGTGCTTAGCGGAGAGTTTTCC
AAAGAGAGGGAGAAGGCCAAGGCCCGGGGAGATTTCCAGAAGCTGCGGGAGAAGCAGCAGCTAGAAGAGG
ATCTCAAAGGCTACCTGGATTGGATCACTCAGGCCGAAGACATCGATCCTGAGAATGAGGACGAAGGCAT
GGATGAGGAGAAGCCCCGAAACATGAGCATGCCCACCAGTGAGACCGAGTCCGTCAACACCGAAAACGTG
GCTGGAGGTGACATCGAGGGAGAAAACTGCGGGGCCAGGCTGGCCCACCGGATCTCCAAGTCAAAGTTCA
GCCGCTACTGGCGCCGGTGGAATCGGTTCTGCAGAAGGAAGTGCCGCGCCGCAGTCAAGTCTAATGTCTT
CTACTGGCTGGTGATTTTCCTGGTGTTCCTCAACACGCTCACCATTGCCTCTGAGCACTACAACCAGCCC
AACTGGCTCACAGAAGTCCAAGACACGGCAAACAAGGCCCTGCTGGCCCTGTTCACGGCAGAGATGCTCC
TGAAGATGTACAGCCTGGGCCTGCAGGCCTACTTCGTGTCCCTCTTCAACCGCTTTGACTGCTTCGTCGT
GTGTGGCGGCATCCTGGAGACCATCCTGGTGGAGACCAAGATCATGTCCCCACTGGGCATCTCCGTGCTC
AGATGCGTCCGGCTGCTGAGGATTTTCAAGATCACGAGGTACTGGAACTCCTTGAGCAACCTGGTGGCAT
CCTTGCTGAACTCTGTGCGCTCCATCGCCTCCCTGCTCCTTCTCCTCTTCCTCTTCATCATCATCTTCTC
CCTCCTGGGGGATGCAGCTCTTTGGAGGAAAGTTCAACTTTGATGAGATGCAGACCCGGAGGAGCACATTC
GATAACTTCCCCCAGTCCCTCCTCACTGTGTTTCAGATCCTGACCGGGGAGGACTGGAATTCGGTGATGT
ATGATGGGATCATGGCTTATGGCGGCCCCTCTTTTTCCAGGGATGTTAGTCTGTATTTACTTCATCATCCT
CTTCATCTGTGGAAACTATATCCTACTGAATGTGTTCTTGGCCATTGCTGTGGACAACCTGGCTGATGCT
GAGAGCCTCACATCTGCCCAAAAGGAGGAGGAAGAGGAGAAGGAGAGAAAGAAGCTGGCCAGGACTGCCA
GCCCAGAGAAGAAACAAGAGTTGGTGGAGAAGCCGGCAGTGGGGGAATCCAAGGAGGAGAAGATTGAGCT
GAAATCCATCACGGCTGACGGAGAGTCTCCACCCGCCACCAAGATCAACATGGATGACCTCCAGCCCAAT
GAAAATGAGGATAAGAGCCCCTACCCCAACCCAGAAACTACAGGAGAAGAGGATGAGGAGGAGCCAGAGA
TGCCTGTCGGCCCTCGCCCACGACCACTCTCTGAGCTTCACCTTAAGGAAAAGGCAGTGCCCATGCCAGA
AGCCAGCGCGTTTTTCATCTTCAGCTCTAACAACAGGTTTCGCCTCCAGTGCCACCGCATTGTCAATGAC
ACGATCTTCACCAACCTGATCCTCTTCTTCATTCTGCTCAGCAGCATTTCCCTGGCTGCTGAGGACCCGG
TCCAGCACACCTCCTTCAGGAACCATATTCTGTTTTATTTTGATATTGTTTTTACCACCATTTTCACCAT
TGAAATTGCTCTGAAGATGACTGCTTATGGGGCTTTCTTGCACAAGGGTTCTTTCTGCCGGAACTACTTC
AACATCCTGGACCTGCTGGTGGTCAGCGTGTCCCTCATCTCCTTTGGCATCCAGTCCAGTGCAATCAATG
TCGTGAAGATCTTGCGAGTCCTGCGAGTACTCAGGCCCCTGAGGGCCATCAACAGGGCCAAGGGGCTAAA
GCATGTGGTTCAGTGTGTGTTTGTCGCCATCCGGACCATCGGGAACATCGTGATTGTCACCACCCTGCTG
CAGTTCATGTTTGCCTGCATCGGGGTCCAGCTCTTCAAGGGAAAGCTGTACACCTGTTCAGACAGTTCCA
AGCAGACAGAGGCGGAATGCAAGGGCAACTACATCACGTACAAAGACGGGGAGGTTGACCACCCCATCAT
CCAACCCCGCAGCTGGGAGAACAGCAAGTTTGACTTTGACAATGTTCTGGCAGCCATGATGGCCCTCTTC
ACCGTCTCCACCTTCGAAGGGTGGCCAGAGCTGCTGTACCGCTCCATCGACTCCCACACGGAAGACAAGG
GCCCCATCTACAACTACCGTGTGGAGATCTCCATCTTCTTCATCATCTACATCATCATCGCCTTCTTT
CATGATGAACATCTTCGTGGGCTTCGTCATCGTCACCTTTCAGGAGCAGGGGGAGCAGGAGTACAAGAAC
TGTGAGCTGGACAAGAACCAGCGACAGTGCGTGGAATACGCCCTCAAGGCCCGGCCCCTGCGGAGGTACA
TCCCCAAGAACCAGCACCAGTACAAAGTGTGGTACGTGGTCAACTCCACCTACTTCGAGTACCTGATGTT
CGTCCTCATCCTGCTCAACACCATCTGCCTGGCCATGCAGCACTACGGCCAGAGCTGCCTGTTCAAAATC
GCCATGAACATCCTCAACATGCTCTTCACTGGCCTCTTCACCGTGGAGATGATCCTGAAGCTCATTGCCT
TCAAACCCAAGCACTATTTCTGTGATGCATGGAATACATTTGACGCCTTGATTGTTGTGGGTAGCATTGT
TGATATAGCAATCACCGAGGTAAACCCAGCTGAACATACCCAATGCTCTCCCTCTATGAACGCAGAGGAA
AACTCCCGCATCTCCATCACCTTCTTCCGCCTGTTCCGGGTCATGCGTCTGGTGAAGCTGCTGAGCCGTG

```
GGGAGGGCATCCGGACGCTGCTGTGGACCTTCATCAAGTCCTTCCAGGCCCTGCCCTATGTGGCCCTCCT
GATCGTGATGCTGTTCTTCATCTACGCGGTGATCGGGATGCAGGTGTTTGGGAAAATTGCCCTGAATGAT
ACCACAGAGATCAACCGGAACAACAACTTTCAGACCTTCCCCCAGGCCGTGCTGCTCCTCTTCAGGTGTG
CCACCGGGGAGGCCTGGCAGGACATCATGCTGGCCTGCATGCCAGGCAAGAAGTGTGCCCCAGAGTCCGA
GCCCAGCAACAGCACGGAGGGTGAAACACCCTGTGGTAGCAGCTTTGCTGTCTTCTACTTCATCAGCTTC
TACATGCTCTGTGCCTTCCTGATCATCAACCTCTTTGTAGCTGTCATCATGGACAACTTTGACTACCTGA
CAAGGGACTGGTCCATCCTTGGTCCCCACCACCTGGATGAGTTTAAAAGAATCTGGGCAGAGTATGACCC
TGAAGCCAAGGGTCGTATCAAACACCTGGATGTGGTGACCCTCCTCCGGCGGATTCAGCCGCCACTAGGT
TTTGGGAAGCTGTGCCCTCACCGCGTGGCTTGCAAACGCCTGGTCTCCATGAACATGCCTCTGAACAGCG
ACGGGACAGTCATGTTCAATGCCACCCTGTTTGCCCTGGTCAGGACGGCCCTGAGGATCAAAACAGAAGG
GAACCTAGAACAAGCCAATGAGGAGCTGCGGGCGATCATCAAGAAGATCTGGAAGCGGACCAGCATGAAG
CTGCTGGACCAGGTGGTGCCCCCTGCAGGTGATGATGAGGTCACCGTTGGCAAGTTCTACGCCACGTTCC
TGATCCAGGAGTACTTCCGGAAGTTCAAGAAGCGCAAAGAGCAGGGCCTTGTGGGCAAGCCCTCCCAGAG
GAACGCGCTGTCTCTGCAGGCTGGCTTGCGCACTGCATGACATCGGGCCTGAGATCCGACGGGCCATC
TCTGGAGATCTCACCGCTGAGGAGGAGCTGGACAAGGCCATGAAGGAGGCTGTGTCCGCTGCTTCTGAAG
ATGACATCTTCAGGAGGGCCGGTGGCCTGTTCGGCAACCACGTCAGCTACTACCAAAGCGACGGCCGGAG
CGCCTTCCCCCAGACCTTCACCACTCAGCGCCCGCTGCACATCAACAAGGCGGGCAGCAGCCAGGGCGAC
ACTGAGTCGCCATCCCACGAGAAGCTGGTGGACTCCACCTTCACCCCGAGCAGCTACTCGTCCACCGGCT
CCAACGCCAACATCAACAACGCCAACAACACCGCCCTGGGTCGCCTCCCTCGCCCCGCCGGCTACCCCAG
CACGGTCAGCACTGTGGAGGGCCACGGGCCCCCCTTGTCCCTGCCATCCGGGTGCAGGAGGTGGCGTGG
AAGCTCAGCTCCAACAGGTGCCACTCCCGGGAGAGCCAGGCAGCCATGGCGGGTCAGGAGGAGACGTCTC
AGGATGAGACCTATGAAGTGAAGATGAACCATGACACGGAGGCCTGCAGTGAGCCCAGCCTGCTCTCCAC
AGAGATGCTCTCCTACCAGGATGACGAAAATCGGCAACTGACGCTCCCAGAGGAGGACAAGAGGGACATC
CGGCAATCTCCGAAGAGGGGTTTCCTCCGCTCTGCCTCACTAGGTCGAAGGGCCTCCTTCCACCTGGAAT
GTCTGAAGCGACAGAAGGACCGAGGGGGAGACATCTCTCAGAAGACAGTCCTGCCCTTGCATCTGGTTCA
TCATCAGGCATTGGCAGTGGCAGGCCTGAGCCCCCTCCTCCAGAGAAGCCATTCCCCTGCCTCATTCCCT
AGGCCTTTTGCCACCCCACCAGCCACACCTGGCAGCCGAGGCTGGCCCCCACAGCCCGTCCCCACCCTGC
GGCTTGAGGGGGTCGAGTCCAGTGAGAAACTCAACAGCAGCTTCCCATCCATCCACTGCGGCTCCTGGGC
TGAGACCACCCCCGGTGGCGGGGGCAGCAGCGCCGCCCGGAGAGTCCGGCCCGTCTCCCTCATGGTGCCC
AGCCAGGCTGGGGCCCCAGGGAGGCAGTTCCACGGCAGTGCCAGCAGCCTGGTGGAAGCGGTCTTGATTT
CAGAAGGACTGGGGCAGTTTGCTCAAGATCCCAAGTTCATCGAGGTCACCACCCAGGAGCTGGCCGACGC
CTGCGACATGACCCATAGAGGAGATGGAGAGCGCGCGGCCGACAACATCCTCAGCGGGGGCGCCCCACAGAGC
CCCAATGGCGCCCTCTTACCCTTTGTGAACTGCAGGGACGCGGGGCAGGACCGAGCCGGGGGCGAAGAGG
ACGCGGGCTGTGTGCGCGCGCGGGGTCGACCGAGTGAGGAGGAGCTCCAGGACAGCAGGGTCTACGTCAG
CAGCCTGTAGTGGGCGCTGCCAGATGCGGGCTTTTTTTTATTTGTTTCAATGTTCCTAATGGGTTCGTTT
CAGAAGTGCCTCACTGTTCTCGTGACCTGGAGTTAACCGGAACAGCGTCTTCATTCATTTCTGTTGGGAC
CAGACGCGGAGCCTGGGTGCGCGAGCCGCCCTCCGGGAGGAAGGCGCCCGGCTGCGTCTGCAGAGGCGGG
GAGAGGAGGCGGCGAGGGTCCCGGGGCGCGAGGAAGGCGCCTGCCCTCTCCCAGCTCGCAGGCCCCGGGC
CCGGCCGCGCCTCCGCGGGGAGAGCACCCCGGCTTCCCGCGCGCCCTCACCAAAAGGACCCTACAGCAAA
CGGGTGTCTTTCGACTCTGCTTGTAGAAACCATTTGCACATATTCTGTACGAGCCTCGCTGTCTCCCTAG
AGCCAGGGCCCTGCGGATTTGGAGAAGGGAGCGGGGCAGGACTTCCAGGAGGACCCCAACCCGGCCCGGA
GAGGGAGGAGGAGGCCTCCAGGGGCGCGGAGCTCTGGGGATGGGCGTCGGGCCGGCAGTGGTGCGGCTCA
CTCCGTCCCTGCCCACCTGCGACGGGATCCCCCGACCGGCACGGGCCACGCCGAGCTCCCGGCCAGCCGC
CGGCCCGCAGGCAGCGCGAGGGAGGAGCTGCGCCGCCGGCTCCGCCCAACCAGGTGGTGCTGAGCTTCCG
CTGAGCGCTCTTTTGTTTTGTGGTTTGACACTTTTCTTGACAGCATGTTGCAGTTTCTTTTCGGTTTTGG
TTTTTTTTAAATGTTTTATTTTGCTTTCCCAGCGGGAGGGGAGGAAGAAGAGTGTTTACAAAGTCCTGTA
GCCCCCTCACCTTTCTGTTTTCACTTTTGCCAATGTACATCGGGTTTGGTTTTCTTGTATTATTTAAACG
GTTGTGGTTTCCTTTTTCCACGGAGGTTCAATAGAAGCCGCTGCAGGAGAGTTTTACCAACCATTGTGTA
TGCCCAATAATTTGTTATCATTTCCTTAGGTAGTAACCTATTTTTGTTCTGGTTTGGTTCGGTTATCTAA
TGGAAAGGTAACTGGCAATGCACTTGATGTGGTCTTGCACATGTGGGTGATAGAGTTGGGTTCCTTTTTA
TGCTGGGTGTACAGGTGGGTTTGGGAGAGAGGAGCATGCGCGAGAGAGTCTCCGAGTGTGTGCGACGCGT
GTGTGTGTGGTGGGTTGTCTGTGTGCATATGTCCTGCCCGTGTATATGCACCCACACCATGTGCCCGTGC
ACACCAGTGACTACGCAGTCCCCCCTTTCTGGTTTAGCTGTGGGAAGATCTGAATCTGGGGCCGTTTGAA
AGCAAAACAAACCACTGTCTCTGCTTCTGAAACGGGAATCAGTAACTCTTTGCATTTTCTGTCCCACAA
GATATGCAAAACAATGCAATAATATTCATTTAAAAATACAATTGTGAGTTGTGTTGGCATTAAAACTGT
ATTTTAAAAAAAGACAGAAATTTAAGGGAAAAACACAAGAAGGCATTTTGCTTCAATATATTCCGTGTAA
TGTTTTATTGCATTGATAATGTTTCTGTTGAAGAAACCGTTATACTTGAATTCAGGTCAGTTTCAGTATT
TTTCAAATATTTTTTTAAAACTGAATTGCAATTGTGCCAAGCGAATATAATGAATTGAATTAAGTTGGTT
TTCGGATTCACTTCTTGTATATTTTGCTGCATGTAAAGTAAATCATTTTGTATTTGGAGTGTGACAAGCT
TTACCTTTGAACTCAAGTGCTTTTCTATATGTGGTTGGGGGAAAGGGAACAAGTTTTCTTTAGTTTGCAC
```

```
AATGAGCAAAGGTATCACCAGTGTAGTCATTATTCTGCTCTCCACAAACAGGTTTGGACATTACTGTTTT
GCATATCTTGTGTTTGCTTACATTTCCCTCAATTTTTCCAAAATCGTTTGCTGGGTATGTTTGTACCGCC
TCTTGCTGTGAGAGACCAGGACCTATTTTATTCCAGTCTTCACTCTGTCCACTCTGCTCTGGTCATCTGA
TTTGGTACTTCTCCAAGAACAGCCCTTCACTGTGAGGTGCAGGGAGGCGTTCTGATGAGCCCTCAGTCAC
TGGGCCGTCATCCGCATCCCCCATGGAAGAGGTAGCTGGCTTTCCCTTCCCTTCCACCACACGGAATTTT
CTCTTTGGCTTCCTTAGGAAAGTGTACACTAACCGGGAGGATAAAATTAAAGTCAGGCTGCTTGGAGGGA
GGGGCATCCTCACTTCCGGATTCTTGTTGCTCTACCCAACAAGGACAGCAGGGGCTCGAGAAAGGAACTG
GTGAAACCCTGATCCATCTGAAAGTCAACTCTGCGTGCTCCTTTCTCCATCCCTTCCTCACTCTGGAGCA
GCCTTTCCTTCAGGCTTGCCCTAATGTTTGGGCTGCCGGGGAGGGGGCCAGGACAAGGGAAGAGGCATCC
GGAGCTCACAGTGGGGGTGGGAACAGATTTTTGTGGGGGCATCTCTAATGCTCACTTATATCTCCCTAGA
ACATCACTCTTTTGGTGCTGTGTCCTTCAAATGTATGTCAACAGTGGTGGCTGAAAAGGGACTGCTTTGG
GGAAAACAGGACCCAACCATTCACCCAGAATTGACCCATTAAATCTCTTCCAGTCCTAGTGTTCCCTGAG
CCCCTCTTGGCACATATATAAGTAAGCTAGAAATTACAATAAGGGACAGTCCATTCCTCTATGACAGCTT
GCTGGACTGATTCATGACAAAGTGGAGAAATGTACTCAATACTCCCGGTTAACACAGTCTAGAAACAGA
GTTTCTTTATGGATATCCACACCCAAGTCATCCAAACTTTCTTGATTCCTTTTCACTGCCATCAAGGTCC
TCTAGAAATTGAGTTTAGGTATCATCCTTTGAAAGTTCCCAAGATTTCTACCAGGAGGTACACACAGGC
GTTCCCTGTCTAGGGCAGGAGGACTATCCTAGCTTGACCTTCTGATCCACTAGAATAAGACTGGCGTATG
ATGCCTGTCATCAGAACAGACTGGCACAAGTAGTGACATCAATGAACCACAGCACAATCTTCCAAGTGAT
GTCTACTCTCCACCTAAAATGGAATTTTCCCCATGACCTTGTAAAACATAATTGTCACATCTTCCATACC
CCTCCTGACAGCCCCCAAGTGTCAGGAGAAACAGTCAGGGGCTAAGGGCCCAAGGGACTTGAAGAAACA
ACAGTTTAAGGTCTGCAGTTTGGTCAACTTAATTCTTGTCCTCCGACCAGCCCTGCCTCTTTCATTTCCA
GACCTTGGAGAATTTTTCCCAGCTTTGATTCAGAAGGTACTAGTTATAACCCCTTTCCTTCTTCTTAATC
CAATAGGCCTCACTCTCACTGGGAAATCCACTCAAAGGAACAAGGCAATGTCTCTCATTCTATTTCCCAG
TTCCAAATTCCAGGTGCTTGTCTGGAGTGAAGCTACCCGTTACTTTCTCCCAGCTTTTCTCCACCCAGCA
TGTCTCCTGCCCATGCAGCTGAAGACAGTGGGGCAACCTCAGGAGAAGCAGACCTTTCCATGCCCAAGTT
CATCTCCTGAGCAACAGTGACACCTAGAAAATGAGGACTTTGGAAGTCACCCAAAAGATGGTGGCTACTT
TATGGAGTCCTGAAGATACACAGCCACCACTCCTAAAGGCAAAGAAAGAAACACGAATGTAGGTCAGGG
ATAGAGTGGAACCCTGGTCATCGGGGTTTTTAGCCTCATCGTGGGAAAGGTGGTAAAGGAGGATGATGGC
ATCTCCATCCCTAGAGGCCAAGAATTGAAATATCATTGTCAAGGATTAGAAACAATTCAGCAAAGAGGCC
ACAAAAAGGGCCTGCTGACTCCCAGAAGACCTCTTTAAACCCCAGGGGAGGCAAATACTTGCTGATGGAG
TCTGGGCCGTTTCCATATTTTAAAGAAGACCTGCCTCTGGGGCAAATGTCAGCACAGAGAGGACTGGGAG
GAGAATGGAGGCAAGAAAAGGGCATATTTTGACTCCCTCTGTGCCTCTTCCCAGTTCATGGAAGGATGTG
TTCAGCTTACCCACCCACAGTGACCAGTGTGGTGGAGCCGCTGACATCTCAAGGATCTATTTGGGAAGGT
GAGAAGAGTACTCATTCCATCTGGGGGTGTTGTTCCAGCCACATCAGCCTACCTGGTGGGATGTGGGGGT
GTCTGCCACCCTGTCCCCCCTCTGCTGATGTCCCTCCCCTCAGGCTGTCCAGGTGCCACCTGACACAGGC
TGCTGTGCAAAGACAGGCGGGGAAGCCCAAACCTCACTCCCAGGGAGGCCCTCAGCCGCCAGAGTCCAGG
TTCTCCAGAGGCTACGATTTGAGGAGGTTGAGGGGGAAGACAGGAGGGAAAGAAAAGTCCTACAACTGTC
AGGAATGGGGCACCTTTCCCTGTCCCTAAGCAAAGCTCCCTCTTCCCACTGCCCTCCCCAGCCCCAGCTC
CCTGTCCTCCCCAACACCTAGTGAGAAAGACGGTGCGTGGAAGGGAGTCCCATGGGCAGATGCTTACACG
ACCTCTTTGTGAAGCCTCTTCTGGGTTTAACTTCATTCATCAATTTATTCTTATGTCAAAGCAATGAAAC
TTTTCTTTCTGGAGCCAGATACCAATACAACAGGTGAACGGGTTTCTGCCACATCTCTACATTGACGGGG
GATGCTTGAACAACCCCCCTCACTACACAGACACACACCGTTAAGGCACAAGGGCTGGGGTTGAGCTCTA
GATGAGGGACTTTCCTGCTCCTGCAAGGGTGAGCACTGTATACACAGACAAGGAGGGTGCAGTAGAGTGA
CTCCCTTGGATGGAAGTAGTACCATCAGAACCTACTATTATTATGACATAAATTCTATTTACATACATTG
AGAGAATACTACAATCAACACTTTTTCCTGGGATGACTTTAAGAGGTTTGAGCCACAGCACCTGAAGTGG
CAAAGATCCATGGTCTTTGTAGGGTATTAGAGAACTCTTCCAGTCACCTCTGAAAGCACTCTAGATCTTG
CAGCTGAGTGGATGAAGTGTAACAAATCTGTTGCACGCTGAGAGGAGTCAGAATTAGCATTTTTCATGAA
AGTTCCCCACGTCTCTACTAAGAATGAGGAAGAAAAGACTAAGACTAGGTAATTACACAGAGGCTTGAAA
TGTTACATCACCAGAGCCAAGTCCTCTCCCTTCAGATCAGTTACTGGCTGCTACACAGGGACACCCCCAC
CTTTTCAGGGCATCCCATGCACTCCACTTCTCAGGATCTAAGGAATTTGACTTTGTAGGGATCCCAGAAA
GGGCACTGTGCCACTTCCCTGGTGTGAATCAGACATACATTGTACATTCATTTCTAAAATTCACTCATG
CACCTCAAACCAAGGTCATTATCCAAAAAAAAAAAAAAAAAAGCTCTGGGTGGAAGAGTTTGTAAGTTTTA
AGAGAGGGTCATTTCTATGTGAGGAAATGCAGAAATGGACAGAATGATTCTTATTCACTGTTTGGGTCTG
GAGAATTCCCATTGTGGAAATCTTAGAGATCTCAAGTTTATTACCAAGGGAATAAGGAAAAAAAGGTGAG
CAGGCACCAGGCCAAGCAGTGGCTCCCTTGCCAAGGAACCTGAGGCTGCAGGTTTCAGGGACCCCCTTGA
AGAAACCTCTCCTGGCCATTGGCCAGGAGAAAGAGAAGTCTCTCCTGTAGAGTCACAAGAGAAGCAAAA
GAGGGTGGGTCACTGGGTCCTGGACATAGCCCCCAACCCCAAGACTTCCCAATATGGAGAAACTACACCA
ATGTTTAAAAGGGGAAAAGGAAAGAACTTGTACATCAAGGGAAGATGATTTGTAAACACACAGTCCTGTG
CAGAAAGATCCCCTTCAGGAGGTGTCTCCAGCATCCCAAAGCTGTGCGCACCTTCTCTTTTCCTGCCTCA
GGCCACCTATGCATCCAGCTGCAGCCCATACCCACACCTGAAATCCATCTCTTGAATCCCAGCCAGGTTA
```

```
TATACCACCCCATTGCCATGTCCTGTCCTGGCCAGAATGCATGCTGTTCCCCCAAGCCTCGTGGGAGTGA
GGCCATGGGAAACAGAGATGAGCATGTCTGGACAAGTCTGTGATGGTAGTGGATGAGAATAACCCATGGC
AAAACACGCACATTCATTAAGAAATAGGGCGACAGATTCCCCGTTGGTGAAGCACTGAAAGGTCTATTAC
TCTCATAATATTGCTGTTTTATTTTAATCCACCAGAGCTACCATGCAAAACTTTCCTCCTGTGAAACGCT
CCAGATAAAGCTCCTCTAATCTCCCCTTCCCTCATGTCCTCCAGCTCAAACCCACCTTCATCCCCCAAAC
CAATCTGTATCATGCCTGTTATCAGAGAGGCACAGAAAGATGGGCAGTGCCTCCGTTGTCACCATTCCCC
ACACCCCTACACACCCCCACACCCTCCCCTCCAGGCTCCACGACTTCACAGTCTTACTGTTGTAAATATC
ATTGTACAGTTTGTAATCCTCAAATAATCCCATTGTCAGAGGCCTCGCCTGATGGGCCTTCTCACCCTCG
AGAAAGGCCAGGGAATCTAGAAGGGGCAACCCTTCAAGGAGAGCTTCAGGGTCATCTCTGTGTGAGACAC
TATTGTATATTCCTGTAAGATTGCATTTTTATCTAAGGAATGATGTTATTTAAAAAACAAACAAAAAACA
CAAAAAATAAGAATTGCAAATAAATTTCTTAACAATGTCT
```

>NM_014316.3 Homo sapiens calcium regulated heat stable protein 1 **(CARHSP1)**, transcript variant 1, mRNA

```
GCTCTCAGTCGGAGCGAAGCGGCTGGCGGAGCAGAACGGATTGCAGGGTCAGCCATGTCATCTGAGCCTC
CCCCACCACCACAGCCCCCCACCCATCAAGCTTCAGTCGGGCTGCTGGACACCCTCGGAGCCGTGAGCG
CTCACCATCCCCTCTGCGGGGCAACGTGGTCCCAAGCCCACTGCCCACTCGCCGGACGAGGACCTTCTCG
GCGACGGTGCGGGCTTCACAGGGCCCCGTCTACAAAGGAGTCTGCAAATGCTTCTGCCGGTCCAAGGGCC
ATGGCTTCATTACCCCAGCTGATGGCGGCCCCGACATCTTCCTGCACATCTCTGATGTGGAAGGGGAGTA
TGTCCCAGTGGAAGGCGACGAGGTCACCTATAAATGTGCTCCATCCCACCCAAGAATGAGAAGCTGCAG
GCCGTGGAGGTCGTCATCACTCACCTGGCACCAGGCACCAAGCATGAGACCTGGTCTGGACATGTCATCA
GCTCCTAGGAGATGGTGGAAGCACCCCTTGTCCTGTGCTTGTGGGAGACTTTGCAGGGAGGAGGCAGCAG
ACACTGGAGATGACATTCTTCCACGAGACGGGGCTTCAGCCGGGCATGGTCCCTCTCAAGTATCTCCT
GGAGGAAGGGGTATGGGGGGCAGGTGTGGGGTGTGGGGTGTTCCCGGCCATCAGCACAGCCTATGACCAT
TGCAACAACCTCTCACCATCTGAAGAGCATTAAAAGCATTTAAAAAGGAGAGGTGCCCACTGGTGGCTGA
GTGGAGGTTCCAACCCCATCCCAGGGAGTGGATCAAGGGTGGTATTTCTCCAGCTGCTCAGACACATGGG
CTCAACCCACAGAATCCCTCTTCCTCCTGGAGCTGGAGGCCCCAGATTCCCAGATCTGGCCCCCTGGCAG
CCTGACAGGGACCTTGCGTGACTTCTCCAAGGCAAATTTCCACCTAAGTGCCCCTTGCGCCTCTCCTGGG
GCCTGGGCAAAGCAGTTTTCTAATTCTTGGCTTGGTTGGTTCTAGGGGAGCTGGCTTGAAGTGGGTGGGG
AAAGGCGGGGGTGGCGGTCTTTGGATTGGACGGATGTTGCCTTTTGGTGCCTTTGCAGTGGGAGGCGGCA
TAGCTGCCTGTCTGGGGAAGACAGTTCTCCCAGCACTCCCACCCCTGGGCACAGCAGGCTGGTACTGGGA
GGCTGAACCCCTCTTAGAGCCTGACCTTTTCATCTGCCTTCTGGTTGTGTGACCATCACTCAACAGCCAT
TTCACAGCCCCTGTAATTATGGCGGCGGGGGGCTGGGGTGGTGGTGGTGGGAAGGGCTTGTGGAGAGGAC
ACAGTCTTTGTTTAAAAACTTTGTCCCGATCCATCCAGAAAAGAGTAGGTAGCTTGCATCCTGACAGCCT
GGCAAAGTCAAGAAAGTTGAAGGAGAAACATACCTTTGGAGAGGGGGTTTTCTTTAAAACTAGTGTTAAG
AAATGCTTAGGGATTTTTTTTTTCTTATTTTTCATAACTAAAGCTTTCACCCAGAGCCGGCTCTGTTTGC
ACTTTGCTGCCGACATTGCAAACTTTTTGGCAGGGTGGGAGACTGAGTCTCATTCTGTCACCCAGGCTGG
AGTGCAGTGGCCCGATCTCAGCTTACTGCAACCTCTGCCCTCCAGGGTTCTGGCAATTCCGCCTCAGTCT
CCTGAGTAGCTGGGATTACAGGCATGCGCCACCACACCCGGTTAATTTTTGTATTTTTAGTAGAGACCAG
GTTTCATCATGTTGGGCAGGATGGTCTTGAACCCCTGACCTCAGATGATCTGCCCATCTCGGCCTCTCAA
AGTGCTGGGATTACAAGTGTGAGCCATCGCGCCCGGCCTGCAAACTTTTTTGTAGGTATTTCTGGTAAAC
AAATCCTTAGGTTATCTTTGCTGTGGTTGTGGTTTGGCTTTAGTCATGATTTCAAAGTAGAAATAGCTAG
GCATTATTTTTTGAAATATATGACCTATATGTAGTCAAGAATCCACTGAACAGAGGCAAGCAAACCTTTT
GGAAACTGGCTTTTGGGCAGACAGTAAACGTCCAGTTTGATGCTGGAAGCATGAACAGCTTCATCAGGTA
GGTACTCCTCAACTCTGATGAGTTTGTCCTTTCAGCCTAAGGGGGTGGAAGGGAGTTGTTTGAGAATAGC
AAATACGCATGTTGATTGCGAGTGTGTGGAGACAAAGGCAGTTCCCACCACAGTTAGGTCCTGGCCATTG
TTTCCTCGCCTGCGATGCTCCTTGTACATCCTCACCCTCCTCTCCCGCCTCTGCCTTCTGCTGGGTCAAA
GGTGGCCTTTTCTCTCCAGCCTTGAATTGTTCCCTGTTGGCTTCCCAAGGGCCCATCTGCTGGTACAGTC
CACACTTCCACAGCCAAGACCCGAGAGGGCTTTCACTGCCCCAAGCCTCTCTCCTGTGACCCTGGGATTC
TGTCTTGGCAGAATCCTTTGTCAGCGGCTCTTACTCTGTCCTTCCTGTTTGGCCACAGCTCTTTCAATCA
ATGGGTATTCTAGAACCGCAGGATGTCAGAGCTGGAAGGGACGCGATACCGGTTTACACAAGGGGAAACT
CCTCGAGGCTCTGGGAGGGACGGAGGGTTTTGGTGACAGAGCGAGAGCTAAAATTGAGGATTCCTGAATC
CAGATCTTGCCTCCCATCAGCCATCTTTCTCCAATAAATTTTTGTTTTGTGCAAGGCTAAAAAAAAAAA
AAAAAAA
```

>NM_006037.3 Homo sapiens histone deacetylase 4 **(HDAC4)**, mRNA

GGAGGTTGTGGGGCCGCCGCCGCGGAGCACCGTCCCCGCCGCCGCCCGAGCCCGAGCCCGAGCCCGCGCA
CCCGCCCGCGCCGCCGCCGCCGCCGCCCGAACAGCCTCCCAGCCTGGGCCCCCGGCGGCGCCGTGGCCGC
GTCCCGGCTGTCGCCGCCCGAGCCCGAGCCCGCGCGCCGGCGGGTGGCGGCGCAGGCTGAGGAGATGCGG

CGCGGAGCGCCGGAGCAGGGCTAGAGCCGGCCGCCGCCGCCCGCCGCGGTAAGCGCAGCCCCGGCCCGGC
GCCCGCGGGCCATTGTCCGCCGCCCGCCCCGCGCCCCGCGCAGCCTGCAGGCCTTGGAGCCCGCGGCAGG
TGGACGCCGCCGGTCCACACCCGCCCCGCGCGCGGCCGTGGGAGGCGGGGGCCAGCGCTGGCCGCGCGCC
GTGGGACCCGCCGGTCCCCAGGGCCGCCCGGCCCCTTCTGGACCTTTCCACCCGCCGCGAGGCGGCTT
CGCCCGCCGGGGCGGGGGCGCGGGGGTGGGCACGGCAGGCAGCGGCGCCGTCTCCCGGTGCGGGGCCCGC
GCCCCCCGAGCAGGTTCATCTGCAGAAGCCAGCGGACGCCTCTGTTCAACTTGTGGGTTACCTGGCTCAT
GAGACCTTGCCGGCGAGGCTCGGCGCTTGAACGTCTGTGACCCAGCCCTCACCGTCCCGGTACTTGTATG
TGTTGGTGGGAGTTTGGAGCTCGTTGGAGCTATCGTTTCCGTGGAAATTTTGAGCCATTTCGAATCACTT
AAAGGAGTGGACATTGCTAGCAATGAGCTCCCAAAGCCATCCAGATGGACTTTCTGGCCGAGACCAGCCA
GTGGAGCTGCTGAATCCTGCCCGCGTGAACCACATGCCCAGCACGGTGGATGTGGCCACGGCGCTGCCTC
TGCAAGTGGCCCCCTCGGCAGTGCCCATGGACCTGCGCCTGGACCACCAGTTCTCACTGCCTGTGGCAGA
GCCGGCCCTGCGGGAGCAGCAGCTGCAGCAGGAGCTCCTGGCGCTCAAGCAGAAGCAGCAGATCCAGAGG
CAGATCCTCATCGCTGAGTTCCAGAGGCAGCACGAGCAGCTCTCCCGGCAGCACGAGGCGCAGCTCCACG
AGCACATCAAGCAACAACAGGAGATGCTGGCCATGAAGCACCAGCAGGAGCTGCTGGAACACCAGCGGAA
GCTGGAGAGGCACCGCCAGGAGCAGGAGCTGGAGAAGCAGCACCGGGAGCAGAAGCTGCAGCAGCTCAAG
AACAAGGAGAAGGGCAAAGAGAGTGCCGTGGCCAGCACAGAAGTGAAGATGAAGTTACAAGAATTTGTCC
TCAATAAAAAGAAGGCGCTGGCCCACCGGAATCTGAACCACTGCATTTCCAGCGACCCTCGCTACTGGTA
CGGGAAAACGCAGCACAGTTCCCTTGACCAGAGTTCTCCACCCCAGAGCGGAGTGTCGACCTCCTATAAC
CACCCGGTCCTGGGAATGTACGACGCCAAAGATGACTTCCCTCTTAGGAAAACAGCTTCTGAACCGAATC
TGAAATTACGGTCCAGGCTAAAGCAGAAAGTGGCCGAAAGACGGAGCAGCCCCCTGTTACGCAGGAAAGA
CGGGCCAGTGGTCACTGCTCTAAAAAAGCGTCCGTTGGATGTCACAGACTCCGCGTGCAGCAGCGCCCCA
GGCTCCGGACCCAGCTCACCCAACAACAGCTCCGGGAGCGTCAGCGCGGAGAACGGTATCGCGCCCGCCG
TCCCCAGCATCCCGGCGGAGACGAGTTTGGCGCACAGACTTGTGGCACGAGAAGGCTCGGCCGCTCCACT
TCCCCTCTACACATCGCCATCCTTGCCCAACATCACGCTGGGCCTGCCTGCCACCGGCCCCTCTGCGGGC
ACGGCGGGCCAGCAGGACGCCGAGAGACTCACCCTTCCCGCCCTCCAGCAGAGGCTCTCCCTTTTCCCCG
GCACCCACCTCACTCCCTACCTGAGCACCTCGCCCTTGGAGCGGGACGGAGGGGCAGCGCACAGCCCTCT
TCTGCAGCACATGGTCTTACTGGAGCAGCCGCCGGCACAAGCACCCCTCGTCACAGGCCTGGGAGCACTG
CCCCTCCACGCACAGTCCTTGGTTGGTGCAGACCGGGTGTCCCCCTCCATCCACAAGCTGCGGCAGCACC
GCCCACTGGGGCGGACCCAGTCGGCCCCGCTGCCCCAGAACGCCCAGGCTCTGCAGCACCTGGTCATCCA
GCAGCAGCATCAGCAGTTTCTGGAGAAACACAAGCAGCAGTTCCAGCAGCAGCAACTGCAGATGAACAAG
ATCATCCCCAAGCCAAGCGAGCCAGCCCGGCAGCCGGAGAGCCACCGGAGGAGACGGAGGAGGAGCTCC
GTGAGCACCAGGCTCTGCTGGACGAGCCCTACCTGGACCGGCTGCCGGGGCAGAAGGAGGCGCACGCACA
GGCCGGCGTGCAGGTGAAGCAGGAGCCCATTGAGAGCGATGAGGAAGAGGCAGAGCCCCCACGGGAGGTG
GAGCCGGGCCAGCGCCAGCCCAGTGAGCAGGAGCTGCTCTTCAGACAGCAAGCCCTCCTGCTGGAGCAGC
AGCGGATCCACCAGCTGAGGAACTACCAGGCGTCCATGGAGGCCGCCGGCATCCCCGTGTCCTTCGGCGG
CCACAGGCCTCTGTCCCGGGCGCAGTCCTCACCCGCGTCTGCCACCTTCCCCGTGTCTGTGCAGGAGCCC
CCCACCAAGCCGAGGTTCACGACAGGCCTCGTGTATGACACGCTGATGCTGAAGCACCAGTGCACCTGCG
GGAGTAGCAGCAGCCACCCCGAGCACGCCGGGAGGATCCAGAGCATCTGGTCCCGCCTGCAGGAGACGGG
CCTCCGGGGCAAATGCGAGTGCATCCGCGGACGCAAGGCCACCCTGGAGGAGCTACAGACGGTGCACTCG
GAAGCCCACACCCTCCTGTATGGCACGAACCCCCTCAACCGGCAGAAACTGGACAGTAAGAAACTTCTAG
GCTCGCTCGCCTCCGTGTTCGTCCGGCTCCCTTGCGGTGGTGTTGGGGTGGACAGTGACACCATATGGAA
CGAGGTGCACTCGGCGGGGGCAGCCCGCCTGGCTGTGGGCTGCGTGGTAGAGCTGGTCTTCAAGGTGGCC
ACAGGGGAGCTGAAGAATGGCTTTGCTGTGGTCCGCCCCCCTGGACACCATGCGGAGGAGAGCACGCCCA
TGGGCTTTTGCTACTTCAACTCCGTGGCCGTGGCAGCCAAGCTTCTGCAGCAGAGGTTGAGCGTGAGCAA
GATCCTCATCGTGGACTGGGACGTGCACCATGGAAACGGGACCCAGCAGGCTTTCTACAGCGACCCCAGC
GTCCTGTACATGTCCCTCCACCGCTACGACGATGGGAACTTCTTCCCAGGCAGCGGGGCTCCTGATGAGG
TGGGCACAGGGCCCGGCGTGGGTTTCAACGTCAACATGGCTTTCACCGGCGGCCTGGACCCCCCCATGGG
AGACGCTGAGTACTTGGCGGCCTTCAGAACGGTGGTCATGCCGATCGCCAGCGAGTTTGCCCCGGATGTG
GTGCTGGTGTCATCAGGCTTCGATGCCGTGGAGGGCCACCCCACCCCTCTTGGGGGCTACAACCTCTCCG
CCAGATGCTTCGGGTACCTGACGAAGCAGCTGATGGGCCTGGCTGGCGGCCGGATTGTCCTGGCCCTCGA
GGGAGGCCACGACCTGACCGCCATTTGCGACGCCTCGGAAGCATGTGTTTCTGCCTTGCTGGGAAACGAG
CTTGATCCTCTCCCAGAAAAGGTTTTACAGCAAAGACCCAATGCAAACGCTGTCCGTTCCATGGAGAAAG
TCATGGAGATCCACAGCAAGTACTGGCGCTGCCTGCAGCGCACAACCTCCACAGCGGGGCGTTCTCTGAT
CGAGGCTCAGACTTGCGAGAACGAAGAAGCCGAGACGGTCACCGCCATGGCCTCGCTGTCCGTGGGCGTG
AAGCCCGCCGAAAAGAGACCAGATGAGGAGCCCATGGAAGAGGAGCCGCCCCTGTAGCACTCCCTCGAAG
CTGCTGTTCTCTTGTCTGTCTGTCTCTGTCTTGAAGCTCAGCCAAGAAACTTTCCCGTGTCACGCCTGCG
TCCCACCGTGGGGCTCTCTTGGAGCACCCAGGGACACCCAGCGTGCAACAGCCACGGGAAGCCTTTCTGC
CGCCCAGGCCCACAGGTCTCGAGACGCACATGCACGCCTGGGCGTGGCAGCCTCACAGGGAACACGGGAC
AGACGCCGGCGACGCGCAGACACACGGACACGCGGAAGCCAAGCACACTCTGGCGGGTCCCGCAAGGGAC
GCCGTGGAAGAAAGGAGCCTGTGGCAACAGGCGGCCGAGCTGCCGAATTCAGTTGACACGAGGCACAGAA

AACAAATATCAAAGATCTAATAATACAAAACAAACTTGATTAAAACTGGTGCTTAAAGTTTATTACCCAC
AACTCCACAGTCTCTGTGTAAACCACTCGACTCATCTTGTAGCTTATTTTTTTTTAAAGAGGACGTTTTC
TACGGCTGTGGCCCGCCTCTGTGAACCATAGCGGTGTGCGGCGGGGGGTCTGCACCCGGGTGGGGGACAG
AGGGACCTTTAAAGAAAACAAAACTGGACAGAAACAGGAATGTGAGCTGGGGGAGCTGGCTTGAGTTTCT
CAAAAGCCATCGGAAGATGCGAGTTTGTGCCTTTTTTTTTATTGCTCTGGTGGATTTTTGTGGCTGGGTT
TTCTGAAGTCTGAGGAACAATGCCTTAAGAAAAAACAAACAGCAGGAATCGGTGGGACAGTTTCCTGTGG
CCAGCCGAGCCTGGCAGTGCTGGCACCGCGAGCTGGCCTGACGCCTCAAGCACGGGCACCAGCCGTCATC
TCCGGGGCCAGGGGCTGCAGCCCGGCGGTCCCTGTTTTGCTTTATTGCTGTTTAAGAAAAATGGAGGTAG
TTCCAAAAAAGTGGCAAATCCCGTTGGAGGTTTTGAAGTCCAACAAATTTTAAACGAATCCAAAGTGTTC
TCACACGTCACATACGATTGAGCATCTCCATCTGGTCGTGAAGCATGTGGTAGGCACACTTGCAGTGTTA
CGATCGGAATGCTTTTTATTAAAAGCAAGTAGCATGAAGTATTGCTTAAATTTTAGGTATAAATAAATAT
ATATATGTATAATATATATTCCAATGTATTCCAAGCTAAGAAACTTACTTGATTCTTATGAAATCTTGAT
AAAATATTTATAATGCATTTATAGAAAAAGTATATATATATATAAAATGAATGCAGATTGCGAAGGTC
CCTGCAAATGGATGGCTTGTGAATTTGCTCTCAAGGTGCTTATGGAAAGGGATCCTGATTGATTGAAATT
CATGTTTTCTCAAGCTCCAGATTGGCTAGATTTCAGATCGCCAACACATTCGCCACTGGGCAACTACCCT
ACAAGTTTGTACTTTCATTTTAATTATTTTCTAACAGAACCGCTCCCGTCTCCAAGCCTTCATGCACATA
TGTACCTAATGAGTTTTTATAGCAAAGAATATAAATTTGCTGTTGATTTTTGTATGAATTTTTTCACAAA
AAGATCCTGAATAAGCATTGTTTTATGAATTTTACATTTTTCCTCACCATTTAGCAATTTTCTGAATGGT
AATAATGTCTAAATCTTTTTCCTTTCTGAATTCTTGCTTGTACATTTTTTTTTACCTTTCAAAGGTTTTT
AATTATTTTTGTTTTTATTTTTGTACGATGAGTTTTCTGCAGCGTACAGAATTGTTGCTGTCAGATTCTA
TTTTCAGAAAGTGAGAGGAGGGACCGTAGGTCTTTTCGGAGTGACACCAACGATTGTGTCTTTCCTGGTC
TGTCCTAGGAGCTGTATAAAGAAGCCCAGGGGCTCTTTTTAACTTTCAACACTAGTAGTATTACGAGGGG
TGGTGTGTTTTTCCCCTCCGTGGCAAGGGCAGGGAGGGTTGCTTAGGATGCCCGGCCACCCTGGGAGGCT
TGCCAGATGCCGGGGGCAGTCAGCATTAATGAAACTCATGTTTAAACTTCTCTGACCACATCGTCAGGAT
AGAATTCTAACTTGAGTTTTCCAAAGACCTTTTGAGCATGTCAGCAATGCATGGGGCACACGTGGGGCTC
TTTACCCACTTGGGTTTTTCCACTGCAGCCACGTGGCCAGCCCTGGATTTTGGAGCCTGTGGCTGCAAGG
AACCCAGGGACCCTTGTTGCCTGGTGAACCTGCAGGGAGGGTATGATTGCCTGACCAGGACAGCCAGTCT
TTACTCTTTTTCTCTTCAACAGTAACTGACAGTCACGTTTTACTGGTAACTTATTTTCCAGCACATGAAG
CCACCAGTTTCATTCCAAAGTGTATATTGGGTTCAGACTTGGGGGCAGAAGTTCAGACACACCGTGCTCA
GGAGGGACCCAGAGCCGAGTTTCGGAGTTTGGTAAAGTTTACAGGGTAGCTTCTGAAATTAACTCAAACT
TTTGACCAAATGAGTGCAGATTCTTGGATTCACTTGGTCACTGGGCTGCTGATGGTCAGCTCTGAGACAG
TGGTTTGAGAGCAGGCAGAACGGTCTTGGGACTTGTTTGACTTTCCCCTCCCTGGTGGCCACTCTTTGCT
CTGAAGCCCAGATTGGCAAGAGGAGCTGGTCCATTCCCCATTCATGGCACAGAGCAGTGGCAGGGCCCAG
CTAGCAGGCTCTTCTGGCCTCCTTGGCCTCATTCTCTGCATAGCCCTCTGGGGATCCTGCCACCTGCCCT
CTTACCCCGCCGTGGCTTATGGGGAGGAATGCATCATCTCACTTTTTTTTTTTAAGCAGATGATGGGATA
ACATGGACTGCTCAGTGGCCAGGTTATCAGTGGGGGGACTTAATTCTAATCTCATTCAAATGGAGACGCC
CTCTGCAAAGGCCTGGCAGGGGGAGGCACGTTTCATCTGTCAGCTCACTCCAGCTTCACAAATGTGCTGA
GAGCATTACTGTGTAGCCTTTTCTTTGAAGACACACTCGGCTCTTCTCCACAGCAAGCGTCCAGGGCAGA
TGGCAGAGGATCTGCCTCGGCGTCTGCAGGCGGGACCACGTCAGGGAGGGTTCCTTCATGTGTTCTCCCT
GTGGGTCCTTGGACCTTTAGCCTTTTTCTTCCTTTGCAAAGGCCTTGGGGGCACTGGCTGGGAGTCAGCA
AGCGAGCACTTTATATCCCTTTGAGGGAAACCCTGATGACGCCACTGGGCCTCTTGGCGTCTGCCCTGCC
CTCGCGGCTTCCCGCCGTGCCGCAGCGTGCCCACGTGCCCACGCCCCACCAGCAGGCGGCTGTCCCGGAG
GCCGTGGCCCGCTGGGACTGGCCGCCCCTCCCCAGCGTCCCAGGGCTCTGGTTCTGGAGGGCCACTTTGT
CAAGGTGTTTCAGTTTTTCTTTACTTCTTTTGAAAATCTGTTTGCAAGGGGAAGGACCATTTCGTAATGG
TCTGACACAAAAGCAAGTTTGATTTTTGCAGCACTAGCAATGGACTTTGTTGTTTTTCTTTTTGATCAGA
ACATTCCTTCTTTACTGGTCACAGCCACGTGCTCATTCCATTCTTCTTTTTGTAGACTTTGGGCCCACGT
GTTTTATGGGCATTGATACATATATAAATATATAGATATAAATATATATGAATATATTTTTTTAAGTTTC
CTACACCTGGAGGTTGCATGGACTGTACGACCGGCATGACTTTATATTGTATACAGATTTTGCACGCCAA
ACTCGGCAGCTTTGGGGAAGAAGAAAAATGCCTTTCTGTTCCCCTCTCATGACATTTGCAGATACAAAAG
ATGGAAATTTTTCTGTAAAACAAACCTTGAAGGAGAGGAGGGCGGGGAAGTTTGCGTCTTATTGAACTT
ATTCTTAAGAAATTGTACTTTTTATTGTAAGAAAAATAAAAAGGACTACTTAAACATTTGTCATATTAAG
AAAAAAAGTTTATCTAGCACTTGTGACATACCAATAATAGAGTTTATTGTATTTATGTGGAAACAGTGTT
TTAGGGAAACTACTCAGAATTCACAGTGAACTGCCTGTCTCTCGAGTTGATTTGGAGGAATTTTGTTTT
TGTTTTGTTTTGTTTGTTTCCTTTTATCTCCTTCCACGGGCCAGGCGAGCGCCGCCCGCCCTCACTGGCC
TTGTGACGGTTTATTCTGATTGAGAACTGGGCGGACTCGAAAGAGTCCCCTTTTTCCGCACAGCTGTGTTG
ACTTTTTAATTACTTTTAGGTGATGTATGGCTAAGATTTCACTTTAAGCAGTCGTGAACTGTGCGAGCAC
TGTGGTTTACAATTATACTTTGCATCGAAAGGAAACCATTTCTTCATTGTAACGAAGCTGAGCGTGTTCT
TAGCTCGGCCTCACTTTGTCTCTGGCATTGATTAAAAGTCTGCTATTGAAAGAAAAAGAAAGCGAACAGT
TTTTGTTTGTTTTTTTTGCCGTGTGTGCTCCATAGTGGAGGCGCTATTTTCCAATTGATGAGAATGACAA
ACATATATAATCTATCTATCTATCTATCTATCTATCTATCTATACAGGGGGCTTGAACCTTACTCACCCA

```
AGAGCTTCTTACGGAATGTGGTAGAAAACCAAGTTGTAACGACACTGTAACCTACCTGATGCCTGTTCGC
GCCCGCCGTGAGCTGCGCACTGGCCGTGGCCACCATTCACCTCTGTAATTTAATCCGTTTCTCTTGGATT
GTCTGGACGTGCCCGATGGTTCTTTCTTTTGCTCAGTGGAGTTGGAGGTTTTGTGTTTGGTTTTCTCATT
CTTGTCTTGTTTTGTGTGGGTGGATTTTCACCGACCAATGATATCCTCTTCTGACGGTCACCTTCTTTCC
ACTTCACTGGAGTCCAGTATTCTGTACCACATCACGCAACGTGTTATCTTGTGGTGTAAATAAAGACTGC
GTTACTTGCCCTCCCAAAAA
```

>NM_001200049.2 Homo sapiens cilia and flagella associated protein 46 **(CFAP46)**, mRNA

```
CAACCGTGGCCGGGTCCTCGCGGCTGGAGAACCCAACCGACAGTGGGCGGCAGGACGCACCGCGGACCCC
GGAGAGAGCGGACGAGCAGGGCGCCGGCGCCATGGACCTGGTCATCACGCAGGAGCTGGCCCGCGCCGAG
AGCCAGCAAGATGCTGCGTCCTTGAAGAAGGCCTACGAGTTGATCAAATCGGCCAACCTAGGGAAATCGG
AGTTTGACCCCTCAGAGAGCTTCAGCCCAGACCTGTTTGTTCTGTGTGCAGAGCAGGCCCTGAAGATGAG
GCAGCCAGAGGTGAGCGAGGACTGCATCCAAATGTACTTCAAGGTGAAGGCGCCCATCACCCAGTTTCTG
GGCCGAGCGCACCTGTGCAGGGCCCAGATGTGTGCCCCGAAGTCGGCAGAAAACCTGGAGGAATTTGAAA
ATTGCGTGACTGAGTACATGAAGGCCATAAACTTTGCCAAAGGAGAACCGAGGTACTACTTTTTGGTGTA
CAATGCATCAGTCCTCTACTGGCAGATGGTGAGGCCGTTCCTCAAGCCTGGATATCGTCACCATCTGATC
CCCAGCCTTTCCCAAATCATAAACGTGCTGAGTCAGACTGAGGAGGAAGACAAGGAGTGGCGTGCTGAGC
TGATGCTGGAACTTCTGGAGTGTTATCTGCAAGCCGGAAGAAAGGAGGAGGCTGCCAGGTTCTGCTCCAC
GGCAGCTCCGTTCATTAAGTCTCACGTGCCACAGAAATACCGGCAGATATTCTCTGTTATGGTTCGTCAT
GAATTAATGGACGAACTTCAGTTAAAGGAAGAAAGAAAAATTCCATTAGCCTGTCAGTCACTTTCTATA
TTAATATGCTAAAGGCAAAAGCGGAGCAAAATGATTTACCAGGTGACATCAGTGTCATTCTGAGGAAGGC
CTACAGACACTTAGGTCATTACAACCACCAGCGCTTTCCCTCTATCAGTGAAGAAAAAATGCTTTTGCTT
TTTGAATTGGCGCGTTTTTCCTTGACCTTGAAATGCATGGAGATCTCCTCTGCCTGCCTCTCAGACCTGA
AGAAGATGGAAAGCAAAGATCCTGGGAAGCTTATTGAAATGGAATGTCTGGAGTGTGAATCGGAAGCTTT
AAGACTTGAAAGTAAGATGAAAGTGTACAACCGAGCGGCTGTTGAGGCCCAGCTGGATATCATACAGAGG
CTAGACGTCGCGCTGCAGCGAGCCGTGCGCCTGGGCGACCCCGGGTCATCCACGTGGTGTGCGCCACGC
AGTGGAACACCTGCCTGCCCCTGCTGCAGCACAACCTGCGGCACCACCTGCGGAAGCCCCTGGCTGGCGT
TGCGGACGTGCTGGAGAAGCTGGACAGCCTCATGACGCTTCTCCGCTGTCAAGTGCACATGGAGATGGCG
CAGATCGAGGAGGACGAGGACCGGCTGGAGCCCGCCACGGAGCACCTCCGGAAAGCCGCGCGCCTGGACA
GCCTGGGCCTCTACCGGGACAGGATCCAGATGGCCTCCACCCGGCTGCGTCTGTGCACCACGCTATACCA
GGCCCCTGAGCGCGCAGAGGACAAGGCCATCATGGCCGTTGAGCAGGCAAAAAAAGCTACACCAAAGGAC
AGCGTCAGGAAGAAGCGGGCCCTCCTGGTGAATGCAGGCCTGGCCTTAGCCCCTGACGCGTTTCAGATTG
TGCTGGACAGTGAGAATGAGGCCAAAGTCTCCACCGGGAAGAACAGGGGCCGGTTCACCTACCTCTGTGC
GAAGGCGTGGCACCACACCGTCAGCGTGGACAAAGCTGCCGGGCACCTGCGGCGCCTGGGCAACGAAAAC
GACAAGGAGAGGATACAGATTTGGGCAGAGCTGGCCAAAGTGGCCCGGAAACAAGGCGTGTGGGACGTCT
GTCGGACGGCGAGCCGCTTCTGCCTCCTGTATGACAACGTCAAGGTGAAGAAGTTGAGGCTGCGGCGAGG
GAAGAAGAAGCGAGGTAGGGACGGCTCGGTGCAGGACACCTGGAGCCAGCCTGAGGTCGTCCTGCAGAGG
CAGGTGTGCCCCGACCTGCTGCGGAAGTTCGCGGAGGTGGGGTTCATCCATGCTGAGGCCACGGTTCATT
TGCTGCGGTCAGAAGGTGTAGAGCTGAATGACCGGGCCATCCCCCCCGAAGACCTGAGCCAGCACCCAGC
TGGCTACGTGCCTGAGCCCCCGGAGGTGAATGCTGAGTGGATCACATACAGAACCTGGATCGAGAGTCTG
TCCCGGTGTGCCATGAATAACTGGCTGCGCTCCGCAGAGATCGGACAGGAGATCCAGGAGGCGTGGATTG
TGCAGAACGCCGTGGTCTACGTCCTGAACCACAACCACCACCTGATCCTGGCCGGGCGGCAGAAGGAGCT
GGTGGACGCCCTGTACCACCTCCTGAGCATCGTTAAGGCCACAGGCCACAGTGGGGACCCCGTGATGCTG
GTGACGCTCTGCAACACCTTGGCGCGAGGCCTGATCATCAGCTGGATTCCAGTCCAGGCTGCCGAGAAGT
CCAGGAAATTCATGCGACCAAACGCGTTTCACAGCCCACTGGACGCAGGAGCCACTTCCGAGATCAAAAC
AGCGGTGGAGGTCTGCGAGTTTGCCCTGAACCTGACCAATGGGAGTGCGCCCGAGGAGACGGTGCCCACC
GGCACCCGGCAGCAGCTTATCGCCACCTGGGTCAAGGCCAAGCAGCTGCTGCAGCAGCAGATTGGGCCAC
GGCTGGGCACCGAGGAGCAGGGCACCAATGAGGATGTCAGCTCGGTGACCAGAGTCCTCGTTGCCTTGGA
AATGTACTCATGCAACGGGCTGGGCCTCATGGACTTCACTGTCCCCTCCCTGGCCCAGTTGGTGAAAATG
GCTTCCGAGTGCAACTGGTCGGACCCCCTGGTGGAGCTGCAGACCCTGACGCGGCTGACCCACTTCGCCC
ATGCAGCGCGTGACCATGAGACCACCATGGCCTGTGCTCACAGGGCTCTGGAGATGGGCATCAAGTACCT
GAAGAAATTTGGGCCCGAGGAGTCCCGGCTGGTGGCAGAGATGCTGTGCACAGCCACGGCCATCCAGGGC
AGGAGCATCATGGAAAACCTGAAGGGCCGGAAGCAGCTGCGACTGGTGGCAGCCAAGGCCTTCACGGAGA
GCGCCAGGTTCGGAGGCATCGCGGGCAGCAGCGCCCTGGTGATGCTGGCCGCGCGGCATTACTGGAACGC
CTGGCTCCCACTGCTGTCCTCAGCCGTCTACAGGAAGAAGGCCAAGGGTGCCCTGAAGAGGCTCATCGGC
ATCATCAACAAGACAGAGGCCAGAAAGCAGGAGAAAGGAAAGACGCTGCTTCTGCACCAGTGGCCCACGG
CCGACTTCCAGGGTGGCGGGACGACCGAAGGATATTTTCTTCCAGGGGCTGAGGACGACCTGGCGCTCCG
TGCTGCGCTCTACGGCCTGCTCTTCCACAGCCATGCCGACCAGGACGACTGGGAGGGCGGCCTCAAGGTG
CTGGACGAGGCTGTGCAGGTGCTGCCAAGGACGGCCCACCGCCTCTTGATCTTCAAGCACATGGTCATCG
```

```
TGAAGGCCAAGCTCGGGCAGAATTTTTCGATGGAAATACAGAAATTCAAGGCCGAGAGTGAGGACTACTT
GGCGCGCATGTGGCACCGCCTGGCCCTGAACTCGCCGAGCGTGTCTGGAGAGCTGGCCTGCTACAACAAC
GCCATCCAGGCCTTGCAGAAGCCTGAGATGGAGTGGCAGAAGGTGGAGTACCTCATGGAGTTCGGCCAGT
GGCTCCATCACAGACACTTTCCTCTCGAGGACGTGGTCTTCCACCTCCGCTGGGCTGTCGAGATCCTGCT
GGCCATGAAGCCGCCCGGCGATGTCCCTGAGCCACAGCCCACGCCGGATGGGGAGTACGTGGCTGTGGAG
ATGCCCCCACGGAGCCCCGTGTCCGAGGCCGAGGAGGCGGTGTCCTTGGAGCAGCTGCGTAGCGTGCGGC
AGCTGGAGGCGCTGGCCCGCGTGCACATCCTGCTGGCCCTGGTGCTGTCGCCGGGCGCCGAGGGCTACGA
GGACTGCTGCCTTGCAGCCTACGCCTTCTTCAGGCACATCTGGCAGGTTTCTTTGATGACAGCAGGAAAA
TCAGTTCTGGAAAACAGACCCCTGGCAGCAACCAGCTCACATCTGTTATTGCCTAAAAAAGAGAAGGAGA
ATGAGAGGAGTAAAGAGAAGGAGAAGGAGAGGAGTAAAGAGAAGGAGAATGAGAGGAGTAAAGAGAAGGA
CAAGGAGAAGGGAAAGGAGGAGAAAGTCAAGGAGCCCAAGCAGTCTCAAAGCCCAGCTCCTATCAAACAA
CTGGAAGACTTACCCATGAGCATAGAAGAGTGGGCTTCCTACTCCTGCCCCGAGGAAGTGCTGTCTGTAC
TGAAACAGGACAGAAGTGACTCTACTGTGAACCCCTCAAGTATCCAGAAGCCGACATACAGTTTGTATTT
CCTGGACCACCTGGTCAAGGCCCTGCAGAAGATGTGCCTGCACGAACTCACGGTTCCCGTCCTGCAGTTG
GGGGTGCTGATTTCGGACTCCGTGGTGGGAAGCAAGGGCCTGTCGGATCTCTACCACCTTCGCCTCGCCC
ACGCGTGCTCCGAGCTGAAGCTGAGAGAAGCAGCCGCGCGCCATGAAGAGGCGGTCGGGCAGGTGTGCGT
CAGCGAGCTGGAGCAGGCCAGCTGCAGAAAAGAGATCGCGTTGAAAAAAGAGAAAAATAAGGAGCCTTTA
TTAGAAGAAAGCCTGCCAGCACTGAATGAGCAGACACTTCCTGTCCAGCCTGGGGAGATCAAACCACTGG
ACGCCAAGGACAAGATTTTGAAGATGAATGGGGAGACCGGGAGGGACCTGGATGGGACGTCCTTTCCCCA
CCTGTGGATGCTGAAGGCAGAAGTTCTGCTGGAGATGAACCTGTACCAGCCTGCACGGCTGCTCCTGTCG
GAGGCTTACCTGGCTTTCCAGGAGCTGGATGAGCCTTGTGCAGAGGCCCAGTGCCTGCTCCTCCTCGCAC
AGTTGGCCAACAAGGAGAAAAACTATGGACAAGCCAAGAAAATGATCGCACAGGCCCAGCACCTGGGCGG
AAGTGAGGAGTTCTGGTACAATTCCACTCTGACCCTGGCAGAGGCGCTCTTGTCCATGGAACACTCAGGA
AGGGAAGCTACGGTGTGTCACATATTTCAGAAGCTCATCAATGCCTTCAAGATCCTCAAGAAAGAAAGAC
CAAACCGATTGCCTTTACTGGAATTCATGATCACAGATCTAGAAGCCAGGTGCCTGAGCCTGCGGGTCAG
AGTTGCGCAGCACTCAGCGGTCACTGAACCCACAGAGTGCTCGTTGCTACTGAAAGAGATGGATGATGGC
CTGTTGGAAATTGAGAGAAAGTTTATCGACTGTGGCTGCAAAGAGAATTGTGTTGACGTAAAACTAGAGC
GTGCGAAGATCAAGAGGTTACGTGCCCAGAACGAGAAAGATGAAGAACAAAAAACTGCGTATTACTTGGA
AGCGTATGGCCTGGCCCAGGGTGCCGTAGCTGAGGAAGAAGGGAGGCTTCACAGCATCCAGGGCTTATAT
GGCCTGGCCCAGGGCGCCATGGCTGAGGAAGAAGGGAGGCTTCACAGCGTCCAGGGCCTATTGTCACTTC
AAGACTTGCAGAACGTCAACACGCCCCTGATGAGGAAGCTGGCGCGCCTCAAGCTCGGCCTCGTGGAAAT
GGCTCTGGACATGCTCCAGTTCATCTGGGAGGAGGCCCACGGGCAGCAGAGTGAGCAGGGGTCCCTGGAG
AAGCTGCTGGCGGACTATCTGCAGAACACCAGTGACTACACTTCCGTCGGCCTGCAATGGTTCACGCTGA
AGCGGACTCTAGCACACGGGGCACTGGCACAGCTGGGGAGCCTGCAGCCGCTGAGCGTGGGCTGTGTGGA
GATCCGCGCCCGGCTCCTGGGCCTGGCCGGGAGGGCCCTGCACCTGCTGGCCATGCAAGCTGACCCTGTG
CACCCTACCTGCTACTGGGAGGCGGGCCCCTCGGTGGGCGCCAAGCTGAGCGGCCTCAAGTCTCTGGAGC
TGGAGGTAGAGGAAGAGGGTGCCACAAAGTCCAGCAGGGACCCGCCGGCCTCCAGGGCAGCCCCGGAGGA
GCACTGCAGGAGAGGCGAGGACCTGAAGAGGAGGATGGTTCTGGCCCAGCAGTACCTGGCTCAGGCGTCA
GAGGTGCTGCTGCAGTGCCTACAGGTGGCCCTTGGCAGTGGCCTCCTGGATGTCGCAGCAGCCGCCAGCC
TGGAGATGGTGGAGTGTGTCGGCACCCTGGACCCTGCAACTACCTGCCAGTTCCTGGCTCTGTCTCAGAG
CTGCTCGGCCTCAGAGACGATGAGGGATGTCCTGCTTGCAGCCACAGCCAACACCAGCAGCTCACAGCTG
GCGGCCCTGCTGCAGCTACAGCACCAGCTCCGGTGCCAAGACAGGACCACCACCAGCCTGGGCGCCCGTG
TGGAGCAGAGGCTGGCCGCCGTGTCCAAGGCTTGGCAAAATCTCTGCGTCACTGAGCAGCATTTTAACCT
CTTGAATGAGATGCCTCCGACCTTTTGGATCCTCTTTCTGCACCTCTCAGGGGACAGGTCCCGTCTGTAC
GGCGCTGCCTACGAGAAACCCAAGTTCATTACTGCAGCCAAAGGAAAGGTGCAGGCGGTGGGAGGCTCCT
GCAAGGTGATGCGTCTGGCCATAAGTCCCACTGCCTTCTCCCACCTGCTGGCCTGTGCCCAGCAGTTCCG
GAAGCAGACCCAGGCCCAGGTGTACAGTGAGGACATGGCCCTGAACATAGGCTCGGAACCAGAAGGCCTG
CAGGTGGAAGAGAAGGAGCGCCCTGTGCAGAGGCTCAGTAGCGTCCTGGGGCCCCTGGAGGAGCTTCTGC
AGCCGCTATTCCCCCTGCTCAGCCTCTCCAAGGCCAGAGTGCAGACACCTGCGGTTGTTGCCGATTCAGG
GAAGTCGAAGGGCAAAGACAAGGAGAGGAAAACGTCCACAGGACAACACAGCACAGTCCAGCCTGAGGTT
GCCGATAAGATAGTCCTGGTCGCAGACAGACATCTCCTGGAGCTGCCACTGGAAGGTCTCTCTGTGTTCG
ATGAAGGGACAATTTCCTCTGTGTCACGAGAATTTTCTCTTCAAATGCTGTGGAATCGCCTCCATAAAGA
AGAGACAGAAGGTGGCGTGAAAAAGGAGGGAAGAAGCAGAGACCCCAAAAAGAGAAGCCTAGCGAAGAAG
GGCAGGAAGGGCAGCATCCCCCGGACCATCCCCCCTGACTGCATCATAGTCGACTCAGACAACTTCAAGT
TCGTCGTGGACCCATACGAGGAGCCCAGGGCCCAGAAATGCTAACTCCTGTCTCCATCACCCAAGACAT
TTTGGAAAGATTCCAAGACACATTCACGTCGCGATGGGCGGGACATCTGGGAAGCAAGCACTTTCCCAGC
CAGGCCCAGTGGGAGCAGGCCCTGGGCAGCTGCAGCGGTTTCTTCTTCTATGGAATGGAGAGCTTCCTGT
CCCATATATTAGTGGAGAGATTGGTCGCCATGAACTTGCAAGAGTGCCAGGTGGCAGTCCTGCTGGACCT
GGCGCGGTCCTACCAGAGCTTGAAGAGGCACATGGAGAGCGTGGAGCACAGGAGATCTGTTGGCCGTTGG
GAAGCCAATTGGAGAAACAGTGCGTCTCCTTCAGAAGATGAGTGGCGACGAGGCGGTGAACCAAGACGAG
```

```
GCTTCTCAGACCTTGAAGGACAAGCTGCTGCTGCTCCAAAGCTCCGAGCTCCTTCCCACCACGCTCAACT
TGGTCCTGTATGGGCTGCCGCACCAAGCCATCGGGTAGTGCAGGCCTGGACCTGCCTCCCATCAGCTGCT
GGGGCCCCAGCACTTGCCTCTGCCCTTGGCTCTGCCCCTCTGCCAACCCATCCCCACCTCCCGGCTCCCA
TCCCCAGCTCCCAGCTCGCTCTCCCCTTCCTGGGCCTCTCCCCAGCCCTTGGTGCAGCCTCAGCCAGGGA
CCCTCCCCCAGCGACTTCCCGCAAGGCAGCCGCCTGGACCTCGAGCTCTGCCTGCCTGTGTGCGCCATGG
GGTCTGCGTCGGGGCTGGAGCTGCGTCTCTTCCCGGGGCCAGGACAAGGGCGGCCTCCCCTTGGCGGCGC
TGGTGCTGAGTTGCTTAGACCAGAAGACTATTCAGACCGTGAGCCTGTTTTTGATTTGAGTGTTCCACTA
AACAAACAACAAAGCCAAAAAAAAAAAAAAAA
```

>NM_198433.2 Homo sapiens aurora kinase A (AURKA), transcript variant 1, mRNA

```
CTTAAACGCGACTCAAGGCGTCGGGTTTGTTGTCAACCAATCACAAGGCAGCCTCGCTCGAGCGCAGGCC
AATCGGCTTTCTAGCTAGAGGGTTTAACTCCTATTTAAAAAGAAGAACCTTTGAATTCTAACGGCTGAGC
TCTTGGAAGACTTGGGTCCTTGGGTCGCAGGTGGGAGCCGACGGGTGGGTAGACCGTGGGGGATATCTCA
GTGGCGGACGAGGACGGCGGGGACAAGGGGCGGCTGGTCGGAGTGGCGGAGCGTCAAGTCCCCTGTCGGT
TCCTCCGTCCCTGAGTGTCCTTGGCGCTGCCTTGTGCCCGCCCAGCGCCTTTGCATCCGCTCCTGGGCAC
CGAGGCGCCCTGTAGGATACTGCTTGTTACTTATTACAGCTAGAGGGTCTCACTCCATTGCCCAGGCCAG
AGTGCGGGGATATTTGATAAGAAACTTCAGTGAAGGCCGGGCGCGGTGGCTCATGCCCGTAATCCCAGCA
TTTTCGGAGGCCGAGGCTGGAGTGCAATGGTGTGATCTCAGCTCACTGCAACCTCTGCTTCCTGGGTTTA
AGTGATTCTCCTGCCTCAGCCTCCCGAGTAGCTGGGATTACAGGCATCATGGACCGATCTAAAGAAAACT
GCATTTCAGGACCTGTTAAGGCTACAGCTCCAGTTGGAGGTCCAAAACGTGTTCTCGTGACTCAGCAATT
TCCTTGTCAGAATCCATTACCTGTAAATAGTGGCCAGGCTCAGCGGGTCTTGTGTCCTTCAAATTCTTCC
CAGCGCATTCCTTTGCAAGCACAAAAGCTTGTCTCCAGTCACAAGCCGGTTCAGAATCAGAAGCAGAAGC
AATTGCAGGCAACCAGTGTACCTCATCCTGTCTCCAGGCCACTGAATAACACCCAAAAGAGCAAGCAGCC
CCTGCCATCGGCACCTGAAAATAATCCTGAGGAGGAACTGGCATCAAAACAGAAAAATGAAGAATCAAAA
AAGAGGCAGTGGGCTTTGGAAGACTTTGAAATTGGTCGCCCTCTGGGTAAAGGAAAGTTTGGTAATGTTT
ATTTGGCAAGAGAAAAGCAAAGCAAGTTTATTCTGGCTCTTAAAGTGTTATTTAAAGCTCAGCTGGAGAA
AGCCGGAGTGGAGCATCAGCTCAGAAGAGAAGTAGAAATACAGTCCCACCTTCGGCATCCTAATATTCTT
AGACTGTATGGTTATTTCCATGATGCTACCAGAGTCTACCTAATTCTGGAATATGCACCACTTGGAACAG
TTTATAGAGAACTTCAGAAACTTTCAAAGTTTGATGAGCAGAGAACTGCTACTTATATAACAGAATTGGC
AAATGCCCTGTCTTACTGTCATTCGAAGAGAGTTATTCATAGAGACATTAAGCCAGAGAACTTACTTCTT
GGATCAGCTGGAGAGCTTAAAATTGCAGATTTTGGGTGGTCAGTACATGCTCCATCTTCCAGGAGGACCA
CTCTCTGTGGCACCCTGGACTACCTGCCCCCTGAAATGATTGAAGGTCGGATGCATGATGAGAAGGTGGA
TCTCTGGAGCCTTGGAGTTCTTTGCTATGAATTTTTAGTTGGGAAGCCTCCTTTTGAGGCAAACACATAC
CAAGAGACCTACAAAAGAATATCACGGGTTGAATTCACATTCCCTGACTTTGTAACAGAGGGAGCCAGGG
ACCTCATTTCAAGACTGTTGAAGCATAATCCCAGCCAGAGGCCAATGCTCAGAGAAGTACTTGAACACCC
CTGGATCACAGCAAATTCATCAAAACCATCAAATTGCCAAAACAAAGAATCAGCTAGCAAACAGTCTTAG
GAATCGTGCAGGGGGAGAAATCCTTGAGCCAGGGCTGCCATATAACCTGACAGGAACATGCTACTGAAGT
TTATTTTACCATTGACTGCTGCCCTCAATCTAGAACGCTACACAAGAAATATTTGTTTTACTCAGCAGGT
GTGCCTTAACCTCCCTATTCAGAAAGCTCCACATCAATAAACATGACACTCTGAAGTGAAAGTAGCCACG
AGAATTGTGCTACTTATACTGGTTCATAATCTGGAGGCAAGGTTCGACTGCAGCCGCCCCGTCAGCCTGT
GCTAGGCATGGTGTCTTCACAGGAGGCAAATCCAGAGCCTGGCTGTGGGGAAAGTGACCACTCTGCCCTG
ACCCCGATCAGTTAAGGAGCTGTGCAATAACCTTCCTAGTACCTGAGTGAGTGTGTAACTTATTGGGTTG
GCGAAGCCTGGTAAAGCTGTTGGAATGAGTATGTGATTCTTTTTAAGTATGAAAATAAAGATATATGTAC
AGACTTGTATTTTTTCTCTGGTGGCATTCCTTTAGGAATGCTGTGTGTCTGTCCGGCACCCCGGTAGGCC
TGATTGGGTTTCTAGTCCTCCTTAACCACTTATCTCCCATGAGAGTGTGAAAAATAGGAACACGTGCT
CTACCTCCATTTAGGGATTTGCTTGGGATACAGAAGAGGCCATGTGTCTCAGAGCTGTTAAGGGCTTATT
TTTTTAAAACATTGGAGTCATAGCATGTGTGTAAACTTTAAATATGCAAATAAATAAGTATCTATGTCTA
AAAAAAAAAAAAAA
```

>NM_004048.2 Homo sapiens beta-2-microglobulin **(B2M)**, mRNA

```
AATATAAGTGGAGGCGTCGCGCTGGCGGGCATTCCTGAAGCTGACAGCATTCGGGCCGAGATGTCTCGCT
CCGTGGCCTTAGCTGTGCTCGCGCTACTCTCTCTTTCTGGCCTGGAGGCTATCCAGCGTACTCCAAAGAT
TCAGGTTTACTCACGTCATCCAGCAGAGAATGGAAAGTCAAATTTCCTGAATTGCTATGTGTCTGGGTTT
CATCCATCCGACATTGAAGTTGACTTACTGAAGAATGGAGAGAGAATTGAAAAAGTGGAGCATTCAGACT
TGTCTTTCAGCAAGGACTGGTCTTTCTATCTCTTGTACTACACTGAATTCACCCCCACTGAAAAAGATGA
GTATGCCTGCCGTGTGAACCATGTGACTTTGTCACAGCCCAAGATAGTTAAGTGGGATCGAGACATGTAA
GCAGCATCATGGAGGTTTGAAGATGCCGCATTTGGATTGGATGAATTCCAAATTCTGCTTGCTTGCTTTT
TAATATTGATATGCTTATACACTTACACTTTATGCACAAATGTAGGGTTATAATAATGTTAACATGGAC

ATGATCTTCTTTATAATTCTACTTTGAGTGCTGTCTCCATGTTTGATGTATCTGAGCAGGTTGCTCCACA
GGTAGCTCTAGGAGGGCTGGCAACTTAGAGGTGGGGAGCAGAGAATTCTCTTATCCAACATCAACATCTT
GGTCAGATTTGAACTCTTCAATCTCTTGCACTCAAAGCTTGTTAAGATAGTTAAGCGTGCATAAGTTAAC
TTCCAATTTACATACTCTGCTTAGAATTTGGGGGAAAATTTAGAAATATAATTGACAGGATTATTGGAAA
TTTGTTATAATGAATGAAACATTTTGTCATATAAGATTCATATTTACTTCTTATACATTTGATAAAGTAA
GGCATGGTTGTGGTTAATCTGGTTTATTTTTGTTCCACAAGTTAAATAAATCATAAAACTTGATGTGTTA
TCTCTTA
```

>NM_002761.2 Homo sapiens protamine 1 **(PRM1)**, mRNA

```
GACTCACAGCCCACAGAGTTCCACCTGCTCACAGGTTGGCTGGCTCAGCCAAGGTGGTGCCCTGCTCTGA
GCATTCAGGCCAAGCCCATCCTGCACCATGGCCAGGTACAGATGCTGTCGCAGCCAGAGCCGGAGCAGAT
ATTACCGCCAGAGACAAAGAAGTCGCAGACGAAGGAGGCGGAGCTGCCAGACACGGAGGAGAGCCATGAG
GTGCTGCCGCCCCAGGTACAGACCGCGATGTAGAAGACACTAATTGCACAAAATAGCACATCCACCAAAC
TCCTGCCTGAGAATGTTACCAGACTTCAAGATCCTCTTGCCACATCTTGAAAATGCCACCATCCAATAAA
AATCAGGAGCCTGCTAAGGAACAATGCCGCCTGTCAATAAATGTTGAAAGTCATCCCAAAAAAAAAAAA
AAAAAA
```

**Revendications**

1.  Procédé de sélection d'un spermatozoïde, comprenant les étapes suivantes :

    a) extraction des acides nucléiques des spermatozoïdes contenus dans un premier échantillon de spermatozoïdes préalablement obtenu auprès d'un sujet humain ;
    b) mesure du niveau d'expression d'au moins un gène marqueur choisi parmi le groupe constitué par AURKA, CFAP46, CCDC60, CCDC88B, HDAC4, CACNA1C, CACNA1H, CARHSP1, DNAH2 et SPATA18 ou un gène homologue de ceux-ci, à partir des acides nucléiques extraits;
    c) détermination de l'existence d'un différentiel d'expression du au moins un gène marqueur par rapport à un contrôle ; et
    d) sélection du spermatozoïde au sein d'un second échantillon de spermatozoïdes préalablement obtenu auprès d'un sujet humain : si il existe un différentiel d'expression, la sélection du spermatozoïde est réalisée par observation à un grossissement supérieur à x5000; si il n'existe pas de différentiel d'expression, la sélection du spermatozoïde est réalisée par observation à un grossissement inférieur à x500.

2.  Procédé selon la revendication 1, **caractérisé en ce que**, à l'étape b), le niveau d'expression du ou des gène(s) marqueur est mesuré par le niveau de la transcription de l'ARN ou de l'ADNc dudit gène.

3.  Procédé selon la revendication 1 ou 2, dans lequel, à l'étape b), outre le niveau d'expression du au moins gène, le niveau d'expression d'au moins un gène de référence est effectué.

4.  Procédé selon l'une quelconque des revendications 1 à 3, dans lequel le contrôle de l'étape c) est le niveau d'expression dudit au moins un gène marqueur, mesuré dans un échantillon de spermatozoïdes comportant une morphologie non altérée.

5.  Procédé selon la revendication 4, dans lequel, à l'étape b), la mesure du niveau d'expression (Cp) de chaque gène est effectuée par PCR quantitative en temps réel (qPCR) ; et à l'étape c), pour un gène marqueur, le différentiel d'expression est calculé selon les étapes suivantes :

i) normalisation du niveau d'expression (Cp) du gène marqueur mesuré à l'étape b) par rapport à un niveau d'expression d'un gène de référence selon

$$\Delta Cp_{GM} = [Cp_{GM}] - [Cp_{GR}]$$

où GM représente le gène marqueur et GR représente le gène de référence ; puis
ii) détermination du différentiel d'expression $\Delta\Delta Cp_{GM}$ selon

$$\Delta\Delta Cp_{GM} = [\Delta Cp_{GM}] - [\Delta Cp_{GM}]_{contrôle}$$

6. Procédé selon la revendication 5, dans lequel, à l'étape b), pour chaque gène, le niveau d'expression est un niveau d'expression moyen obtenu à partir d'une moyenne d'au moins deux mesures de niveau d'expression (Cp).

7. Procédé selon la revendication 5 ou 6, dans lequel, à l'étape b), le niveau d'expression d'un gène de référence est :

- le niveau d'expression moyen dudit gène de référence, ou
- un niveau d'expression moyen des gènes de référence calculé selon

$$Cp\ moyen\ GRs = \frac{1}{k} \sum_{i=1}^{k} (Cp\ moyen\ GRi)$$

lorsque le niveau d'expression k gènes de référence est mesurée, k étant supérieur ou égal à 2.

8. Procédé selon l'une quelconque des revendications 5 à 7, **caractérisé en ce que** l'existence d'un différentiel d'expression est établie lorsqu'un différentiel d'expression d'au moins 10% est déterminé entre le niveau d'expression du au moins un gène extrait de l'échantillon de spermatozoïdes et le contrôle.

9. Procédé selon l'une quelconque des revendications 1 à 8, **caractérisé en ce qu'**on mesure à l'étape c) le niveau d'expression d'au moins deux gènes, avantageusement de 5, et préférentiellement de 10 gènes.

10. Utilisation *in vitro* d'au moins un gène choisi parmi le groupe constitué par les gènes *AURKA, CFAP46,* CCDC60, CCDC88B, *HDAC4*, *CACNA1C*, *CACNALH*, *CARHSP1*, *DNAH2* et *SPATA18* ou des gènes homologues, ou de la combinaison des 10 gènes ou des gènes homologues, pour la détermination de la méthode de sélection visuelle d'un spermatozoïde, effectuée par microscopie optique ou photonique.

**Patentansprüche**

1. Verfahren zur Auswahl eines Spermatozoons, umfassend die folgenden Schritte:

a) Extraktion von Nukleinsäuren aus Spermatozoen, die in einer ersten Spermatozoenprobe enthalten sind, die zuvor von einem menschlichen Subjekt erhalten wurde;
b) Messung des Expressionsniveaus von mindestens einem Markergen, ausgewählt aus der Gruppe bestehend aus AURKA, CFAP46, CCDC60, CCDC88B, HDAC4, CACNA1C, CACNA1H, CARHSP1, DNAH2 und SPATA18 oder einem homologen Gen davon, aus den extrahierten Nukleinsäuren;
c) Bestimmung des Vorliegens eines Expressionsdifferentials des mindestens einen Markergens im Vergleich zu einer Kontrolle; und
d) Auswahl des Spermatozoons aus einer zweiten Spermatozoenprobe, die zuvor von einem menschlichen Subjekt erhalten wurde: wenn ein Expressionsdifferential vorliegt, wird die Auswahl des Spermatozoons durch Beobachtung bei einer Vergrößerung von mehr als x5000 durchgeführt; wenn kein Expressionsdifferential vorliegt, wird die Auswahl des Spermatozoons durch Beobachtung bei einer Vergrößerung von weniger als x500 durchgeführt.

2. Verfahren nach Anspruch 1, **dadurch gekennzeichnet, dass** in Schritt b) das Expressionsniveau des Markergens oder der Markergene durch das Transkriptionsniveau der RNA oder der cDNA des Gens gemessen wird.

3. Verfahren nach Anspruch 1 oder 2, wobei in Schritt b) neben dem Expressionsniveau des mindestens einen Gens auch das Expressionsniveau mindestens eines Referenzgens durchgeführt wird.

4. Verfahren nach einem der Ansprüche 1 bis 3, wobei es sich bei der Kontrolle in Schritt c) um das Expressionsniveau des mindestens einen Markergens handelt, das in einer Spermatozoenprobe mit unveränderter Morphologie gemessen wurde.

5. Verfahren nach Anspruch 4, wobei in Schritt b) die Messung des Expressionsniveaus (Cp) jedes Gens durch quantitative Echtzeit-PCR (qPCR) durchgeführt wird; und in Schritt c) für ein Markergen das Expressionsdifferential gemäß den folgenden Schritten berechnet wird:

i) Normalisierung des in Schritt b) gemessenen Expressionsniveaus (Cp) des Markergens im Vergleich zu einem Expressionsniveau eines Referenzgens gemäß

$$\Delta Cp_{GM} = [Cp_{GM}] - [Cp_{GR}]$$

wobei GM für das Markergen und GR für das Referenzgen steht; und dann
ii) Bestimmung des Expressionsdifferentials $\Delta\Delta Cp_{GM}$ gemäß

$$\Delta\Delta Cp_{GM} = [Cp_{GM}] - [\Delta Cp_{GM}]_{Kontrolle}$$

6. Verfahren nach Anspruch 5, wobei es sich in Schritt b) für jedes Gen bei dem Expressionsniveau um ein durchschnittliches Expressionsniveau handelt, das aus einem Durchschnitt von mindestens zwei Messungen des Expressionsniveaus (Cp) erhalten wird.

7. Verfahren nach Anspruch 5 oder 6, wobei es sich in Schritt b) bei dem Expressionsniveau eines Referenzgens um Folgendes handelt:

- das durchschnittliche Expressionsniveau des Referenzgens oder
- ein durchschnittliches Expressionsniveau der Referenzgene, berechnet gemäß

$$\text{Mittelwert } CP \ GRs = \frac{1}{k} \sum_{i=1}^{k} (\text{Mittelwert } Cp \ GRi)$$

wenn das Expressionsniveau von k Referenzgenen gemessen wird, wobei k größer oder gleich 2 ist.

8. Verfahren nach einem der Ansprüche 5 bis 7, **dadurch gekennzeichnet, dass** das Vorliegen eines Expressionsdifferentials festgestellt wird, wenn ein Expressionsdifferential von mindestens 10 % zwischen dem Expressionsniveau des mindestens einen Gens, das aus der Spermatozoenprobe extrahiert wurde, und der Kontrolle bestimmt wird.

9. Verfahren nach einem der Ansprüche 1 bis 8, **dadurch gekennzeichnet, dass** in Schritt c) das Expressionsniveau von mindestens zwei Genen gemessen wird, vorteilhafterweise von 5 und vorzugsweise von 10 Genen.

10. In-vitro-Verwendung von mindestens einem Gen, ausgewählt aus der Gruppe bestehend aus den Genen AURKA, *CFAP46, CCDC60, CCDC88B, HDAC4, CACNA1C, CACNA1H, CARHSP1, DNAH2* und *SPATA18* oder homologen Genen, oder der Kombination von 10 Genen oder homologen Genen, zur Bestimmung der Methode der visuellen Auswahl eines Spermatozoons, die durch Licht- oder Photonenmikroskopie durchgeführt wird.

**Claims**

1. A method for selecting a spermatozoon, comprising the following steps:

   a) extracting nucleic acids from spermatozoa contained in a first spermatozoa sample previously obtained from a human subject;
   b) measuring the expression level of at least one marker gene selected from the group consisting of AURKA, CFAP46, CCDC60, CCDC88B, HDAC4, CACNA1C, CACNA1H, CARHSP1, DNAH2 and SPATA18 or a homologous gene thereof, from the extracted nucleic acids
   c) determining the existence of an expression differential of the at least one marker gene compared to a control; and
   d) selecting the spermatozoon from a second spermatozoa sample previously obtained from a human subject: if there is an expression differential, the selection of the spermatozoon is carried out by observation at a magnification greater than x5000; if there is no expression differential, the selection of the spermatozoon is carried out by observation at a magnification lower than x500.

2. The method according to claim 1, **characterised in that** in step b), the expression level of the marker gene(s) is measured by the level of transcription of the RNA or cDNA of said gene.

3. The method according to claim 1 or 2, wherein in step b), in addition to the expression level of the at least one gene, the expression level of at least one reference gene is performed.

4. The method according to any of claims 1 to 3, wherein the control in step c) is the expression level of said at least one marker gene, measured in a spermatozoa sample with unaltered morphology.

5. The method according to claim 4, wherein in step b), the measurement of the expression level (Cp) of each gene is performed by quantitative real-time PCR (qPCR); and in step c), for a marker gene, the expression differential is calculated according to the following steps:

   i) normalizing the expression level (Cp) of the marker gene measured in step b) to an expression level of a reference gene according to

   $$\Delta Cp_{GM} = [Cp_{GM}] - [Cp_{GR}]$$

   where GM represents the marker gene and GR represents the reference gene; and then
   ii) determining the expression differential $\Delta\Delta Cp\ GM$ according to

   $$\Delta\Delta Cp_{GM} = [\Delta Cp_{GM}] - [\Delta Cp_{GM}]_{control}$$

6. The method according to claim 5, wherein in step b), for each gene, the expression level is an average expression level obtained from an average of at least two measurements of expression level (Cp).

7. The method according to claim 5 or 6, wherein in step b) the expression level of a reference gene is:

   - the average expression level of said reference gene, or
   - an average expression level of the reference genes calculated according to

   $$\text{GRs Average Cp} = \frac{1}{k} \sum_{i=1}^{k} (\text{GRi Average Cp})$$

   when the expression level of k reference genes is measured, k being greater than or equal to 2.

8. The method according to any of claims 5 to 7, **characterised in that** the existence of an expression differential is established when an expression differential of at least 10% is determined between the expression level of the at least one gene extracted from the spermatozoa sample and the control.

9. The method according to any of claims 1 to 8, **characterized in that** in step c), the expression level of at least two genes, advantageously 5, and preferably 10 genes is measured.

10. An *in vitro* use of at least one gene selected from the group consisting of AURKA, CFAP46, CCDC60, CCDC88B, HDAC4, CACNA1C, CACNA1H, CARHSP1, DNAH2 and SPATA18 or homologous genes, or of the combination of the 10 genes or homologous genes, for the determination of the method of visual selection of a spermatozoon by optical or photonic microscopy.

| | AURKA | CACNA1C | CACNA1H | CARHSP1 | CCDC60 | CCDC88B | CFAP46 | DNAH2 | HDAC4 | SPATA18 |
|---|---|---|---|---|---|---|---|---|---|---|
| Score 0 | 0,4 | 0,4 | 0,8 | 0,7 | 0,5 | 0,3 | 0,3 | 0,6 | 0,3 | 0,7 |
| Score 5 | 1,0 | 1,0 | 1,0 | 1,0 | 1,0 | 1,0 | 1,0 | 1,0 | 1,0 | 1,0 |

Fig. 1

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **NG CASSUTO et al.** *Fertility and Sterility,* Novembre 2009, vol. 92 (5), 1616-1625
- Laboratory manual for the examination of human semen and sperm-cervical mucus interaction. World Health Organization. Cambridge University Press, 1999
- **HELLEMANS J et al.** *Genome Bio,* 2007